# EUROPEAN PATENT APPLICATION

(11) **EP 1 422 242 A1**
(43) Date of publication of application: **26.05.2004**
(21) Application number: 03257349.5
(22) Date of filing: 21.11.2003
(51) Int. Cl.: C07K 14/78, A61K 38/39, A61L 27/22

(54) **Plastic and elastic protein copolymers**

(30) Priority: 22.11.2002 US 428438 P; 29.01.2003 CA 2417634; 01.04.2003 JP 2003098691; 27.08.2003 AU 2003236491
(71) Applicant: Emory University, Atlanta, Georgia 30322 (US)
(72) Inventor: Chaikof, Elliot Lorne, Atlanta, GA 30350 (US); Nagapudi, Karthik, Woddbridge, NJ 07095 (US)
(74) Representative: Denness, James Edward

(57) **Abstract**

Synthetic protein copolymers with plastic and elastic properties, and methods producing the copolymers, are provided. For example, a BAB triblock copolymer comprises a hydrophilic block and one or more hydrophobic blocks. The mechanical properties of a gel, fiber, fiber network, or film form of the copolymer are varied by one or more conditions before or after copolymer production. For example, a copolymer sequence can be varied before production, and one or more processing conditions such as solvent, pH, or temperature can be varied after production.

## Description

### CROSS-REFERENCES TO RELATED APPLICATIONS

This application claims priority to U.S. Provisional Patent Application Serial Number 60/428,438 filed on November 22, 2002. This application also claims all benefits under the Paris Convention, including claims to priority applications as follows: Canada Patent Application 2,417,634 filed January 29, 2003; Japan Patent Application 2003-98691 filed April 1, 2003; and Australia Patent Application 2003236491 filed August 27, 2003. Each priority application is individually incorporated herein by reference.

### FEDERAL FUNDING

This invention was made with government support under grant NSF: EEC-9731643 E-15-AO1-G1 awarded by the National Science Foundation. The government has certain rights in the invention.

### BACKGROUND

The ever-increasing demand for polymers with defined physical properties has resulted in synthetic methods, such as block copolymerization where distinct polymer chains with unique chemical and mechanical properties are covalently coupled. Such methods offer precise control of molecular composition, architecture, and organization. For example, ABA-triblock copolymers aggregate or self-assemble into micelles when mixed in a solvent that dissolves the A-blocks, but is incompatible with the B-block (Tuzar Z and Kratochvil P, 1976, Adv. Coil. Int. Sci. 6: 201).

While it is possible to generate a gel, true bridging of copolymer chains to form a network does not occur. However, on mixing in a solvent that is specific for the B-block alone, a true network can be produced consisting of insoluble end blocks that act as virtual or physical crosslinks connecting solvent-swollen central blocks (Raspaud E et al., 1994, Macromolecules 27: 2956). Triblock copolymers of this type, such as the thermoplastic elastomer, styrene-butadiene-styrene (SBS) copolymers, have traditionally been derived from conventional organic monomers, which display aggregation behavior in organic solvents.

For biological applications, however, organization at the nanoscale or microscale level of block copolymers is preferably sought for physiologically compatible solvents such as water-based solvents. This has led to the development of amphiphilic block copolymers, which contain blocks of hydrophobic and hydrophilic chains (Alexandridis, P., & Lindman, B., eds., Amphiphilic Block Copolymers, Elsevier, Amsterdam, 2000; Nowak, A. P., et al., 2002, Nature 417, 424-428; Pochan, D. J. et al., 2002, Macromolecules 35, 5358-5360). Nevertheless, the synthetic repertoire of these materials has been limited to tapered blocks of uniform sequence, which potentially restricts the functional complexity of the resulting microstructures.

The emergence of genetic engineering of synthetic polypeptides (Ferrari F and Cappello J, 1997, in Protein-Based Materials (eds McGrath, K., & Kaplan, D.), Ch. 2, Birkhäuser, Boston) has recently enabled the preparation of block copolymers composed of complex peptide sequences in which individual blocks may have different mechanical, chemical, and biological properties (Petka WA et al., 1998). The segregation of protein blocks into compositionally, structurally, and spatially distinct domains occurs in a fashion analogous to that in synthetic block copolymers, affording ordered structures in the nanoscale to mesoscale size range.

Despite more than four decades of research, a clinically durable, small diameter arterial prosthesis remains an elusive goal. Several approaches directed at reproducing a biocomposite equivalent of the arterial wall have included: (i) layered co-culture of endothelial and smooth muscle cells (Weinberg CB, Bell E., Science 1986;231:397-400); (ii) endothelialization of synthetic polymers, such as expanded-PTFE in vitro (Herring MB, Dilley R, Jersild RAJ, Boxer L, Gardner A, Glover J., Ann Surg 1979;190:84-90); and (iii) induced transmural angiogenesis in vivo for coverage of a synthetic polymer or tissue-based scaffold with an inner lining of endothelial cells (see Gray JL, Kang SS, Zenni GC, Kim DU, Kim PI, Burgess WH, et al. FGF-1 affixation stimulates ePTFE endothelialization without intimal hyperplasia. J Surg Res 1994;57(5):596-612; Greisler HP, Cziperle DJ, Kim DU, Garfield JD, Petsikas D, Murchan PM, et al. Enhanced endothelialization of expanded polytetrafluoroethylene grafts by fibroblast growth factor type 1 pretreatment. Surgery 1992;112(2):244-54; Golden MA, Hanson SR, Kirkman TR, Schneider PA, Clowes AW. Healing of polytetrafluoroethylene arterial grafts is influenced by graft porosity. J Vasc Surg 1990;11(6):838-44; discussion 45). While these strategies have had some success, none has resulted in a clinically durable device.

As biomaterials, elastin-mimetic protein polymers have been processed largely into elastomeric hydrogels of various forms including sheets and tubular constructs by chemical, enzymatic, and gamma-irradiation mediated crosslinking of protein solutions (Urry DW, Pattanaik A. Elastic protein-based materials in tissue reconstruction. Ann NY Acad Sci 1997;831:32-46; Urry DW, Pattanaik A, Accavitti MA, Luan CX, McPherson DT, Xu J, et al. Transductional elastic and plastic protein-based polymers as potential medical devices. In: Domb AJ, Kost J, Wiseman DM, editors. Handbook of Biodegradable Polymers. Amsterdam: Harwood; 1997. p. 367-86). Similarly, type I collagen has been predominantly used either after processing into a dry powder or slurry, a hydrogel after solution phase crosslinking, or as a porous matrix with or without the addition of other components after freeze-drying (Silver FH, Garg AK. Collagen: Characterization, processing and medical applications. In: Domb AJ, Kost J, Wiseman DM, editors. Handbook of Biodegradable Polymers. Amsterdam: Harwood; 1997. p. 319-46). Nonetheless, it is as integrated fiber networks that collagen and elastin constitute the principal structural elements of tissue.

Thus a question remains, regarding matrix proteins when produced as genetically engineered recombinants, as to whether their versatility as scaffolds for tissue engineering applications will be sufficiently desirable when reformulated into fiber networks.

Although the structural features of the vascular wall vary with location, the lamellar unit of the aortic media provides a useful starting point for bioprosthesis design that is based upon a consideration of the ultrastructure of the arterial wall (Wolinsky H, Glagov S. A lamellar unit of aortic medial structure and function in mammals. Circ Res 1967;20:99-111; Clark JM, Glagov S. Transmural organization of the arterial media: The lamellar unit revisited. Arteriosclerosis 1985;5:19-34; Dingemans KP, Teeling P, Lagendijk JH, Becker AE. Extracellular matrix of the human aortic media; An ultrastructural histochemical and immunohistochemical study of the adult aortic media. Anat Record 2000;258:1-14).

Characteristically, alternating layers of elastin, type I collagen, and smooth muscle cells constitute a repeating lamellar unit where the geometry and loading pattern of collagen dominate mechanical responses at high strains, while the behavior of elastin fiber networks dictate low strain mechanical behavior (Roach MR, Burton AL. The reason for the shape of the distensibility curves of arteries. Can J Biochem Physiol 1957;35:681-90; Humphrey JD. Mechanics of the arterial wall: review and directions. Critical Rev Biomed Eng 1995;23(1-2):1-162).

In short, collagen fiber networks serve a critical function by limiting high strain deformation, which prevents aneurysm formation with inevitable disruption of the vascular wall. In contrast to collagen, elastin is much weaker, softer and can undergo significant deformation without rupture. Notably, elastin is also highly resilient with very little energy stored during cyclic loading (Apter JT, Marquez E. A relation between hysteresis and other visco elastic properties of some biomaterials. Biorheology 1968;5(4):285-301; Urry DW, Haynes B, Thomas D, Harris RD. A method for fixation of elastin demonstrated by stress/strain characterization. Biochem Biophys Res Comm 1988;151(2):686-92). Thus, elastin networks maximize the durability of tissues that are loaded by repetitive forces through minimizing the dissipation of transmitted energy as heat, which over time would lead to catastrophic tissue failure due to thermally induced degradation of collagen, elastin or other structural constituents.

It is noteworthy that the integrated nature of both protein network systems also establishes a unique biomechanical microenvironment for optimal smooth muscle and endothelial cell function (51-53). Current acellular matrix bioprostheses do not have mechanical properties that compare favorably with those of a native blood vessel, primarily due to the loss or degradation of the elastin protein network.

Elastin, which is derived from the soluble precursor tropoelastin, is widely distributed in vertebrate tissues where it consists of repetitive glycine-rich hydrophobic elastomeric domains of variable length that alternate with alanine-rich, lysine-containing domains that form crosslinks (Sandberg LB, Soskel NT, Leslie JG. Elastin structure, biosynthesis, and relation to disease states. New Eng J Med 1981;304(10):566-79; Indik Z, Yeh H, Ornstein-Goldstein N, Sheppard P, Anderson N, Rosenbloom JC, et al. Alternative splicing of human elastin mRNA indicated by sequence analysis of cloned genomic and complementary DNA. Proc Nat Acad Sci USA 1987;84(16):5680-4; Rosenbloom J, Abrams WR, Indik Z, Yeh H, Ornstein-Goldstein N, Bashir MM. Structure of the elastin gene. Ciba Foundation Symp 1995;192:59-74).

Native elastin's intrinsic insolubility, however, has restricted its capacity to be purified and processed into forms suitable for biomedical or industrial applications. This limitation has been partly overcome by the structural characterization of the elastomeric domains. Specifically, sequence analysis has revealed the presence of consensus tetra- (VPGG; SEQ ID NO:1), penta- (VPGVG; SEQ ID NO:2), and hexapeptides (APGVGV; SEQ ID NO:3) repeat motifs (Gray WR, Sandberg LB, Foster JA. Molecular model for elastin structure and function. Nature 1973;246(5434):461-6; Urry DW, Mitchell LW, Ohnishi T. Studies on the conformation and interactions of elastin secondary structure of synthetic repeat hexapeptides. Biochimica et Biophysica Acta 1975;393(2):296-306; Sandberg LB, Gray WR, Foster JA, Torres AR, Alvarez VL, Janata J. Primary structure of porcine tropoelastin. Adv Exp Med Biol 1977;79:277-84; Rapaka RS, Okamoto K, Urry DW. Non-elastomeric polypeptide models of elastin. Synthesis of polyhexapeptides and a cross-linked polyhexapeptide. Inter J Pept Protein Res 1978;11(2):109-27; Urry DW, Harris RD, Long MM, Prasad KU. Polytetrapeptide of elastin. Temperature-correlated elastomeric force and structure development. Inter J Pept Protein Res 1986;28(6):649-60; Broch H, Moulabbi M, Vasilescu D, Tamburro AM. Quantum molecular modeling of the elastinic tetrapeptide Val-Pro-Gly-Gly. J Biomol Structure Dynamics 1998;15(6):1073-91).

Notably, only polymers of the pentapeptide exhibit elastic behavior with spectroscopic features that are consistent with those of native elastin, including a highly mobile backbone and the presence of beta-turns and a loose helical beta-spiral (Urry DW, Long MM, Cox BA, Ohnishi T, Mitchell LW, Jacobs M. The synthetic polypentapeptide of elastin coacervates and forms filamentous aggregates. Biochimica et Biophysica Acta 1974;371(2):597-602; Urry DW, Long MM. On the conformation, coacervation and function of polymeric models of elastin. Adv Exper Med Biology 1977;79:685-714; Urry DW, Luan CH, Peng SQ. Molecular biophysics of elastin structure, function and pathology. Ciba Foundation Symp 1995;192:4-22). Thus, the pentapeptide sequence (VPGVG) has formed the basis for the synthesis of protein polymers with elastomeric domains by standard solution and solid phase chemical methodologies and, more recently, by genetic engineering strategies, as developed by Conticello V.P. (McMillan RA, Lee TAT, Conticello VP. Rapid assembly of synthetic genes encoding protein polymers. Macromolecules 1999;32:3643-8; McMillan RA, Conticello VP. Synthesis and characterization of elastin-mimetic protein gels derived from a well-defined polypeptide precursor. Macromolecules 2000;33:4809-21 ), among others (McPherson DT, Morrow C, Minehan DS, Wu J, Hunter E, Urry DW. Production and purification of a recombinant elastomeric polypeptide, G-(VPGVG)19-VPGV (SEQ ID NO:4), from Escherichia coli. Biotech Progress 1992;8(4):347-52; Daniell H, Guda C, McPherson DT, Zhang X, Xu J, Urry DW. Hyperexpression of a synthetic protein-based polymer gene. Methods Mol Biol 1997;63:359-71; Panitch A, Yamaoka T, Fournier MJ, Mason TL, Tirrell DA. Macromolecules 1999;32:1701-3).

A general challenge remains of generating desirable synthetic polypeptides that mimic natural structural matrix proteins. This challenge extends to the field of tissue engineering, for example, in the area of fabrication of an arterial bioprosthesis that is tailored to one or more targeted design criteria such as, tensile strength, elastic modulus, viscoelasticity, and in vivo stability, as well as the optimization of a desired host response.

### SUMMARY OF THE INVENTION

This invention provides a synthetic protein copolymer having selected plastic and elastic properties comprising at least one hydrophilic block and at least one hydrophobic block. Protein copolymers of the invention can comprise two blocks, three blocks, or more than three blocks.

An embodiment of the invention comprises a protein copolymer having a first end block, a second end block, and a middle block, wherein said first and second end blocks are substantially identical. In a preferred embodiment, the protein copolymer comprises hydrophobic end blocks and a hydrophilic middle block.

In a particular embodiment, the first end block comprises a nucleic acid sequence capable of encoding an amino acid sequence of [VPAVG(IPAVG)₄]ₙ or a [(IPAVG)₄(VPAVG)]ₙ sequence. In another embodiment, the middle block comprises a nucleic acid sequence capable of encoding an amino acid sequence selected from the group consisting of: [(VPGEG) (VPGVG)₄]ₘ , [(VPGVG)₄(VPGEG)]ₘ , and [(VPGVG)₂VPGEG(VPGVG)₂]ₘ. In another embodiment, the protein copolymer comprises endblocks selected from the above endblock sequences and a middle block selected from the above middle block sequences, n is from about 5 to about 100, and m is from about 10 to about 100. In a particular embodiment, n is about 16. The following cross-references to sequence listings are noted.

| | |
|---|---|
| [VPAVG(IPAVG)₄]ₙ | [SEQ ID NO:5(SEQ ID NO:6)4]n;[ SEQ ID NO:11]n |
| [(IPAVG)₄(VPAVG)]ₙ | [(SEQ ID NO:6)4 (SEQ ID NO:5)]n; [SEQ ID NO:12]n |
| [(VPGEG) (VPGVG)₄]ₘ | [(SEQ ID NO:13)(SEQ ID NO:2)4]m; [SEQ ID NO:14]m |
| [(VPGVG)₄(VPGEG)]ₘ | [(SEQ ID NO:2)4 (SEQ ID NO:13)]m; [SEQ ID NO:15]m |
| [(VPGVG)₂VPGEG(VPGVG)₂]ₘ | [(SEQ ID NO:2)2 (SEQ ID NO:13) (SEQ ID NO:2)2]m; |
| | [SEQ ID NO:18]m |

In an embodiment of the invention, the middle block is selected from the group consisting of:

| STRUCTURE | SEQ ID NO: |
|---|---|
| VPGVG [VPGVG(VPGIGVPGVG)₂]₁₉VPGVG; | 21 |
| VPGVG [(VPGVG)₂VPGEG(VPGVG)₂]₃₀VPGVG; | 23 |
| VPGVG [(VPGVG)₂VPGEG(VPGVG)₂]₃₈VPGVG; | 24 |
| VPGVG [(VPGVG)₂VPGEG(VPGVG)₂]₄₈VPGVG; | 25 |
| VPGVG [VPGVG(VPNVG)₄]₁₂VPGVG; | 30 |
| VPGVG [(APGGVPGGAPGG)₂]₂₃VPGVG; | 33 |
| VPGVG [(APGGVPGGAPGG)₂]₃₀VPGVG; | 35 |
| [VPGVG(IPGVGVPGVG)₂]₁₉; | 38 |
| [VPGEG(VPGVG)₄]₃₀; | 41 |
| [VPGEG(VPGVG)₄]₄₈; | 42 |
| [(APGGVPGGAPGG)₂]₂₂; and | 43 |
| [(VPGMG)₅]ₓ, wherein x is from about 10 to about 100. | 63 |

The invention provides copolymers having a range of mechanical properties. In an embodiment, a copolymer is capable of elongation up to about 14 times its initial length. In a particular embodiment, a protein copolymer of an initial length has elasticity for elongation of from about at least 2.5 said initial length to about 13 times said initial length.

Protein copolymers of the invention can be converted into a variety of forms. For example, the protein copolymers can be in the form of a film, a gel, a fiber or fiber network, or small, roughly spherical or bead-like particles. Such forms can be used in a variety of applications. For example, a film form can be used to at least partially cover a medical device, cell, tissue, or organ. By so doing, the at-least-partially-covered object can be rendered more biocompatible or otherwise have its overall mechanical or surface properties altered.

A given form can be manipulated into a selected physical shape. For example, a film, fiber, or fiber network form can be manipulated into the shape of a planar sheet or a tubular conduit.

An embodiment of the invention is a medical device, cell, tissue, or organ at least partially covered or reinforced with a fiber or fiber network form of a protein copolymer.

A protein copolymer of the invention can be in the form of a biocompatible coating on a device. An example of such a device is a medical implant.

An embodiment is a wound dressing comprising a protein copolymer of the invention.

Another embodiment is a cell, tissue, or organ partially or completely encapsulated by a protein copolymer. A protein copolymer can be in a gel form or film form, or other form for encapsulation. The encapsulation can serve in a variety of functional ways. One way is to confer a level of protection to a transplanted material from immune reaction. Another way is to control the release of a material from an encapsulated object; for example, wherein the encapsulation surface affects the ability of the material to diffuse through, permeate, or otherwise elute through a protein copolymer embodiment. A particular embodiment is a pancreatic islet cell so encapsulated. In another embodiment, the cell to be encapsulated is selected from the group consisting of a smooth muscle cell, a fibroblast, an endothelial cell, a stem cell, a chondrocyte, an osteoblast, a pancreatic islet cell, or a genetically engineered cell.

The invention provides a protein copolymer that can be non-covalently crosslinked or have a combination of non-covalent and covalent crosslinking.

The invention provides a complex comprising a first and a second protein copolymer wherein the first and second copolymers are joined by non-covalent crosslinks, covalent crosslinks, or a combination of non-covalent and covalent crosslinks. The invention further provides such a complex wherein the first and second protein copolymers are substantially identical.

The invention provides a protein copolymer further comprising a chemical substituent. In an embodiment, such a substituent can be an amino acid capable of facilitating crosslinking or derivatization. In a particular embodiment, the amino acid can be, for example, lysine, glutamine, or other amino acid as known in the art.

The invention provides a protein copolymer comprising a functional site capable of facilitating chemical derivitization for a covalent crosslinking reaction. In an embodiment, a protein copolymer comprises a photocrosslinkable acrylate group capable of forming stable crosslinks upon an interaction with an appropriate initiator and light. Other cross-linking groups known to the art may also be used.

An embodiment of the invention is a protein copolymer comprising a functional site capable serving as a binding site, e.g., for an enzyme or antibody. In a particular embodiment, the functional site comprises a selected protease site capable of allowing degradation of said protein copolymer. In another embodiment, a protein copolymer comprises a metal or other inorganic ion nucleation site.

An embodiment is a protein copolymer comprising an adhesion molecule recognition site or enzyme active site.

An embodiment of the invention is a protein copolymer comprising an agent wherein the agent is a drug or biologically active molecule or biomacromolecule. Such agent can be covalently bound or non-covalently bound to said copolymer. A related embodiment comprises a method of controlled release or delivery of said agent, wherein a protein copolymer is in the form of a film, gel, fiber, or fiber network.

An embodiment of the invention is a protein copolymer further comprising a selected molecule wherein the selected molecule is a saccharide, oligosaccharide, polysaccharide, glycopolymer, ionic synthetic polymer, non-ionic synthetic polymer, or other organic molecule. Such a selected molecule can be joined to said copolymer by covalent binding, non-covalent binding, or a combination of covalent and non-covalent binding.

An embodiment is a protein copolymer further comprising a synthetic or natural compound capable of effecting an alteration of a surface property of said copolymer.

The invention provides a method for producing a plastic elastic protein copolymer comprising the steps of a) providing a first block of nucleic acid sequence, wherein said first block encodes a hydrophilic protein; b) providing a second block of nucleic acid sequence, wherein said second block encodes a hydrophobic protein; c) synthesizing a nucleic acid molecule comprising said first and second blocks; and d) expressing said nucleic acid molecule to produce said protein copolymer. By assembling a copolymer at the nucleic acid sequence level, the advantages of recombinant engineering allow the copolymer to be varied regarding sequence identity, the number of repeating sequence units, and overall size.

Corresponding methods for synthesizing copolymers having three or more blocks are also provided comprising synthesizing nucleic acid molecules coding for such copolymers and expressing the nucleic acid to produce the copolymer.

A possible explanation of the mechanism of aspects of the invention involves the thermodynamic properties of a copolymer in relation to a system, where the system is a set of conditions that can include solvent, pH, and temperature. For example, in an embodiment a hydrophobic end block will tend to orient away from a polar solvent, whereas a hydrophilic middle block will tend to orient towards a polar solvent. The addition to the system of heat may at least partially allow a reversal of the tendency of a hydrophobic end block to orient away from the polar solvent. At a certain critical temperature, the thermodynamic properties can cause a protein copolymer solution to agglomerate to form a non-covalently crosslinked mass. The noncovalent crosslinks can also be referred to as virtual or physical crosslinks, and are distinguished from covalent crosslinks. An embodiment of the invention, however, can additionally include covalent crosslinking.

An embodiment is a copolymer that has an inverse transition temperature. A particular embodiment is a copolymer having a transition temperature of from about 4°C to about 40°C. A more particular embodiment is a protein copolymer having a transition temperature of from about 16°C to about 25°C. Another more particular embodiment is a protein copolymer having a transition temperature of from about 32°C to about 37°C.

The invention provides a method of generating a plastic elastic copolymer protein having a desired transition temperature. The method involves selecting a desired transition temperature. The method comprises the steps of: a) selecting a nucleic acid sequence corresponding to an amino acid sequence, wherein a protein of said amino acid sequence is capable of having a thermoplastic and an elastomeric property; b) expressing the protein using recombinant technology; c) selecting a set of conditions comprising a solvent, a pH, and a first temperature in accordance with the teachings hereof; d) exposing the expressed protein to the set of conditions; e) bringing the protein to a second temperature at which a transition occurs; f) comparing the observed transition temperature to a desired transition temperature; and g) repeating the above steps if the second temperature at which a transition occurs does not approximate the desired transition temperature. In an embodiment of such method, such desired transition temperature can range from about 5°C to about 9°C, from about 10°C to about 15°C, from about 16°C to about 20°C, from about 21 °C to about 25°C, from about 26°C to about 30°C, from about 31 °C to about 37°C, or other range.

The invention further provides the copolymer in a solvent. A solvent can be polar or non-polar. In a preferred embodiment, the solvent is polar. In an embodiment, the solvent is water, trifluoroethanol (TFE), or hexafluoroisopropanol, or a combination of two or more of those. A solvent can comprise an aqueous component, an organic component, or a mixture of aqueous and organic components. A solvent can be adjusted with respect to pH. For example, in an embodiment the pH is adjusted to a basic condition. In a particular embodiment, the basicity is sufficient to allow ionization of a glutamic acid amino acid residue. A protein copolymer can be solubilized in a solvent. A solubilized copolymer can produce a hydrogel. A solvent can be selected so as to produce a desired conformation of said copolymer in solution. A solvent can be selected so as to produce a desired mechanical property or a desired biological property.

In film embodiments of the invention, there can be variations of several types. For example, a film can have a plurality of layers. Furthermore, the film can have homogeneous or heterogeneous layers. A film can further comprise a synthetic or natural fiber, a drug, or other biologically active compound or material.

The invention further provides a method of making a film. The method utilizes a protein copolymer of the invention and comprises the steps of a) selecting a set of conditions where the conditions include a solvent, a first temperature, and a pH value in accordance with the teachings hereof to provide a film having the desired properties; b) exposing the protein to the condition set, thereby making a solution; c) bringing the solution to a second temperature, and d) removing the solvent, thereby generating a film. In a particular embodiment, a solution of copolymer in water is prepared at about 5°C and poured onto a planar surface; next the water is allowed to evaporate at about 23°C, generating a film.

In a particular embodiment, the solvent is trifluoroethanol and the film material is plastic. In another particular embodiment, the solvent is water and the film material is elastic. In yet another particular embodiment, the solvent facilitates the development of a film having a combination of plastic and elastic properties.

In making a film, the film can be modified by including a substance such as a protein, polysaccharide, or other bioactive compound. Such modification can be achieved in at least three ways. First, the substance can be included in the solvent. Second, the substance can be included by direct adsorption in contact with a cast film. Third, the substance can be included in a separate solvent, producing a solution of the substance; this solution can be used as a coating solution for a cast film.

The invention provides a drug delivery material comprising a protein copolymer of the invention. In an embodiment, a protein copolymer can be in the form of a small particle having a diameter of less than one micrometer up to about 500 micrometers. In a particular embodiment, the small particle is a roughly spherical nanoparticle or microparticle.

The invention provides a protein copolymer and an agent. An embodiment of the invention is a method of preparing such composition, involving the addition of an agent to a solution of solvent and copolymer. The agent can be a drug, biologically active molecule, or biomacromolecule.

The invention provides a method of delivery of a drug or biological agent via a stent, embolization coil, vascular graft, or other implanted biomedical device. By including the drug or biological agent in the solvent, one can thereby make a mixture with said copolymer. In an embodiment of the invention, the mixture is in the form of a gel, film, fiber, or fiber network. One can apply said mixture to said stent, embolization coil, vascular graft, or other implanted biomedical device. In a particular embodiment, the drug is sirolimus. In another embodiment of the invention, the drug is amphiphilic.

The invention provides a method of generating a protein-based small particle capable of drug or biological agent delivery comprising the steps of a) selecting conditions comprising a solvent, a temperature, and pH in accordance with the teachings hereof; b) incorporating a drug or biological agent with the solvent; c) exposing the protein to the conditions; and d) removing the solvent.

The invention provides a method of delivering a drug or biological agent comprising the step of applying a small particle to a subject. In an embodiment, the particle is applied via intravenous, subcutaneous, intraosseus, intravitreal, intranasal, oral or other appropriate route as known in the art. In a particular embodiment, the drug is amphiphilic.

The invention provides a method of controlling a release of drug or biological agent during drug delivery from a copolymer of this invention comprising the steps of observing a release profile, comparing the observed profile to a desired release profile, and varying the selection of the first block, the second block, or the conditions of making the copolymer described above if the observed release profile does not approximate the desired release profile.

The invention further comprises a method of making a fiber or fiber network. A fiber or fiber network can be formed by electrospinning. For example, the method of Patent Cooperation Treaty International Publication Number WO 01/80921 (International Application Number PCT/US01/12918), incorporated by reference to the extent not inconsistent herewith, can be applied to a copolymer of the invention.

The invention provides a method for reinforcing closure of a surgical incision. An incision can be treated with a fiber, fiber network, gel, or film form of an embodiment of the invention. In a particular embodiment, the surgical incision is associated with a lung biopsy or an intestinal anastomasis.

The invention provides a method of treating a non-surgical injury comprising applying to the injury an implant or dressing. The invention provides a method of preventing adhesion formation or treating an adhesion following a surgical intervention by applying a film, fiber, or fiber network to the surgical site. In an embodiment, the method can further comprise the step of incorporating a hydrophilic polysaccharide or a glycopolymer.

The invention provides a method of delivering a drug or biological compound to a subject comprising the steps of formulating a solution of the drug or compound with a protein copolymer and solvent, bringing the solution to a desirable temperature thereby producing a gel, and exposing the subject to the gel. In an embodiment, the exposing to the subject is by subcutaneous injection, intramuscular injection, intradermal injection, intraocular injection, or subconjunctival application. In a particular embodiment, the gel is in a blood vessel, thereby effecting an embolization. In another embodiment, the drug or biological compound is a chemotherapeutic agent.

The invention provides a method of treating a tumor comprising the steps of applying to the tumor a solution or gel form of a copolymer. In an embodiment, the application is by intra-arterial injection via a catheter.

The invention provides a method of generating a medical implant having a selected mechanical property comprising applying a gel, film, fiber, or fiber network to the implant. In an embodiment, the implant comprises skin, vein, artery, ureter, bladder, esophagus, intestine, stomach, heart valve, heart muscle, or tendon.

In an embodiment the invention provides a method of generating a wound dressing having a selected mechanical property and having a selected shape, comprising forming a film or fiber into the selected shape.

Resultant polypeptides of the invention can self-assemble above a critical solution temperature due to the formation of virtual or physical (non-covalent) crosslinks between the terminal blocks. Proteins of the invention can be fabricated into stable elastic and plastic fibers, fiber networks, films, gels, particles, and cell encapsulation surfaces.

The invention provides for the ability to modulate parameters and conditions such as the protein sequence, number of repeats, concentration of protein, solvent, pH, temperature, and combinations thereof. Such modulation can effect changes in mechanical properties, for example, elasticity, plasticity, and a combination of elasticity and plasticity. Such modulation can effect changes in biological properties, for example, biocompatibility and biological function.

The invention provides stable protein-based materials that are mechanically robust without covalent crosslinking. Such materials can be stable under physiologic conditions. Blends with other protein-based materials can be formed. The protein materials can be functionalized through conjugation reactions along the protein backbone or through amino acid residues. The protein materials can be used to generate elastic and plastic fiber-reinforced composites. A protein copolymer in gel, film, fiber, or fiber network form can be conjugated to a sugar molecule.

The invention provides particular embodiments such as blood vessel substitutes, heart valve substitutes, artificial skin, wound healing barriers, cell encapsulation surfaces, drug delivery injectables, embolic and chemoembolic agents, and drug-eluting small particles such as nanoparticles and microparticles.

In an embodiment of the invention, genetic engineering strategies are applied to the design of protein polymers for tissue engineering applications. The capacity to vary molecular weight, peptide sequence, as well as the density and position of potential crosslinking sites facilitates tailoring of the morphological and physiochemical properties of recombinant protein analogues to natural proteins. A protein copolymer in the form of a film, gel, fiber, or fiber network provides a tissue engineering scaffold. A scaffold can function with resilience and long-term durability under environmental conditions of repetitive loading.

In an embodiment of the invention, solution and solid-state techniques are used to define the molecular and supramolecular characteristics of mimetic fibers that dictate the effects on fiber strength and modulus in response to sustained mechanical loads in a biological environment. Materials and methods are established for improving the durability of tissue engineered constructs that operate under conditions of prolonged static or cyclic tensile stress under physiologically relevant conditions.

In an embodiment, the defined mechanical properties of a copolymer aid in the analysis of local wall stresses and stress distribution in early tissue development and in the susceptibility of fiber networks to biodegradation through cell-mediated or other processes.

In an embodiment, triblock copolymers allow clustering of bioactive sequences into high-density regions by engineering the target sequence(s) into the central elastomeric block.

In an embodiment, integrated collagen and elastin-mimetic fiber networks are produced with modulated mechanical properties through appropriate choice of fiber type and three-dimensional architecture.

In an embodiment, a method of reducing neointimal hyperplasia is provided by minimizing a compatibility difference, for example a compliance mismatch difference, between an implanted bioprosthesis and a host vessel.

The design of protein polymers that mimic native structural proteins, and the assembly of these recombinant proteins under various conditions and in various combinations with naturally-occurring matrix proteins, provides an opportunity to optimize the mechanical properties of a material, well as other biologically related characteristics. For example, an at least partially synthetic arterial bioprosthesis can be generated. One approach is to develop an arterial prosthesis with mechanical properties that approximate or exceed those of a native artery, along with sufficient biostability as compared to decellularized allogeneic or xenogeneic tissue.

The following definitions are intended to be used herein.

ABA and BAB triblock copolymers. Each of these terms refers to a copolymer comprising three blocks, where two of the blocks are substantially identical. The A designation can have a particular attribute, for example, hydrophilicity or hydrophobicity. In such case, the B block would have the alternative attribute. In the polymer field, there is no universally accepted convention that A designates a hydrophobic block. Herein, the intention is to refer to the B block as the hydrophobic block; therefore the A block designates a hydrophilic block. Thus a BAB triblock copolymer refers to a protein comprising three blocks; there are two substantially identical hydrophobic end blocks and a hydrophilic middle block.

Plastic. This term refers to a mechanical property, the capacity of a material to undergo irreversible deformation. While the term thermoplastic can refer to the capability of a material to soften or fuse when heated and harden, gel, or solidify again when cooled, in many embodiments of this invention the term refers to the capability for hardening, gelling, or solidifying upon heating and at least partially softening upon cooling.

Elastic. This term refers to a mechanical property relating to the capacity of a material to undergo reversible deformation. The term elastomeric also refers to the elastic quality of a substance.

Transition temperature (Tt). This term refers to the temperature associated with the equilibrium point relating to a phase change from one state of matter to another, for example from a liquid phase to a solid phase. An exemplary embodiment having an inverse transition temperature can change from a liquid phase to a solid phase as the temperature increases.

### BRIEF DESCRIPTION OF THE DRAWINGS

Figure 1: Differential Scanning Calorimetry (DSC) thermograms of P2 and B9 in water.

Figure 2: Stress-strain curves for the B9 triblock copolymer cast at different temperatures from water or trifluoroethanol (TFE).

Figure 3: Hysteresis curves for B9 cast from TFE and water.

Figure 4: Dynamic mechanical data for B9 cast from water or TFE at 5°C.

Figure 5a: Recognition and cleavage sequence of Eam1104 I.

Figure 5b: Schematic representation of cleavage pattern.

Figure 6: Rheological behavior of Copolymer 1.

Figure 7: SEM of spun fibers of Copolymer 2.

Figure 8: Stress-strain curve for hydrated fabric sample of Copolymer 2.

Figure 9: Dynamic shear storage and loss modulus for B9 at 1 rad/s.

Figure 10: Dynamic shear storage and loss modulus for B9 at 10 rad/s.

Figure 11: Dynamic shear storage and loss modulus as a function of temperature.

Figure 12: Dynamic shear storage and loss modulus as a function of frequency.

Figure 13: Shear storage modulus and complex viscosity as a function of time at 37°C

Figure 14: First normal stress difference as a function of time at 37°C

Figure 15: Dynamic shear storage modulus, shear loss modulus and complex viscosity as a function of strain amplitude at a frequency of 10 rad/s for P2Asn.

Figure 16: Dynamic shear storage modulus and loss modulus as a function of temperature at strain amplitude of 5% and at a frequency of 10 rad/s for P2Asn.

Figure 17: Dynamic shear storage modulus, loss modulus and complex viscosity as a function of frequency (strain amplitude = 5% and temperature = 37°C) for P2Asn.

Figure 18: Shear storage modulus and complex viscosity as a function of time at 37°C with a strain amplitude of 5% and a frequency of 10 rad/sec for P2Asn.

Figure 19: Dynamic shear storage modulus, shear loss modulus and complex viscosity as a function of strain amplitude at a frequency of 10 rad/s for PHP.

Figure 20: Dynamic shear storage modulus and loss modulus as a function of temperature (strain amplitude=2.5% and frequency = 10 rad/s) for PHP.

Figure 21: Dynamic shear storage modulus, loss modulus and complex viscosity as a function of frequency (strain amplitude = 5% and temperature = 37°C) or PHP.

Figure 22: Shear storage modulus and complex viscosity as a function of time at 37°C with a strain amplitude of 5% and a frequency of 10 rad/sec for PHP.

Figure 23: Stress-strain curves for copolymers P2, P2Asn, PHP, and B9.

Figure 24: mechanical properties of triblock copolymer materials are modulated in laminates

Figure 25a: B9 copolymer fiber from TFE, 350X

Figure 25b: B9 copolymer fiber from TFE, 10kX

Figure 26a: C5 copolymer fiber from TFE, 300X

Figure 26b: C5 copolymer fiber from TFE, 5kX.

Figure 27a: B9 copolymer fiber from water, 1 kX

Figure 27b: B9 copolymer fiber from water, 5kX.

Figure 28: Structure of S1 P.

Figure 29: Domain sizes and interface profiles.

Figure 30: Domain sizes in B9 from different solvents.

Figure 31: 13C solution-state spectrum of unlabeled B9

Figure 32: 13C solution-state spectrum of B9 labeled in the end block alanine.

Figure 33: Absorbance versus wavelength curve for S1P in phosphate buffered saline.

Figure 34: Absorbance versus concentration of S1P.

Figure 35: Release of S1 P from B9 films over time.

Figure 36: Diagrams of triblock copolymers

Figure 37: Morphological change of triblock copolymer upon reaching a transition temperature, Tt.

Figure 38: Copolymers in block-specific solvents.

Figure 39: Virtual or phyically crosslinked network of copolymers.

Figure 40a: Diagram of synthetic thermoplastic elastomers.

Figure 40b: Stress versus elongation for synthetic thermoplastic elastomers.

Figure 41: Diagram of mechanical properties as a function of transition temperature.

Figure 42: Diagram of blocks and amino acid sequences.

Figure 43: NMR spectroscopy of phase transition.

Figure 44: Shear storage modulus and complex viscosity versus time for a film.

Figure 45: Stress versus strain for a film.

Figure 46: Mechanical properties of copolymers and system morphology.

Figure 47: Solvent effect on films.

Figure 48a: Rheological comparison of copolymers P2Asn, B9, and PHP; G' versus frequency.

Figure 48b: Rheological comparison of copolymers P2Asn, B9, and PHP; tan delta versus frequency.

Figure 49: Microsphere from copolymer B9.

### DETAILED DESCRIPTION OF THE INVENTION

### EXAMPLES

### Example 1. Construction of C5 and B9.

We have used pET-24a (available from Novagen, Inc.) as the expression plasmid. The plasmid has been modified. For such modifications, and other materials, methods, and results, two publications (Wright ER et al., 2002 Adv. Funct. Mater. 12:149-154; Wright ER and Conticello VP, 2002 Adv. Drug Deliv. Rev. 54(8):1057-1073) are incorporated herein by reference to the extent not inconsistent herewith.

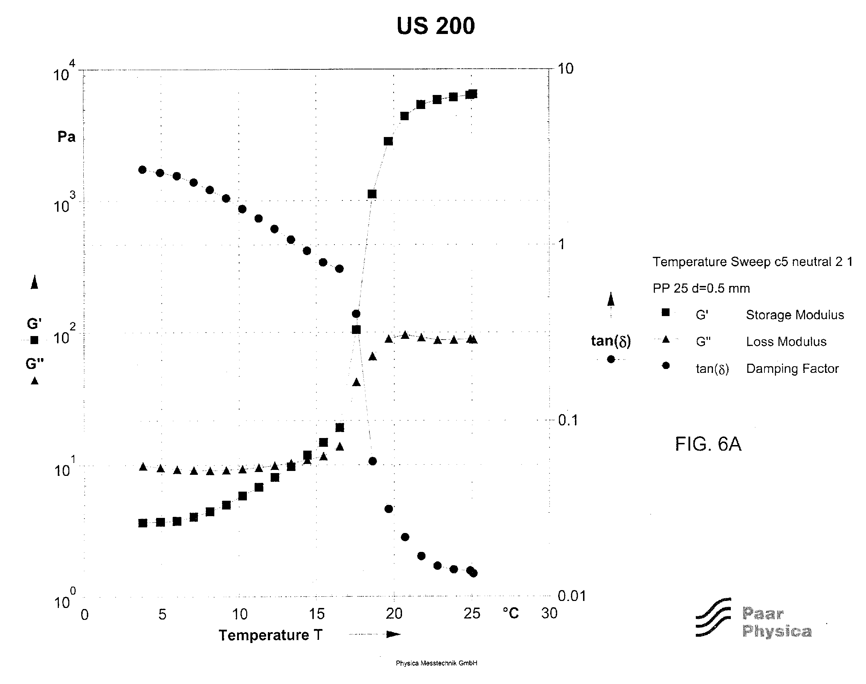

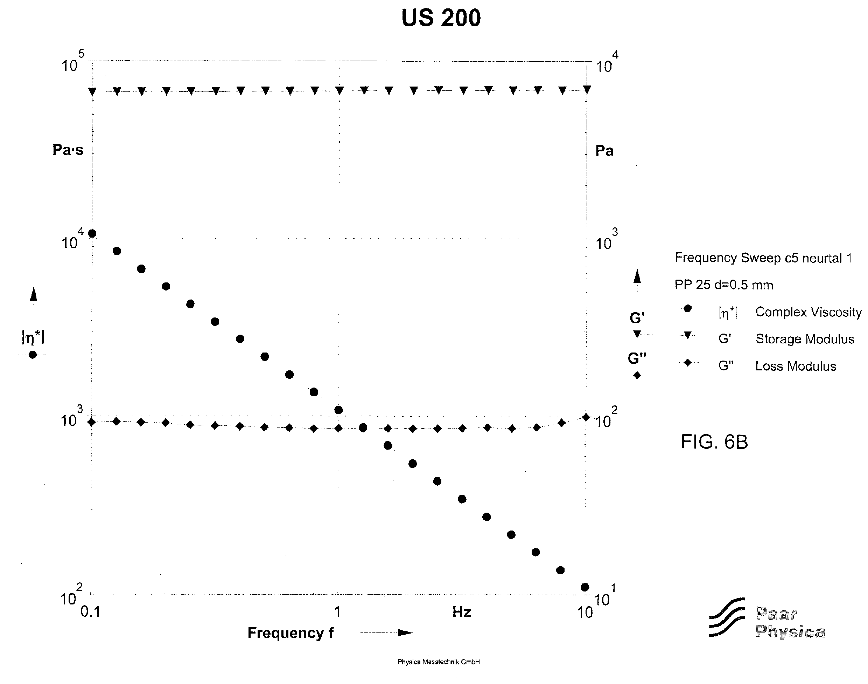

The preparation of the construct leading to copolymer B9 (see Table 1) is analogous to that described in the experimental section of the former publication for copolymer 1 (C5). The B9 clone obtained corresponded to a repetitive gene with a longer central block than that of C5. Proteins B9 and C5 were obtained simultaneously in the same cloning experiment. A pool of concatamers was cloned into the same acceptor plasmid, generating a distribution of clones with the same endblock sizes and sequence but different sizes of the central block. The clones C5 and B9, eponymously referring to each clone and the protein derived from expression of each clone, were selected from the same experiment via screening the inserts via restriction digestion and analyzing on the basis of size of the central block insert; refer to Figure 6 of Wright ER and Conticello VP, 2002 Adv. Drug Deliv. Rev. 54(8):1057-1073).

**Table 1.**

| Structure and molecular weights of protein block copolymers. | | | |
|---|---|---|---|
| **Protein** | **Hydrophilic Block** | **Hydrophobic block** | **M**_{**w**} **(kD)** |
| Plastin (P2) | * | [(IPAVG)₄(VPAVG)] | 72 |
| Plastin-Elastin Triblock (B9) | [(VPGVG)₄(VPGEG)] | [(IPAVG)₄(VPAVG)] | 172 |

| | | | |
|---|---|---|---|
| *The midblock for P2 is VPGVGVPGVG. | | | |

### Example 2. Construction of modular genes encoding elastin-mimetic block copolymers.

An alternative strategy for the construction of modular genes encoding elastin-mimetic block copolymers was devised based on iterative ligation of large DNA cassettes encoding the individual elastin blocks into a suitably modified acceptor plasmid. This approach was based on our previous use of the seamless cloning technique (see McMillan RA, Lee TAT, Conticello VP., 1999, Macromolecules 32: 3643-3648) to generate concatameric genes encoding repetitive polypeptide sequences. We designed three representative DNA monomers based upon elastin-mimetic sequences to achieve properties that would be useful for the construction of self-assembling block copolymer systems. The repeat units of the monomers were defined by the position of the type IIs restriction endonuclease recognition/cleavage sites Bbsl and BsmB I, respectively, in the synthetic DNA monomer. Restriction cleavage of the DNA monomer with these two enzymes generates non-palindromic, complementary cohesive-ended fragments that are competent for self-ligation into a library of concatamers. The concatamers obtained from self-ligation of each DNA monomer have compatible cohesive ends such that they can be joined together to form modular DNA cassettes encoding block copolymers of elastin-mimetic sequences. The order of addition as well as the number of blocks of each sequence was straightforwardly controlled via conventional plasmid-based cloning protocols. A representative example of this procedure is described as follows.

DNA cassettes encoding the respective elastic (S1 and S2) and plastic (S3) repeat units were independently synthesized from annealing of complementary oligonucleotides and inserted into the compatible *HinD* III/*Bam*H I sites within the polylinker of plasmid pZErO-2 (Invitrogen, Inc.). See Table 2 for repeat unit segments. Recombinant clones were isolated after propagation in *E*. *coli* strain Top10F', and the sequences of the inserts were verified by double-stranded automated DNA sequence analysis. These clones were propagated in *E*. *coli* strain Top10F' in order to isolate preparative amounts of plasmid DNA. DNA monomers S1, S2, and S3 were liberated from the respective plasmids via sequential restriction digestion with type IIs *Bbs* I and BsmB I at 37°C and 55°C, respectively. The DNA monomers were purified via preparative agarose gel electrophoresis (2% NuSieve GTG agarose, 1XTBE buffer) and isolated from the gel via the crush and soak method and alcohol precipitation. Self-ligation of each DNA cassette afforded a population of concatemers encoding repeats of the respective monomer sequences. Concatemers were separated by size via agarose gel electrophoresis (1% SeaPlaque agarose, 0.5XTBE buffer). The concatemers were isolated as size fractions with given length ranges (500-1000 bp; 1000-2000 bp; and 2000-3000 bp) by excision of the bands from the gel and extraction and purification using the Zymoclean gel extraction kit (Zymo Research, Inc.). The concentration of DNA within each sample was estimated using the DNA dipstick technique (Invitrogen, Inc.) and usually fell within the range from 1 to 5 nanogram per microliter.

The acceptor plasmid for each monomer corresponded to the original plasmid from which the monomer was obtained. These plasmids were cleaved via restriction digestion with endonuclease Bbs I to generate a construct in which the ends were compatible with the respective concatemer pool. The acceptor plasmids were dephosphorylated with shrimp alkaline phosphatase and purified via gel electrophoresis. The concatemer pools derived from monomers S1, S2, and S3, respectively, were inserted into the corresponding acceptor plasmids via enzymatic ligation as follows. The plasmid (50 nanogram) and concatemers (50 nanogram) were combined with T4 DNA ligase (200 Weiss units) in a total volume of 20 microliters of 1XT4 DNA ligase buffer (New England Biolabs, Inc.). The mixture was incubated at 16°C for 16 hours. An aliquot (5 microliters) of the ligation mixture was used to transform chemically competent cells of *E*. *coli* strain Top10F'. The plasmid DNA from individual clones was isolated via automated miniprep on a MacConnell Miniprep 24. The sizes of the concatmers were analyzed via double restriction digestion with *Hin*D III and *Bam*H I, followed by agarose gel electrophoresis of the restriction fragments (1% SeaPlaque agarose, 0.5XTBE buffer). Representative clones within each size range were selected for further propagation.

Initial experiments were directed toward the synthesis of a gene encoding a diblock elastin-mimetic polypeptide sequence in which the endblock domains were based on the plastic sequence S3 and the central block was derived from the elastic sequence S1. Clones encoding concatemeric DNA cassettes of approximately 2000 base pairs in size were selected for these experiments. The plasmid encoding the first S3 endblock was digested sequentially with the restriction endonucleases *Nco* I and *Bbs* I. The restriction fragments were separated via gel electrophoresis and the fragment containing the S3 concatemer was isolated from the gel and purified as described above. The plasmid containing the central S1 block was sequentially digested with *Nco* I and *Bsm*B I. As described above, the restriction fragments were separated via gel electrophoresis and the fragment containing the S1 concatemer was purified via the ZymoClean gel extraction kit. The two plasmid fragments were joined together via enzymatic ligation at 16 C as described above. Note that the Nco I site cuts within the kanamycin resistance gene on the plasmid and that only productive ligation of the two fragments to give a diblock sequence would result in reconstitution of the antibiotic resistance marker. An aliquot (5 microliters) of the ligation mixture was used to transform competent cells of *E*. *coli* strain Top10F'. Positive transformants were isolated via automated miniprep on a MacConnell miniprep 24 instrument. These clones were screened via restriction digestion with *Bam*H I and *Hin*D III endonucleases to identify clones containing the diblock sequence. The restriction fragments were analyzed via agarose gel electrophoresis and positive clones were selected for propagation. The DNA cassette encoding the diblock polypeptide was excised from the plasmid via sequential restriction digestion with endonucleases *Bbs* I and *Bsm*B I, respectively. The diblock DNA cassette was separated from the cloning plasmid via preparative agarose gel electrophoresis (0.75% SeaPlaque agarose, 0.5XTBE buffer) and isolated via the ZymoClean Gel extraction procedure as described above. This sequence was competent for ligation into the expression plasmid.

The expression vector was constructed from ligation of a short adaptor sequence into the multiple cloning site (MCS) of plasmid pBAD-HisA (Invitrogen, Inc.). The adaptor was synthesized via annealing of complementary oligonucleotides and inserted into the complementary *Nco* I/*Hin*D III sites of plasmid pBAD-HisA. The ligation mixture was used to transform *E*. *coli* strain Top10F'. The resulting transformants were analyzed via restriction digestion with *Nco* I and *Hin*D III followed by agarose gel electrophoresis (2% NuSieve GTG agarose, 1XTBE buffer). Positive clones were analyzed via double-stranded DNA sequence determination. A clone with the correct adaptor sequence was selected for further propagation. The modified expression plasmid was cleaved within the synthetic adaptor sequence with the restriction endonuclease Bbs I to generate a vector compatible with the diblock DNA cassette. The expression vector was dephosphorylated with shrimp alkaline phosphatase and purified via agarose gel electrophoresis as described previously. A ligation reaction between the diblock DNA cassette and the expression vector was performed as described above. An aliquot of the ligation mixture was used to transform competent cells of *E*. *coli* strain Top10F'. Transformants were analyzed via restriction digestion with *Nco* I and *HinD* III, followed by agarose gel electrophoresis (0.75% SeaPlaque agarose, 0.5XTBE buffer). The sequence of the expression cassette was verified via double-stranded DNA sequence determination. A positive clone was obtained that was used to transform *E*. *coli* expression strain LMG194.

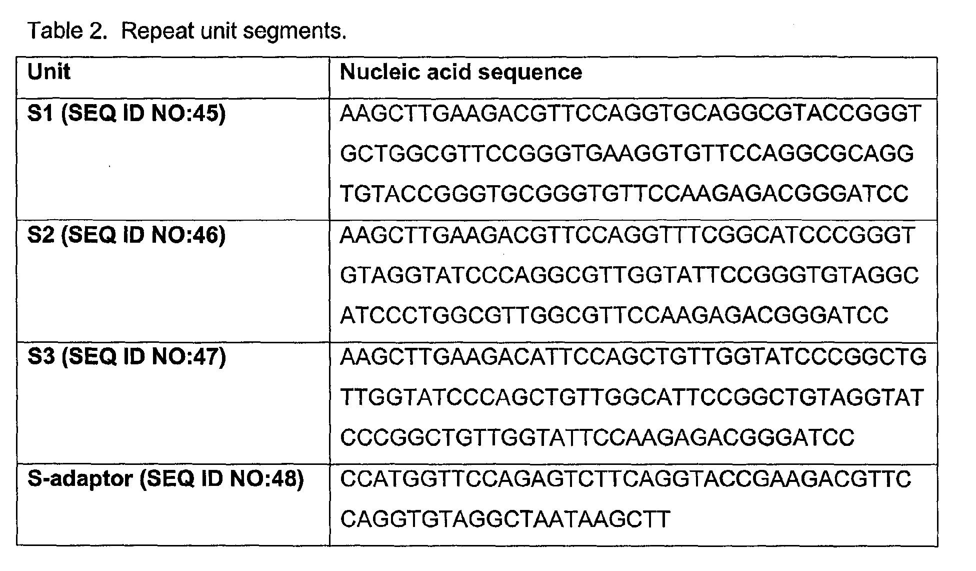

### Example 3. Study A of triblock copolmers.

Recombinant DNA methods were used to synthesize a protein triblock copolymer with flanking hydrophobic endblocks [(IPAVG)₄(VPAVG)] and a hydrophilic midblock [(VPGVG)₄(VPGEG)] that mimics the characteristic behavior of synthetic thermoplastic elastomers. An array of protein-based materials were produced that exhibit a wide range of mechanical properties, including changes of greater than two orders of magnitude in Young's modulus (from 0.03 to 5 MPa) and greater than five-fold in elongation to break (from 2.5 fold to 13 fold), by judicious selection of solvent, temperature, and pH during film casting. For example, hydrated protein films cast from trifluoroethanol (TFE) generally demonstrated plastic deformation behavior while those cast from water were generally elastomeric. Changes in mechanical behavior were attributed to the production of either a mixed or diffuse interface between protein domains when the triblock copolymer was cast from TFE, which acts as a good solvent for both blocks, or a sharp interface when cast from water that preferentially solvates the hydrophilic midblock. Water-cast samples exhibited less hysteresis with a tan delta value that was an order of magnitude lower than that observed for TFE-cast films. Remarkably, the elastomeric characteristics of water-cast samples were significantly enhanced by increasing the temperature of film casting from 5°C to 23°C, presumably due to true microphase separation between the hydrophilic and hydrophobic blocks. Moreover, increasing the pH of the casting solvent to basic conditions, whereby glutamic acid residues in the midblock were ionized, further augmented the elastic nature of the material with an observed strain to failure in water exceeding 13-fold. Akin to synthetic triblock copolymers, protein-based counterparts can be produced with tailored mechanical properties at physiologically relevant conditions, through the rational choice of post-production conditions such as processing solvent, temperature, and pH.

Within the pentapeptide repeat sequence [(Val/Ile)-Pro-Xaa-Yaa-Gly], alterations in the identity of the fourth residue (Yaa) modulate the position of the inverse temperature transition of the polypeptide in aqueous solution in a manner commensurate with the effect of the polarity of the amino acid side chain on polymer-solvent interactions. In addition, substitution of an Ala residue for the consensus Gly residue in the third (Xaa) position of the repeat results in a change in the mechanical response of the material from elastic to plastic deformation. The macroscopic effects of these sequence alterations were employed to design elastin-mimetic polypeptide sequences that mimic synthetic amphiphilic triblock copolymers (ABA). Specifically, these polypeptides incorporate identical endblocks of a hydrophobic plastic sequence (VPAVG) separated by a central hydrophilic elastomeric block (VPGVG); see Table 1.

Above an inverse temperature transition *T*_{*t*}, the block polypeptide undergoes reversible microscopic phase separation from aqueous solution, likely due to the formation of virtual crosslinks between the terminal blocks. In the process a thermoplastic elastomer biosolid is generated. The repeat sequence of the endblocks was chosen such that their inverse temperature transition would reside at or near ambient temperature, which would result in phase separation of the hydrophobic domains from aqueous solution under physiologically relevant conditions. In turn, the sequences of the central elastomeric repeat units were chosen such that their transition temperature was significantly higher than 37°C. These protein polymers reversibly self-assemble from concentrated aqueous solution above the phase transition of the hydrophobic endblocks to form a network of microdomains dispersed in a continuous phase of the elastomeric midblock and aqueous solvent.

Differential scanning calorimetry of the triblock copolymer in dilute aqueous solution confirmed the existence of a reversible endothermic transition at 22°C. The transition temperatures and the enthalpies of transition of the homopolymer, comprised entirely of the endblock, and the triblock were virtually identical indicating that the aggregation phenomenon observed on increasing the temperature likely involves only the hydrophobic domains. Moreover, temperature-dependent ¹H-¹³C HMQC NMR spectra demonstrated that only those cross-peaks associated with the plastic endblocks disappeared at temperatures above the phase transition (Wright ER et al., 2002, Adv. Funct. Mater. 12:149-154). These data suggested that in an aqueous environment at 23°C the endblocks undergo a selective hydrophobic collapse with the formation of virtual crosslinks, while in contrast, the elastin internal block is hydrated and remains conformationally flexible. Presumably, aggregation proceeds through selective desolvation of endblock sequences, which induces microphase separation of the blocks. Consistent with these observations, initial rheological measurements displayed a crossover between the storage modulus and loss modulus near the calorimetric phase transition, indicating onset of gelation (Wright ER et al., 2002, Adv. Funct. Mater. 12:149-154). Changing solvent systems from H2O (distilled/deionized; ddH2O) to TFE precluded the occurrence of such a hydrophobic collapse, demonstrating that TFE is a good solvent for both the blocks in the temperature range investigated.

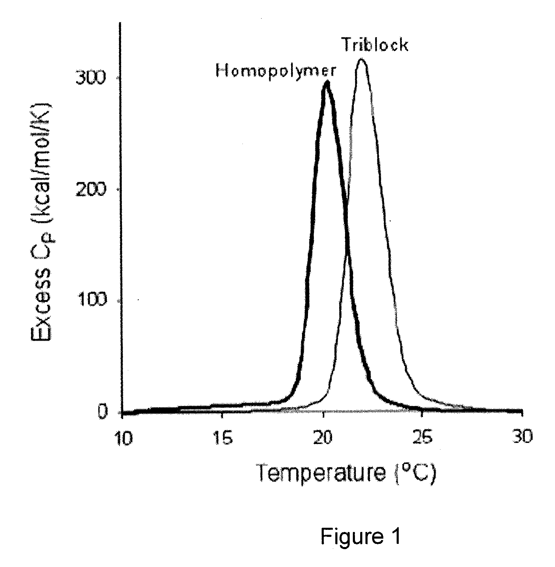

Figure 1 shows differential scanning calorimetry thermograms of P2 and B9 in ddH20. The P2 homopolymer has a transition temperature of 20 degrees C, while the B9 triblock copolymer has a transition temperature of 22°C. Computed enthalpies of both samples are similar, suggesting that only the hydrophobic end blocks are participating in the B9 aggregation process.

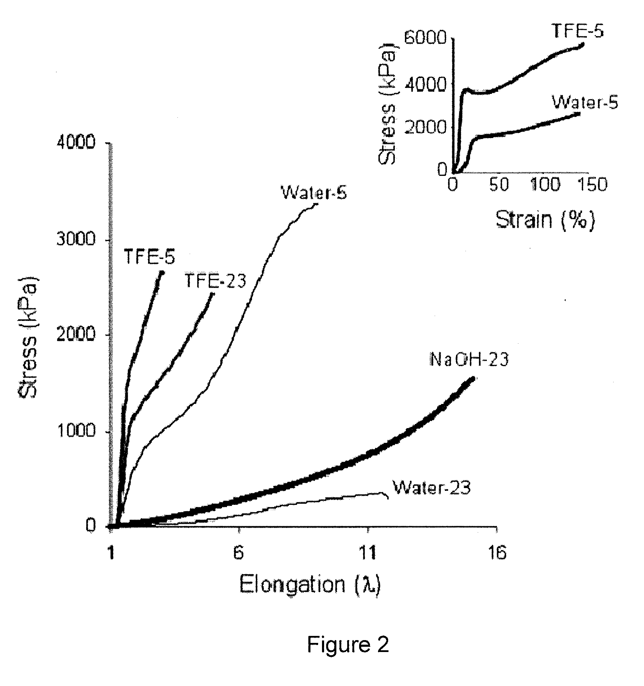

The characteristic engineering stress-strain curves for the homopolymer P2 (shown as inset) and triblock B9 samples cast from TFE and water are shown in Figure 2. For this experiment, all samples were rehydrated in phosphate buffered saline (PBS) for 24 hours prior to testing. The strain rate was 5 mm/min. The samples had dimensions of 5 mm by 5 mm by a variable amount. Changing the pH of the solvent by addition of NaOH enhanced the elastic properties. The inset illustrates the tensile behavior of P2 cast from water and TFE. P2 displayed a yield point and subsequent plastic deformation. Elongation lambda represents strained length divided by initial length (I/Io).

The corresponding tensile data are summarized in Table 3. P2 displays a tensile behavior similar to materials exhibiting plastic or irrecoverable deformation under stress. The modulus of hydrated P2 films in the range of 16-55 MPa, is consistent with similar values reported for P2-like materials in literature (Urry, D. W., Luan, C.-H., Harris, C. M., & Parker, T. M. in Protein-Based Materials (eds McGrath, K., & Kaplan, D.) Ch. 5 (Birkhäuser, Boston, 1997).

**Table 3.**

| Tensile Data for Films. | | | | | | | |
|---|---|---|---|---|---|---|---|
| Protein | Solvent and Temperature, degrees C | Modulus, MPa | Variation | Tensile strength, Mpa | Variation | Elongation to Break (%); strain to failure | Variation |
| P2 | water, 5 | 16.65 | 1.55 | 3.00 | 0.32 | 128 | 33 |
| P2 | TFE, 5 | 55.04 | 11.89 | 5.59 | 1.01 | 152 | 42 |
| B9 | TFE, 5 | 4.03 | 1.30 | 2.70 | 0.40 | 390 | 53 |
| B9 | TFE, 23 | 4.90 | 0.92 | 2.28 | 0.58 | 250 | 47 |
| B9 | water, 5 | 0.93 | 0.06 | 2.87 | 0.88 | 640 | 116 |
| B9 | water, 23 | 0.03 | 0.01 | 0.78 | 0.28 | 1084 | 67 |
| B9 | NaOH, 23 | 0.03 | 0.006 | 1.24 | 0.29 | 1330 | 64 |

Since TFE is a good solvent for both hydrophobic and hydrophilic blocks of B9, film casting from TFE is expected to produce a material with significant interpenetration of both blocks and, as a consequence, a mixed interface between both domains on solvent removal. Although subsequent rehydration in PBS would induce endblock aggregation and consequent phase separation, some portion of the endblock would remain kinetically trapped within the soft midblock segments. This interface component will translate into a material that shows plastic deformation under stress. Indeed, the stress-strain curve of B9 is qualitatively similar to that of P2, when both are cast from TFE. The behavior of B9 can thus be likened to a rubber-toughened version of P2. Tensile properties of B9 films cast from TFE at 5°C and 23°C remained similar since TFE is a good solvent for B9 at both temperatures.

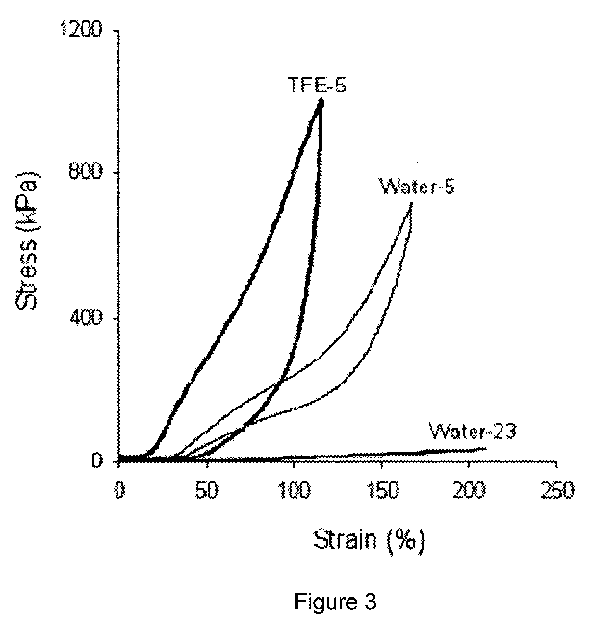

Unlike TFE, water preferentially solvated the hydrophilic midblock than the more hydrophobic endblocks. Thus, limited mixing at the interface of these two domains would be anticipated in films cast from water at 5°C. Subsequent rehydration in PBS thus produced a material with a sharper interface than for films cast from TFE. In turn, this reduced the participation of the endblocks in the tensile deformation process, thereby enhancing material elasticity as is noted in Figure 2. The tensile behavior shown by water-cast samples is similar to that observed for SBS copolymers cast from solvents that preferentially solvate the butadiene block See Séguéla, R., & Prud'homme, J., Macromolecules 11, 1007-1016 (1978); Wilkes, G. L., Bagrodia, S., Ophir, Z., & Emerson, J., J. Appl. Phys. 49, 5060-5067 (1978). Increasing the solvent casting temperature to 23°C, further favors end block aggregation, since this temperature lies above the transition temperature of the endblock. The likely result is a true microphase separated material in which there are islands of hydrophobic endblocks dispersed among solvent swollen and conformationally-labile midblock chains. Owing to almost complete non-participation of the endblocks in the tensile deformation process this material is truly elastic with an observed elongation to break on the order of 10-fold. As shown in Figure 3, hysteresis is significantly greater in TFE-cast samples as compared to the more elastomeric water-cast films produced at 5 and 23°C.

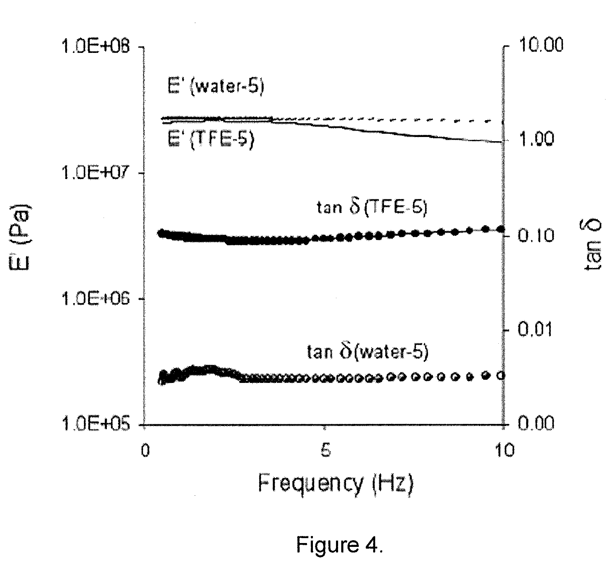

Dynamic mechanical testing provided further insight into mechanical property differences observed as a function of film processing conditions. Figure 4 shows dynamic mechanical data for copolymer B9 cast from water or TFE at 5°C. While the elastic moduli of the TFE- and water-cast samples are similar, the tan delta for the water-cast sample is an order of magnitude lower than that of the TFE-cast sample. Observed losses due to viscous dissipation are much lower in the water-cast sample providing further support to the observed change in elastic behavior.

Since the midblock contains glutamic acid residues, further mechanical modulation of the triblock can be expected with a change in pH. Film samples of B9 cast from a 0.1 N NaOH solution (pH 14) at 23°C and subsequently rehydrated in PBS proved to be more elastic than comparable samples cast from water at 23°C (Fig. 2 and Table 3). In NaOH solution the glutamic acid residue will reside in an ionized state (COO⁻) since their pKa is ∼ 4, which likely contributes to a greater degree of midblock swelling due to charge-charge repulsions. Indeed, the loss modulus for samples cast from NaOH was lower than that observed for water-cast samples (data not shown), demonstrating a further augmentation of elastic behavior. Remarkably, under these conditions maximum elongation to break exceeded 13-fold, which is a value two to three times greater than previously observed among other recombinant elastomeric biosolids (Urry D. W. et al., 1976, Biochemistry 15, 4083-4089), and commensurate with values associated with synthetically produced elastomers (Legge, N. R., Holden, G., & Schroeder, H. E., eds., Thermoplastic Elastomers, Hanser, New York, 1987).

Protein-based triblock materials derived from a consideration of elastin consensus peptide sequences offer several advantages when compared with synthetic triblock copolymers including the capacity for: (i) precise control of molecular architecture, (ii) solvent and pH-tunable morphologies and mechanical properties at physiologically relevant conditions, and (iii) opportunities for bioconjugation through incorporation of reactive side chains. Moreover, using traditional polymer techniques, blends of P2 and B9 yield further changes in material structure and properties that facilitate materials for applications in controlled release, cell and drug encapsulation, as well as scaffolds for tissue engineering.

For copolymer synthesis, genetic engineering methods were employed to synthesize polypeptides with predetermined compositions and precisely controlled molecular architectures, as detailed in McMillan, R. A. et al., 1999, Macromolecules 32, 3643-3648; and Lee, T. A. T., et al., 2000, Adv. Mater. 12, 1105-1110. Briefly, oligonucleotide cassettes encoding the elastic and plastic repeat units were independently synthesized and inserted into plasmids engineered for DNA modifications (pZErO 1 and pZErO 2). Monomers with the correct sequence were purified on a large scale via restriction endonuclease digestion, concatemerized to yield large repetitive genes (containing 1200 to 3000 base pairs), and enzymatically ligated into an expression plasmid (pET24a). Plasmids encoding proteins of suitable sizes and correct sequences were transformed to an E. coli strain (BL21(Gold)DE3). The proteins were expressed and purified in multigram yields using a hyperexpression protocol (Daniell, H., et al., 1997, Methods Mol. Biol. 63, 359).

For Differential Scanning Calorimetry, measurements were conducted on a CSC Nano II Differential Scanning Calorimeter (N-DSC II) at a protein concentration of 1 mg/mL and a scan rate of 1°C/min. Distilled deionized water was used as a solvent in all cases. The thermograms were corrected for instrumental baseline by obtaining the DSC trace of the pure solvent. The transition temperatures and heats of transition were computed using the cpcalc analysis software provided with the instrument.

For tensile measurements, films of the homopolymer and the triblock copolymer were cast from 10 wt% solutions in TFE and water. Although protein solutions were prepared at 5°C, solvent evaporation was performed either at 5°C or at 23°C. Since fluorinated alcohols form strong solid-state complexes with polyamides (Sturgill, G. K., et al., 2001, Macromolecules 34, 8730-8734), TGA analysis was conducted to verify complete removal of solvent from films samples. After complete solvent evaporation, films were hydrated in a phosphate buffer saline (pH 7.4), cut into 5mm x 15mm strips for tensile analysis, and stored in PBS for a further 24 hours prior to tensile testing. Hydrated film thickness was measured by optical microscopy using a standard image analysis protocol.

A miniature materials tester Minimat 2000 (Rheometric Scientific) was used to determine the mechanical film properties in the tensile deformation mode with a 20 N load cell, a strain rate of 5 mm/min, and a gauge length of 5 mm. Eight to ten specimens were tested and average Young's modulus, tensile strength, and elongation to break were determined. Hysteresis measurements were performed at a strain rate of 5 mm/min in both the loading and unloading directions.

Rheological data was measured using a DMTA V (Rheometric Scientific) in the tensile mode. The data was collected with samples immersed in PBS at 23°C. Storage modulus (E'), loss modulus (E"), and tan delta were measured at a strain of 0.5% in the frequency range of 0.5 to 10 Hz.

### Example 4. Synthesis of Recombinant Protein Polymers

The biosynthesis of protein polymers requires the construction of large synthetic genes encoding tandem repeats of target oligopeptide sequences. The most common procedure involves synthesis of double-stranded oligonucleotide cassettes or DNA "monomers" that contain nonpalindromic cohesive ends. These DNA monomers are oligomerized exclusively head-to-tail by enzymatic ligation such that only the sense or coding strand is translated into polypeptide. The DNA concatamers are fractionated, enzymatically joined to an expression vector, and transformed into a suitable expression host under inducible control of a strong promoter. Despite the successful production of numerous synthetic protein polymers, the aforementioned approach has several drawbacks. The most significant disadvantage is the stringent dependence on a limited pool of restriction endonucleases recognizing nonpalindromic cleavage sites. These endonucleases are necessary for the generation of DNA monomers that undergo self-ligation in the correct head-to-tail orientation. Often superfluous amino acid residues are introduced into the repeat sequence of the target polypeptide as a consequence of this requirement. In addition, such endonucleases can have relaxed specificities in recognition sequences, which increase the likelihood of cleavage of internal sites in expression plasmids. Usually, one or more additional cloning steps are required in the gene assembly to prevent this process.

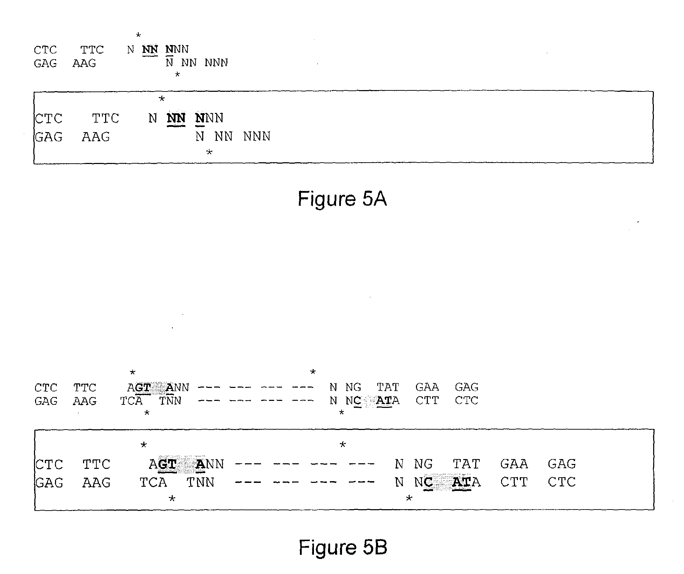

Conticello et al. have recently reported an efficient method for the rapid assembly of synthetic genes encoding repetitive polypeptides and their direct cloning into expression vectors by an extension of the seamless cloning technique (McMillan RA, Lee TAT, Conticello VP., Macromolecules 1999;32:3643-8). The approach allows the ability to clone a given DNA sequence into a desired location without the usual limitation of naturally occurring restriction sites. Briefly, the efficacy of the seamless cloning procedure resides in two specific properties of the type IIs restriction endonuclease Eam1104 I (Figure 5A and Figure 5B). (Figure 5A shows the recognition and cleavage sequence of restriction enzyme Eam 1104 I; in one strand N represents any of the 4 nucleotides. Figure 5B shows a schematic representation of the cleavage pattern for a synthetic DNA duplex flanked by inverted Eam 1104 I recognition sites, which generates complementary 5' cohesive ends having the sequence of the valine codon GTA. The top strand of the duplex is oriented with the 5' to 3' direction proceeding from left to right in the sequence.)

One property is the ability of this endonuclease to cleave a DNA duplex at a specific position downstream of its recognition site (5'-CTCTTC). The second is the ability to inhibit cleavage by incorporation of 5-methyldeoxycytosine into the recognition site of the enzyme.

Cleavage of synthetic duplexes with Eam1104 I generates 5' cohesive ends in which the identity of the three base overhangs is independent of the recognition site. This general procedure can produce any triplet sequence at the 5'-termini of the duplex, which avoids the need for an array of endonucleases with unique internal recognition/cleavage patterns. Moreover, the Eam1104 I restriction sites are cleaved from the DNA cassette and, hence, are not incorporated into the coding sequence of the DNA monomer. Synthetic duplexes flanked by inverted Eam1104 I recognition sites are enzymatically cleaved to generate ligation-competent DNA monomers with nonpalindromic, complementary cohesive ends. These monomers can be enzymatically joined head-to-tail to generate concatamer libraries with seamless junctions.

The ability to inhibit cleavage of the Eam1104 I recognition site by incorporation of 5-methyldeoxy-cytosine is a second feature of this endonuclease that typifies the seamless cloning procedure and facilitates the insertion of concatameric genes directly into the cloning site of an expression plasmid. Synthetic primers are used to direct the amplification of an appropriate expression plasmid using the inverse polymerase chain reaction (PCR) process. These primers anneal to opposite strands of the plasmid such that their 3' termini are outwardly oriented on the circular map. Amplification from these primers affords a linear plasmid. The Eam1104 I recognition sites in the primers are incorporated into the termini of the plasmid upon amplification. If the PCR process is performed in the presence of 5-methyldeoxycytosine, internal Eam1104 I recognition sites in the expression plasmid are protected from enzymatic cleavage, but the terminal sites derived from the primers are not. After PCR amplification, the purified, amplified plasmid is incubated with Eam1104 I, which cleaves primarily at the terminal sites. The primers are chosen such that the cohesive ends that are generated by Eam1104 I cleavage of the amplified plasmid are complementary to those of the multimers. In addition, the nucleotide sequences of the primers are designed such that insertion of the concatamers occurs in the correct reading frame for expression of the desired protein polymer. This method can generate large synthetic genes (> 3000 bp) that encode repetitive polypeptides.

### Example 5. Polypeptide Multi-Block Copolymers Comprised of Elastomeric and Plastic Sequences.

It has been known for over two decades that a variety of AB-diblock or ABA-triblock copolymers aggregate into microdomains when mixed with a solvent that dissolves the A-blocks, but is incompatible with the B-block. On producing BAB-block copolymers, however, a different aggregation behavior occurs. In this situation the solvent is specific for the A-block only and the resulting structure consists of a network of insoluble end blocks that act as virtual or physical crosslinks connecting solvent-swollen central blocks. Triblock copolymers of this type, such as the thermoplastic elastomer, styrene-butadiene-styrene (SBS), traditionally have been derived from conventional organic monomers. However, the synthetic repertoire of these materials has been limited to tapered blocks of uniform sequence, which potentially restricts the functional complexity of the resulting microstructures. Genetic engineering of synthetic polypeptides enables preparation of block copolymers composed of complex block sequences in which the individual blocks may have different mechanical, chemical, and biological properties. The segregation of the protein blocks into compositionally, structurally, and spatially distinct domains should occur in analogy with synthetic block copolymers, affording ordered structures on the nanometer to micrometer size range. The utility of these protein materials depends on the ability to functionally emulate the materials properties of conventional polymer systems, while retaining the benefits of greater control over the sequence and microstructure that protein engineering affords for the construction of materials.

Conticello et al. have recently reported the genetically directed synthesis and characterization of a class of triblock copolymers that are derived from elastin-mimetic polypeptide sequences in which the respective blocks exhibit different mechanical properties in analogy to thermoplastic elastomers (Lee TAT, Cooper A, Apkarian RP, Conticello VP, Adv Mater 2000;12:1105-10). As discussed elsewhere, the phase behavior and mechanical properties of elastin-mimetic polypeptides depend on the identity of the residues within the pentapeptide repeat sequence [(Val/Ile)-Pro-Xaa-Yaa-Gly] (Urry DW, Pattanaik A, Accavitti MA, Luan CX, McPherson DT, Xu J, et al. Transductional elastic and plastic protein-based polymers as potential medical devices. In: Domb AJ, Kost J, Wiseman DM, editors. Handbook of Biodegradable Polymers. Amsterdam: Harwood; 1997. p. 367-86).

Alterations in the identity of the fourth residue (Yaa) modulates the position of the lower critical solution temperature of the polypeptide in aqueous solution in a manner commensurate with the effect of the polarity of the amino acid side chain on polymer-solvent interactions. In addition, substitution of an Ala residue for the consensus Gly residue in the third (Xaa) position of the repeat results in a change in the mechanical response of the material from elastomeric to plastic. The macroscopic effects of these sequence alterations were employed to design elastin-mimetic polypeptide sequences, copolymers 1, 2, and 3, that mimic triblock copolymers (BAB). Specifically, these polypeptides incorporate identical endblocks of a hydrophobic plastic sequence separated by a central hydrophilic elastomeric block, where a polypeptide has a structure as follows:
{VPAVG[(IPAVG)₄(VPAVG)]₁₆IPAVG}-[X]-{VPAVG[(IPAVG)₄(VPAVG)]₁₆IPAVG}; (cross-references to {SEQ ID NO:50-[X]-SEQ ID NO:50}; and SEQ ID NO:51). In such structures, [X] is optionally selected from the following structures:
1: [X] = VPGVG[(VPGVG)₂VPGEG(VPGVG)₂]₃₀VPGVG; (cross-reference to SEQ ID NO:23);
2: [X] = VPGVG[(VPGVG)₂VPGEG(VPGVG)₂]₄₈VPGVG; (cross reference to SEQ ID NO: 25); or
3: [X] = VPGVG[(APGGVPGGAPGG)₂]₃₀VPGVG (cross reference to SEQ ID NO:35).

Above a lower critical solution temperature Tt, the polypeptides undergo reversible microscopic phase separation from aqueous solution due to the formation of virtual crosslinks between the terminal blocks. In the process a thermoplastic elastomer biosolid is generated. The repeat sequence of the end blocks (B) was chosen such that their lower critical solution temperature would reside at or near ambient temperature, which would result in phase separation of the plastic domains from aqueous solution under physiologically relevant conditions. In turn, the sequences of the central elastomeric repeat units were chosen such their phase transition was significantly higher than 37°C. These protein polymers reversibly self-assemble from concentrated aqueous solution above the phase transition of the hydrophobic endblocks to form a network of plastic microdomains dispersed in a continuous phase of the elastomeric midblock and aqueous solvent. Of note, other investigators have demonstrated that the central block sequence is poorly adhesive towards cells and proteins when below its transition temperature.

Synthetic methods used to produce the DNA inserts that encode the various elastin block copolymers have been described previously and are summarized using the preparation of the gene encoding polypeptides 1 as an example. Oligonucleotide cassettes encoding the plastic and elastic repeat units were independently synthesized and inserted into the BamH I/HinD III sites within the polylinkers of pZErO-1 and pZErO-2, respectively. DNA monomers were liberated from the respective plasmids via restriction digestion with BspM I and SexA I, respectively. Self-ligation of each DNA cassette afforded a population of concatamers encoding repeats of the plastic and elastic sequences, respectively. A concatamer encoding sixteen repeats of the plastic sequence was isolated and a pair of recombinant plasmids, pPN and pPC, encoded the N-terminal and C-terminal domains of polymer 1, respectively. Restriction cleavage of each plasmid afforded two fragments, which were separated via preparative agarose gel electrophoresis and ligated into a recombinant plasmid (pPEP) as a single contiguous reading frame. Plasmid pPEP was propagated in E. coli strain SCS110 and cleaved with restriction endonuclease SexA I. Concatamers encoding the elastin sequence were inserted into the compatible SexA I site of pPEP. A clone was isolated that encoded approximately thirty repeats of the elastic sequence. Following protein expression, dialysis and lyophilization afforded protein 1 in an isolated yield of 614 mg/L of culture. SDS-PAGE analysis indicated apparent molar masses of approximately 150 kDa and the structure of the protein was confirmed via a combination of amino acid compositional analysis, MALDI-TOF mass spectrometry, and multi-dimensional NMR spectroscopy. Notably, this procedure can be readily adapted to prepare genes that encode polypeptides with central blocks of altered sizes and sequences. The preparation of polypeptides 2 and 3 provided evidence of the flexibility of this approach.

Protein polymer 1 illustrated the capacity of these triblock polypeptides to reversibly self-assemble into a thermoplastic elastomer. Differential scanning calorimetry of 1 in dilute aqueous solution indicated a reversible endothermic transition at 23°C. In this regard, temperature-dependent ¹H-¹³C HMQC NMR spectra demonstrated that only those cross-peaks associated with the plastic endblocks disappeared at temperatures above the phase transition. These data suggest that in an aqueous environment at 37°C plastic endblocks undergo a selective hydrophobic collapse with the formation of virtual crosslinks, while in contrast, the elastin internal block is hydrated and remains conformationally flexible. Consistent with these observations, rheological measurements demonstrated that the dynamic mechanical moduli G' and G" depended strongly on temperature (Figure 6). Figure 6 shows the rheological behavior of a concentrated aqueous solution of copolymer 1 (25 wt%) as a function of temperature. Inset shows a frequency sweep in the linear viscoelastic regime at 25°C. Temperature sweeps display a crossover between the storage modulus and loss modulus near the calorimetric phase transition. At 25°C, the rheological properties are consistent with a viscoelastic solid, particularly given that the values of the storage modulus and loss modulus differ by approximately two orders of magnitude.

Temperature sweeps displayed a crossover between the storage modulus and loss modulus near the calorimetric phase transition.

### Example 6. Fabrication of Protein Fibers, Fiber Networks, and Films.

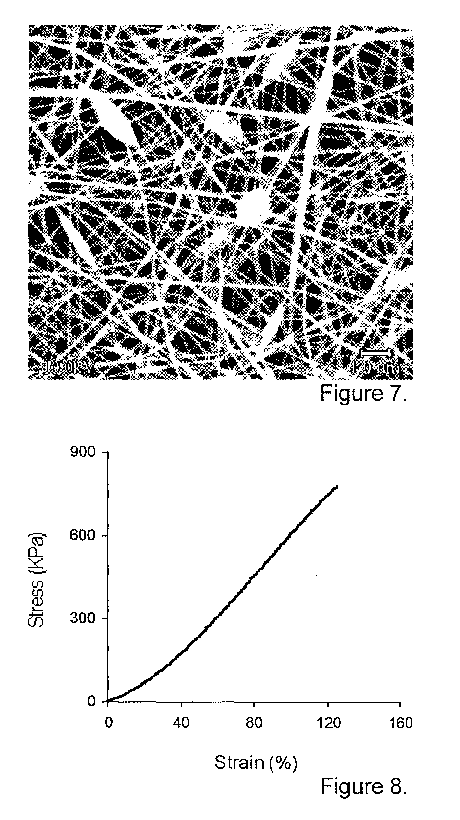

Elastomeric triblock copolymer 1 (from Example 5) was dissolved in trifluorethanol (TFE) at 5 wt% and fibers generated by electrospinning. Figure 7 shows an image at 5000x magnification from scanning electron microscopy (SEM) of the triblock copolymer 2 spun from a 5 wt% solution in TFE; electrospinning parameters were: voltage, 18kV; flow rate, 50 microliters/min. Uniform submicron diameter fibers were produced.

Mechanical properties of dry and hydrated fiber network samples were evaluated at room temperature by uniaxial stress-strain testing (strain rate 1 mm/min). Figure 8 shows stress-strain curves for hydrated fabric sample of the triblock copolymer 2 (strain rate: 1 mm/min, length: 8 mm. UTS 0.64 MPa; elastic modulus 0.56 MPa). Dry samples had a tensile strength of 16.39 MPa, an elastic modulus of 1.15 MPa, and a strain to failure of 20%. Upon hydration the sample exhibited greater compliance and an enhanced strain to failure. Specifically, hydrated samples had a tensile strength of 0.64 ± 0.15 MPa, an elastic modulus of 0.56 ± 0.07 MPa, and a strain to failure of 151 ± 29%. These values are comparable to those obtained for native bovine ligamentum nuchae elastin and the elastin component of the arterial wall (Young's modulus ∼ 0.3 MPa) (Urry DW. Protein elasticity based on the conformation of sequential polypeptides: The biological elastic fiber. J Protein Chem 1984;3:403-36; Niklason LE, Gao J, Abbott WM, Hirschi KK, Houser S, Marini R, et al. Functional arteries grown in vitro. Science 1999;284:489-93).

Protein fibers and fiber networks are produced by electrospinning, as known in the art and disclosed herein, solutions of recombinant peptide polymers containing sites that are capable of forming true or virtual crosslinks. Scanning electron microscopy (SEM) demonstrates that fiber morphology is primarily influenced by solution concentration and flow rate and solid-state NMR confirms the efficacy of photocrosslinking.

The ability to synthesize recombinant triblock elastin analogues that have the capacity to form stable fibers with a reduced requirement for chemical crosslinking provides a complementary approach that helps to generate robust elastomeric fiber networks. Moreover, in forming well-defined microphase separated systems, triblock copolymers also allow one to "cluster" bioactive sequences into high-density regions by engineering the target sequence(s) into the central elastomeric block. A variety of structural features and mechanical properties of single protein fibers and fiber networks formulated as non-woven fabrics are made.

The assembly of fiber-reinforced biocomposites was used to generate model systems for studying the relationship between microscale properties and the mechanical responses of protein based constructs, yielding knowledge about the influence of attributes, such as composition, content, and organization, of substituents (such as collagen and elastin or respective mimetics) on construct mechanical properties.

### Example 7.

Elastin-mimetic fibers are produced with tailored elastomeric properties and enhanced biostability through appropriate choice of recombinant peptide sequences that facilitate crosslink formation. Collagen-mimetic fibers that are biologically stable and of high tensile strength are generated by minimizing the loss of native tertiary molecular structure during fiber processing and crosslink formation.

Features of primary protein structure influence the morphological and physiochemical properties of recombinant fiber analogues. These factors allow generation of fiber networks that are both mechanically resilient and optimally resist degradation processes. Recombinant proteins containing repeating elastomeric peptide sequences are produced by genetic engineering and microbial protein expression. Four classes of elastin analogues are able to form either true and/or virtual crosslinks.

The first class (Type I) consists of elastin analogues capable of undergoing covalent crosslinking. Recombinant proteins are synthesized based upon the elastin-mimetic sequence (VPGVG)n(VPGKG), which contains lysine (K) residues available for methacrylate derivatization.

The second class (Type II) is comprised of elastomeric materials capable of forming virtual crosslinks. Protein polymer triblocks are provided based on the sequence (VPAVG[(IPAVG)₄(VPAVG)]₁₆IPAVG)-[X]ₘ-(VPAVG[(IPAVG)₄(VPAVG)]₁₆IPAVG); (cross reference to SEQ ID NO:51). This class of triblock copolymer is comprised of a central hydrophilic elastomeric block (E) of tailorable identity (X) and two hydrophobic "plastic" end-blocks (P). This class (Type II) of polymer is designated herein as P-E-P elastomeric triblocks.

Another class (Type III) of elastin-mimetic protein-based material is designed to contain both virtual and true crosslinks. As such, a sequence of ten lysine units, available for methacrylate derivatization, is added to the N and C terminal segments of P-E-P elastomeric triblocks. The addition of terminal covalent crosslink sites provides a useful mechanism for modulating the mechanical properties and enhancing the biostability of elastomeric triblock copolymers. The DNA cassettes encoding the triblock polypeptides are cloned via polymerase chain reaction as a Nde I/Xho I fragment into a version of plasmid pET-24a that has been modified with a polylinker that encodes the appropriate number of Lys residues upstream and downstream of the insertion site. The entire construct constitutes a single, contiguous coding sequence in the appropriate reading frame for expression of the target gene encoding the lysine-terminated triblock copolymer. Among the classes of elastin-mimetic protein polymer, a series of related recombinants is produced to characterize the effect of modulating molecular weight, as well as crosslink type, density, and position.

Another class (Type IV) of elastin analogue is synthesized in which a fibronectin (FN) binding sequence is inserted into the central elastomeric block of a Type III protein polymer selected, such that its mechanical properties approach those reported for native elastin. The presence of FN binding sequences facilitates the adsorption of fibronectin onto the surface of elastin-mimetic fibers. In the process, all cell-binding sites (e.g. RGD, synergy, and heparin binding sequences) are available for migrating and proliferating vascular wall cells that are repopulating the construct. In generating this material, microphase separated elastin analogues assemble inserted FN-binding sequences into discrete domains. In turn, by organizing all crosslinks into regions that are located outside of these binding sites, the interference of network crosslinks with the recognition of these sequences by fibronectin is limited. Significantly, a generic approach is established for the facile incorporation of a variety of bioactive recognition sequences into elastomeric materials while preserving previously optimized mechanical properties. This approach has design advantages as compared with strategies based on interspersing "binding sites" within the initial DNA monomer or cassette that provides the basic repeat sequence of the entire protein polymer (e.g. monomer of a Type I elastin analogue). Using the latter strategy, the potential for disrupting previously optimized mechanical properties is significant.

Two fibronectin binding sites (FN1 and FN2 below) are investigated based on peptides derived from collagen that have been shown to have a high binding affinity (KD ∼ 10⁻⁷-10⁻¹⁰ M) for human plasma fibronectin (Gao X, Groves MJ. Fibronectin-binding peptides. I. Isolation and characterization of two unique fibronectin-binding peptides from gelatin. Eur J Pharm Biopharm 1998;45:275-84). Amino acid sequences for FN1 and FN2 are as follows: FN1: Thr-Leu-Gln-Pro-Val-Tyr-Glu-Tyr-Met-Val-Gly-Val (SEQ ID NO:55); FN2: Thr-Gly-Leu-Pro-Val-Gly-Val-Gly-Tyr-Val-Val-Thr-Val-Leu-Thr (SEQ ID NO:56).

The identity of the central block is chosen from among those sequences that display an optimal combination of morphological, mechanical, and biological properties within the class of Type III block copolymers. The synthetic strategy for formation of the genetic constructs encoding the triblock polymers provides a mechanism for facile variation of the identity of the central block while maintaining the integrity of the endblock domains that are responsible for virtual crosslink formation. Synthetic genes encoding the fibronectin binding sites is synthesized using methods described above such that their termini are compatible with those of the central elastomeric block, as well as the SexA I cleavage site of the polylinker domain in expression plasmids. The central block and fibronectin binding block are subjected to co-oligomerization of the compatible cohesive ends under the action of T4 DNA ligase to afford a pool of mixed concatamers that are inserted into the SexA restriction site of an acceptor plasmid encoding the endblock domains of the Type III construct. The density of fibronectin binding sites within the central block is varied through alteration of the ratio of the respective DNA monomers prior to enzymatic ligation. The sizes of the concatamers are assessed via agarose gel electrosphoresis and the identity of the sequences confirmed by forward and reverse DNA sequence analysis from synthetic primers that are specifically designed to anneal upstream and downstream of the concatamer insertion sites. Expression from these plasmids in E coli strain BL21(DE3) under conditions described above for the triblock polymers affords polypeptides functionalized with fibronectin binding sites that have been substituted into the appropriate central block at various levels of incorporation.

The chemical and structural properties of recombinant elastomeric protein polymers are tested by techniques including automated Edman degradation, MALDI-TOF mass spectroscopy of site-specific proteolytic cleavage fragments, SDS-PAGE, as well as by ¹H, ¹³C, and temperature-dependent HMQC NMR spectroscopy. The latter measurements assist in defining the structural features of multiphase elastomers under hydrated, physiologically relevant conditions. Where appropriate photocrosslinkable methacrylate groups are introduced into lysine containing protein polymers (Types I, III, IV) and the degree of functionalization determined by ¹³C NMR. Inverse temperature transitions (Tt) are determined on all final products by temperature-dependent turbidity measurements and/or DSC. The transition temperature influences process conditions for fiber spinning and assists in refining solvent selection. Finally, 1H dipolar magnetization transfer experiments are performed to measure the size of hydrophilic (E) and hydrophobic (P) domains in films (and fibers) produced from triblock copolymers (Vanderhart DL. Proton spin diffusion as a tool for characterizing polymer blends. Makromol Chem Macromol Symp 1990;34:125-59). Characteristically, domain distances that can be observed using spin diffusion range from 2 to 100 nm and the versatility of this approach has been demonstrated in a variety of multiphase polymer systems (Cai WZ, Egger N, Schmidt-Rohr K, Spiess HW. A solid-state NMR-study of microphase structure and segmental dynamics of poly(styrene-b-methylphenylsiloxane)diblock copolymers. Polymer 1993;34:267-76; Kimura T, Neki K, Tamura N, Horii F, Nakagawa M, Odani H. High-resolution solid-state C-13 nuclear-magnetic-resonance study of the combined process of H-1 spin diffusion and H-1 spin-lattice relaxation in semicrystalline polymers. Polymer 1992;33:493-7). Further details are described elsewhere (Huang L, Nagapudi K, Brinkman W, Apkarian RP, Chaikof EL, Engineered collagen-PEO nanofibers and fabrics, J Biomat Sci - Polymer Ed 2001, In press; Nagapudi K et al., Macromolecules 2002, 35:1730-1737).

Protein fibers and fiber networks are produced using electrospinning techniques and the effect of protein solution concentration, flow rate, and operating voltage on fiber morphology is defined using SEM. The efficiency of Eosin Y/VP mediated photocrosslinking is investigated in both films and fibers by solid-state ¹³C CP/MAS/TOSS NMR spectroscopy. Fiber orientation, diameter, porosity, and total pore volume is determined using a combination of quantitative image analysis and diffusion NMR experiments, as previously described.

Both static and dynamic mechanical properties are characterized using preconditioned hydrated model films and random fiber networks (i.e. isotropically oriented fiber networks) at 37°C or other appropriate temperature in PBS (Seliktar D, Black RA, Vito RP, Nerem RM. Dynamic mechanical conditioning of collagen-gel blood vessel constructs induces remodeling in vitro. Ann Biomed Eng 2000;28(4):351-62; Greer LS, Vito RP, Nerem RM. Material property testing of a collagen-smooth muscle cell lattice for the construction of a bioartificial vascular graft. Adv Bioengineering ASME BED 1994;28:69-70; Brossollet LJ, Vito RP. The effects of cryopreservation on the biaxial mechanical properties of canine saphenous veins. J Biomech Eng 1997;119:1-5; Beattie D, Xu C, Vito R, Glagov S, Whang MC. Mechanical analysis of heterogeneous, atherosclerotic human aorta. J Biomech Eng 1998;120:602-7). The relationship of mechanical behavior to protein polymer structure, including molecular weight, fiber architecture, as well as the nature and degree of crosslink formation facilitates the determination of structure-property relationships necessary for rational material design. Stress-strain properties, such as ultimate tensile strength, maximum strain at failure, Young's modulus, and the modulus of resilience (i.e. the ability of the material to store energy without permanent deformation) are determined by uniaxial tensile testing. Such data will be essential for the initial selection of material combinations for load-bearing applications in an arterial environment. Transient mechanical behavior is defined by stress-relaxation (fixed strain) and creep (fixed stress) studies at small deformations in order to define instantaneous, time-dependent and viscoelastic material behavior (117, 121). Dynamic mechanical properties (storage modulus, loss modulus, and tan delta) are measured, which allow the viscoelastic or time-dependent behavior of these materials to be fully characterized. Specifically, the acquisition of relaxation and retardation spectra using a Dynamic Mechanical Thermal Analyzer (DMTA V; Rheometrics Scientific) facilitates the calculation of unique elastic and viscous components of the complex modulus from measurements of dynamic force and the loss factor (tan delta).

The films and fiber networks described above have sufficient biostability for both in vitro and in vivo investigations. All proteins, however, are potentially degradable, for example due to the action of endogenous peptidases.

Material stability of copolymer composites, including gels, films, fibers, and fiber networks, is assessed by characterizing: (i) morphology by SEM and/or TEM; (ii) porosity by diffusion NMR and/or by quantitative image analysis; (iii) release of degradation products from ¹⁴C-labeled elastin-mimetic protein polymers (Koshy PJ, Rowan AD, Life PF, Cawston TE. 96-Well plate assays for measuring collagenase activity using ³H-acetylated collagen. Anal Biochem 1999; 275(2):202-7); and (iv) alteration in fatigue properties. Dynamic fatigue tests are conducted with a range of strain amplitudes from 1 to 20% about an offset strain of 50% strain to failure (determined from uniaxial testing) at predetermined cycle rates on preconditioned samples (Tanaka TT, Fung YC. Elastic and inelastic properties of canine aorta and their variation along the aortic tree. J Biomechanics 1974; 7:357; Hayashi K. Fatigue Properties of Segmented Polyether Polyurethanes for Cardiovascular Applications. In: Kambic HE, Yokobori T, editors. Biomaterials' Mechanical Properties. Philadelphia: ASTM; 1994. p. STP 1173; Sanders JE, Zachariah SG. Mechanical characterization of biomaterials. Ann NY Acad Sci 1997;831:232-43; Bolotin VV. Mechanics of fatigue. Boca Raton: CRC Press; 1999). Cycle rates higher than physiologically relevant values may be employed to accelerate testing. The total number of cycles to failure (N) is plotted versus strain amplitude in order to generate a fatigue curve for each material. In the process, a fatigue limit, defined as the strain below which failure does not occur, is identified. Since testing for failure through fatigue at physiologic temperature (37°C) may be time intensive, accelerated testing at higher temperatures (about 50°C to about 60°C) is available to predict material lifetime at 37°C. Thus, the tests are conducted as a function of both temperature and cycle rates to determine their effect on fatigue. The DMTA apparatus is equipped with an environmental chamber, which facilitates testing in a controlled aqueous environment at any desired temperature. Thus, we are able to examine the synergistic effects of mechanical stress and environmental factors on material stability. In order to distinguish between the effects of offset and dynamic strain, stress relaxation at large deformations are conducted at 50% of failure strain for a duration that is commensurate with the length of the dynamic test. For these tests, the stress developed is monitored periodically until failure. The influence on material stability of physical, chemical, and biological factors that are operative under physiologically relevant conditions are evaluated in two ways.

For in vitro biostability, the effect of degradation mechanisms, for example temperature, pH, oxidative state, and effect of degrading substances, is elucidated. The influence of temperature is assessed by incubation of fiber networks in PBS at 23°C and 37°C. The effect of pH is studied over a pH range of 2 to 8 (T, 37°C) and the impact of oxidative conditions is evaluated by incubating test materials in PBS containing H2O2 10% (w/w) at 37°C. The oxidant solution is replaced weekly in order to maintain the activity of the solution since the half-life of the hydrogen peroxide at 37°C is seven days (129). Notably, hydrogen peroxide is a key oxidative agent present in macrophages and is secreted into an inflammatory environment that may be present at the time of conduit implantation. The effect of enzymatic degradation is determined by incubating a sample in PBS containing an enzyme, for example MMP-9. All samples are incubated either under non-stressed conditions or subjected to sinusoidal stress and periodically removed for analysis during incubation times of up to 30 days. Frequent replacement of incubating solutions is sometimes required, depending upon test duration.

For in vivo biostability, the intended implantation site is an important determinant of the unique set of environmental and mechanical conditions that ultimately determine the fatigue life of a material. In vivo implant studies in the subcutaneous space are relevant to material biostability and material-tissue interactions (Jenney CR, Anderson JM. Alkylsilane-modified surfaces: inhibition of human macrophage adhesion and foreign body giant cell formation. J Biomed Mater Res 1999;46(1):11-21). In one set of experiments, test samples are implanted into a stainless steel cage placed in a subcutaneous pouch of Wistar rats (n = 5). Material properties are analyzed over a 4-week implant interval (3, 7, 14, 28 days) and correlated with the composition of the cellular infiltrate. Analysis of the local cellular response is performed by fluorescence activated cell sorting (FACS) and/or by immunohistochemical staining. In a second set of experiments, test samples are implanted directly into the subcutaneous space, in the absence of a surrounding cage, and material stability and direct tissue-material interactions are characterized.

Fibronectin adsorption and binding affinities (Kd) are defined on selected films by equilibrium binding studies using ¹²⁵I-labeled fibronectin, as detailed elsewhere (131). Test samples are selected from materials that have generated elastin analogues with desirable mechanical and biostability characteristics. Additionally, the surface distribution of fibronectin adsorbed on microphase separated materials with or without a FN-binding sequence are determined by SEM. The adhesion and proliferation of human aortic endothelial and smooth muscle cells on fibronectin treated surfaces are investigated in vitro by ⁵¹Cr cell labeling and ³H-thymidine incorporation, respectively (Chaikof EL, Caban R, Yan CN, Rao GN, Runge MS. Growth-related responses in arterial smooth muscle cells are arrested by thrombin receptor antisense sequences. *J* Biol Chem 1995;270(13):7431-6; Chon JH, Wang HS, Chaikof EL. Role of fibronectin and sulfated proteoglycans in endothelial cell migration on a cultured smooth muscle layer. J Surg Res 1997;72(1):53-9).

Control of fiber orientation and packing density is achieved. Fiber networks are produced with a geometric arrangement of fibers that is isotropic. Oriented networks are fabricated through fiber spinning on mandrels that are capable of controlled translational movement (Leidner J, Wong EW, MacGregor DC, Wilson GJ. A novel process for the manufacturing of porous grafts: Process description and product evaluation. J Biomed Mater Res 1983;17:229-47). This approach controls not only orientation, but packing density, as well. Moreover, a reduction in fiber packing density is achieved by co-spinning PEO fibers along with other fibers through the use of two spinnerets. Following fabric formation, hydration leads to the dissolution of PEO fibers.

Crosslinking is achieved. Methacrylate groups are suitable for solid-state crosslinking. Nonetheless, other photoreactive groups, such as coumarin moieties, are capable of crosslink formation without the need to add an initiator (e.g. Eosin Y) to the fiber forming polymer solution (Kito H, Matsuda T. Biocompatible coatings for luminal and outer surfaces of small-caliber artificial grafts. J Biomed Mater Res 1996;30(3):321-30).

Degradation is monitored using ¹⁴C-labeling of proteins. Protein polymers are labeled by chemical addition of a carboxylic acid reactive [¹⁴C]-labeled esterifying agent (Koshy PJ, Rowan AD, Life PF, Cawston TE, Anal Biochem 1999;275(2):202-7). The fiber spinning polymer solution is then doped with the radiolabeled protein.

### Example 8. Assessment system of mechanical properties.

Through an analysis of experimental mechanical property data in association with simplifying model assumptions, a stress field is related to a material-dependent strain energy function. The functional form of the strain energy function is based upon an analysis of data derived from uniaxial tensile testing with best-fit material parameters determined by nonlinear regression analysis (Fung YC. Elasticity of soft tissues in simple elongation. Am J Physiol 1967;213:1532-44; Patel DJ. Nonlinear anisotropic elastic properties of the canine aorta. Biophysical J 1972;12:1008-27; Raghavan ML, Vorp DA. Towards a biomechanical tool to evaluate rupture potential of abdominal aortic aneurysm: Identification of a finite constitutive model and evaluation of its applicability. J Biomechanics 2000;33:475-82). In order to characterize a stress field, Kirchoff stress tensor are related to the strain energy function, initially assuming material isotropy, homogeneity, incompressibility, and nonlinear hyperelasticity. The validity and limitations of these assumptions for tissues, such as the vascular wall, which are largely composed of collagen and elastin, has been discussed (Vito RP, Hickey J. The mechanical properties of soft tissues - II: The elastic response of arterial segments. J Biomechanics 1980;13:951-7; Carew TE, Vaishnav RN, Patel DJ. Compressibility of the arterial wall. Circ Res 1968;23:61-8; Vorp DA, Rajagopal KR, Smolinksi PJ, Borovetz HS. Identification of elastic properties of homogeneous orthotropic vascular segments in distention. J Biomechanics 1995;28:501-12; Patel DJ, Fry DL. The elastic symmetry of arterial segments in dogs. Circ Res 1969;24:1-8; Chuong CJ, Fung YC. Compressibility and constitutive equation of the arterial wall in radial compression experiments. J Biomechanics 1984;17:35-40). Material constants for the nonlinear constitutive laws are determined via least-squares fits of data from the uniaxial and biaxial tests. Multiple candidate constitutive relationships fit these data well. In order to determine which relationship best represents the behavior of engineered vascular conduits, two-dimensional finite element models utilizing the various constitutive models are generated to simulate the pressure-diameter experiments. The constitutive law that best represents this behavior is used in more complex, three-dimensional models that include the interface with an adjacent native vessel segment, allowing the comparison of engineered and native vessels. Nonlinear finite element modeling is conducted using the commercial finite element package ABAQUS, with constitutive relationships implemented as user defined materials.

A mechanism is established for the calculation of a stress field so as to characterize the intramural stress distribution within a tubular construct under physiologic loads. This relates to the analysis of component stability, as well as initial cell-material interactions and tissue remodeling responses.

Distinct constitutive laws, which account for the unique behavior of fiber networks, facilitate the achievement of properties in bicomponent constructs that mimic the multilamellar arrangement of collagen and elastin in the arterial wall. For example, parametric analysis is performed via repeat finite element computations to determine the effect of varying the content, mechanical characteristics, and lamellar thickness of collagen and elastin analogues on stress distribution, as well as construct strength and compliance. Constitutive models allow incorporation of compliance mismatch into construct design. An initial assessment of the risk of construct failure is performed in view of predicted degrees of strain in response to physiologic loads. That is, load induced material deformation that exceeds an arbitrary level is used as an approximate definition of a rupture point and "acute" construct failure.

For tensile testing, fiber networks can be produced with a geometric arrangement of fibers that is isotropic. Oriented networks can be fabricated through fiber spinning on mandrels that are capable of controlled translational movement (Leidner J, Wong EW, MacGregor DC, Wilson GJ. J Biomed Mater Res 1983;17:229-47). In this instance, biaxial mechanical testing is performed instead of or in addition to uniaxial testing.

Tubular constructs, consisting of one or more types of fiber networks organized in an alternating lamellar structure, are fabricated by fiber spinning onto a rotating mandrel. Constructs are fabricated with mechanical properties (e.g., compliance and tensile strength) comparing favorably to those reported for native arteries. Following the production of multicomponent constructs, selective chemical and structural properties are defined. For example, solid-state ¹³C NMR is used to confirm complete crosslinking and TEM, along with diffusion NMR studies, are used to characterize network porosity and the organization of alternating collagen and elastin analogue layers. Subsequent analyses of construct mechanical properties provide a mechanism for validation and refinement of constitutive models.

Static and/or dynamic mechanical properties are characterized using hydrated bicomponent fiber samples at 37°C in PBS. Specifically, static and transient (i.e. creep and stress-relaxation) stress-strain properties are determined by uniaxial tensile testing and dynamic mechanical behavior is characterized as detailed above. Studies are performed on test strips obtained from fabricated conduits. In addition, pressure-diameter measurements are performed on cylindrical vascular constructs to determine the incremental Young's modulus and burst pressure (Seliktar D, Black RA, Vito RP, Nerem RM. Dynamic mechanical conditioning of collagen-gel blood vessel constructs induces remodeling in vitro. Ann Biomed Eng 2000;28(4):351-62; Greer LS, Vito RP, Nerem RM., Adv Bioengineering ASME BED 1994;28:69-70; Brossollet LJ, Vito RP, J Biomech Eng 1997;119:1-5). Defining the relationship between mechanical behavior and construct structure and composition in association with computational modeling establishes a basis for the design of hierarchical multicomponent systems.

### Example 9. Rheological studies of B9, PHP, and P2Asn.

All protein samples studied had the same hydrophobic endblock consisting of the plastic sequence VPAVG. The triblock copolymers were constructed by inserting various midblocks between the hydrophobic plastic sequences. See Table 4 and Table 5. Molecular weights are shown in Table 6.

**Table 4.**

| Protein samples. | |
|---|---|
| Block | Description |
| 1 | VPAVG [(IPAVG)₄(VPAVG)]₁₆IPAVG; cross reference SEQ ID NO:50 |
| 2 | -[***X***]- |
| 3 | VPAVG [(IPAVG)₄(VPAVG)]₁₆IPAVG cross reference SEQ ID NO:50 |

**Table 5.**

| Description of [X] in block for given protein. | |
|---|---|
| Protein | [X] |
| P2 | VPGVGVPGVG Parent plastic hydrophobic homopolymer; Cross reference SEQ ID NO: 57 for [X] and SEQ ID NO:58 for protein |
| C5 | VPGVG [(VPGVG)₂VPG***E***G(VPGVG)₂]₃₀VPGVG; Cross reference SEQ ID NO:23 for [X] and SEQ ID NO:52 for protein |
| B9 | VPGVG [(VPGVG)₂VPG***E***G(VPGVG)₂]₃₈VPGVG Cross reference SEQ ID NO:24 for [X} and SEQ ID NO:59 for protein |
| PHP | VPGVG [(APGGVPGGAPGG)₂]₂₃VPGVG Cross reference SEQ ID NO:33 for [X} and SEQ ID NO:60 for protein |
| P2Asn | VPGVG [VPGVG(VP***N***VG)₄]₁₂VPGVG Cross reference SEQ ID NO:30 for [X} and SEQ ID NO:61 for protein |

**Table 6.**

| Molecular weights of proteins. | | | |
|---|---|---|---|
| **Protein name** | **B block** | **A block** | **Molecular weight** |
| P2 | 72 | | 72 kDa |
| C5 | 72 | 62 | 134 kDa |
| B9 | 72 | 93 | 165 kDa |
| PHP | 72 | 50 | 112 kDa |
| P2ASn | 72 | 28 | 100 kDa |

All the rheological data were measured on an ARES III rheometer (Rheometric Scientific Inc.) in the parallel plate mode with a plate diameter of 25 mm. Solutions (20 to25% w/v) of the protein in ddH20 were made at 3°C. The gap in the parallel plate setup was adjusted to be between 200 to 350 micrometers depending upon the sample volume. A volume of 300 to 500 microliters of the sample was placed in between the parallel plates at 3°C. A period of 0.5 hour was provided for temperature equilibration.

The rheological properties of B9, PHP and P2Asn were examined. We performed: (a) a strain amplitude sweep at 3°C performed to determine the appropriate strain to apply (in the linear viscoelastic range); (b) a temperature sweep between 3 and 45°C at a predetermined strain amplitude and frequency to determine gel point (G'-G" crossover); (c) a frequency sweep at 37°C to illustrate G', G" plateau after gelation; and (d) a time sweep at 37°C to determine kinetics of gelation.

The following units and abbreviations are noted. G' and G" (storage and loss shear modulus), dyn/cm² (10 dyn/cm² = 1 Pa); eta* (Complex viscosity), Poise; Temperature, °C; omega (Frequency), rad/s; Time, sec; N1 (First normal stress difference), dyn/cm².

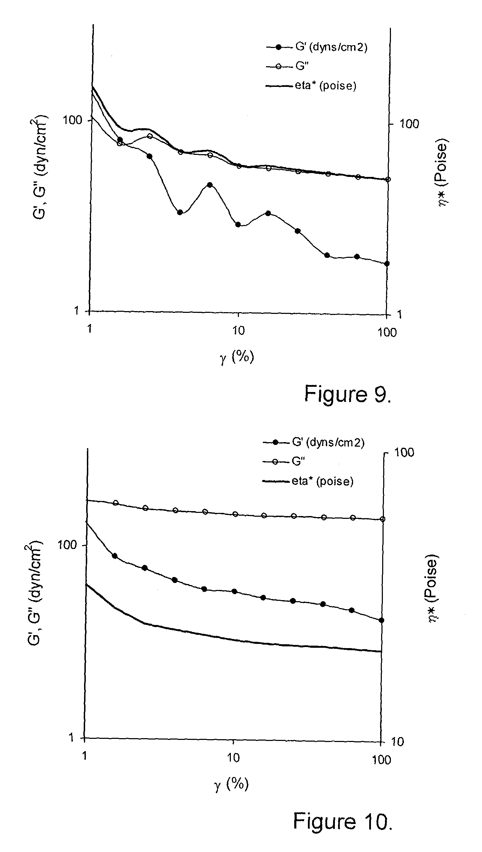

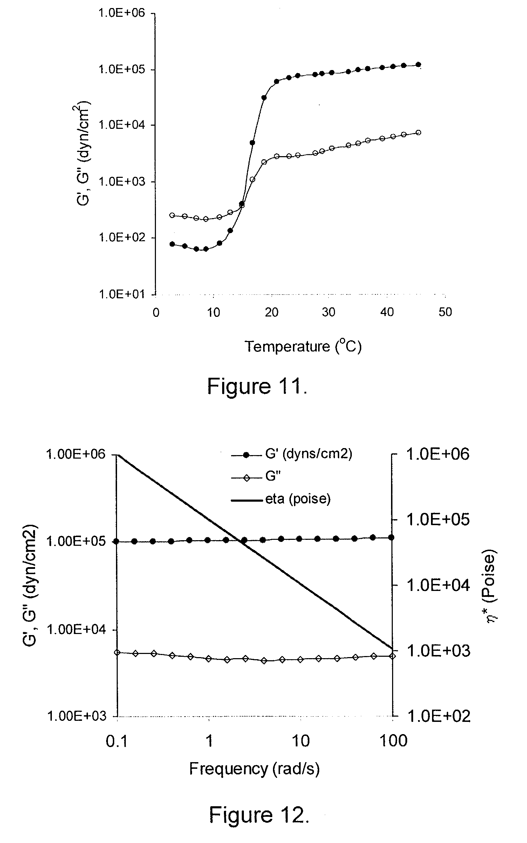

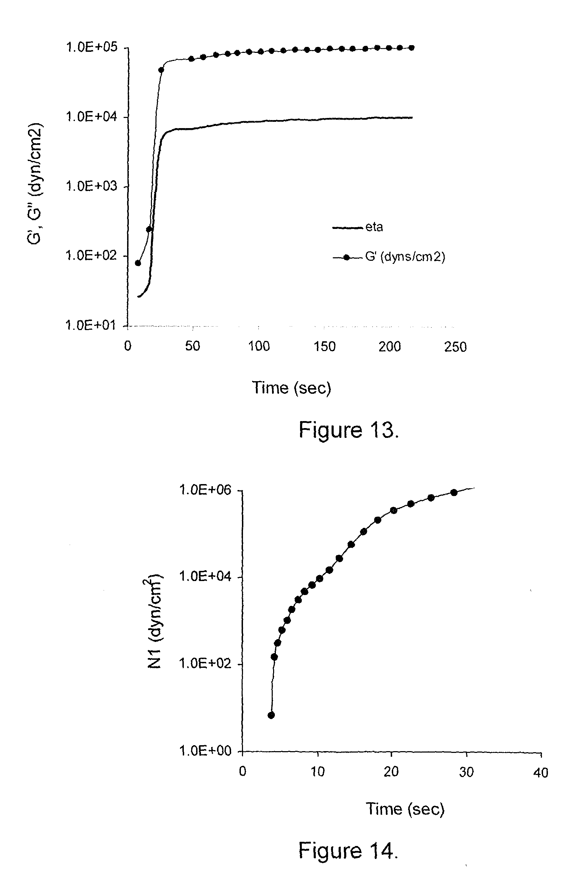

Results are shown in the following Figures. Fig. 9 shows dynamic shear storage and loss modulus as a function of strain amplitude at a frequency of 1 rad/s at 3°C for B9. For Figures 10 and 11, solutions of 25% (w/v) were employed. A strain amplitude of 5% was chosen in the linear range. The gel point was at a temperature of 15°C (G'-G" crossover point), and the gel modulus was 10kPa. Fig. 10 shows dynamic shear storage and loss modulus as a function of strain amplitude at a frequency of 10 rad/s for B9. Fig. 11 shows dynamic shear storage and loss modulus as a function of temperature at a strain amplitude of 5% and a frequency of 10 rad/s, with G'-G" crossover observed at 15°C. Fig. 12 shows dynamic shear storage and loss modulus as a function of frequency at a strain amplitude of 5% and temperature of 37°C for B9. Fig. 13 shows shear storage modulus and complex viscosity as a function of time at 37°C with a strain amplitude of 5% and a frequency of 10 rad/sec for B9. B9 displayed rapid gelation in the range of physiologic temperatures, and gelation was complete in about 20 seconds. Fig. 14 shows first normal stress difference as a function of time at 37°C; this was a transient experiment with a shear rate of 0.1 s⁻¹.

The results for B9 are discussed as follows. A strain amplitude sweep was performed at 3°C at two different frequencies 1 and 10 rad/s (Figures 9 and 10 respectively, showing dynamic shear storage and loss modulus as a function of strain amplitude at given frequency). A frequency of 10 rad/s (Figure C2) yields data where all three quantities G', G" and eta* show a plateau region not seen with a 1 rad/s frequency. This region is called the linear viscoelastic region; any strain amplitude in this region is acceptable for the remaining experiments. Strain amplitude of 5% (at a frequency of 10 rad/s) was chosen since that is right at the beginning of the plateau region.

Figure 11 shows the G' and G" as a function of temperature from 3°C to 45°C at a frequency of 10 rad/s and a strain amplitude of 5%. The gel point (G'-G" crossover) is observed at 15.1°C. A frequency sweep for B9 is shown at 37°C at a strain amplitude of 5% in Figure 12. This data indicates that G' and G" show a plateau region beyond gel point, a result not atypical of most gels beyond gel point. Figure 13 shows the kinetics of gelation for B9. In this experiment G' and eta (η*) are followed as a function of time at 37°C (strain amplitude, 5%; frequency, 10 rad/s). It can be seen from the data that there is about 40 seconds of lag time before the gelation process is complete, after which G' and eta (η*) reach a plateau. The gelation process can be considered instantaneous at 37°C.

Figure 14 shows the first normal stress difference as a function of time in a transient experiment at 37°C (this is not a dynamic experiment, simply the sample is being sheared at 0.1 s⁻¹). The first normal stress difference is an indicator of the elasticity of the gel or melt. We observe for B9 at 37°C an abnormally high first normal stress difference, indicating that the material forms a highly elastic gel at this temperature.

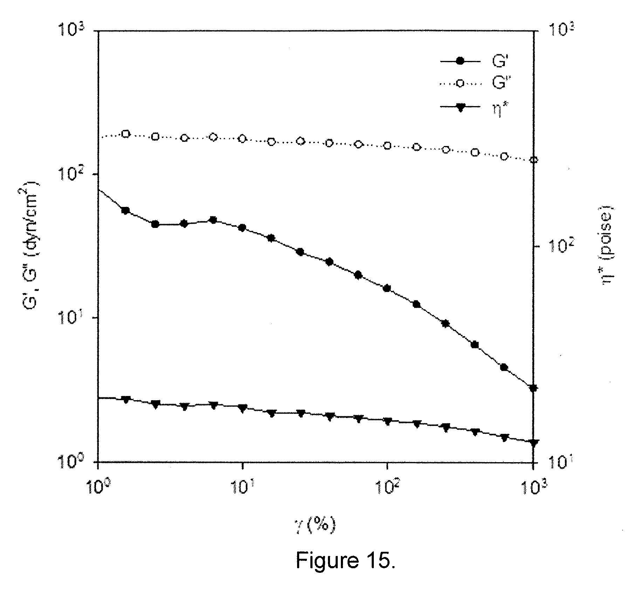

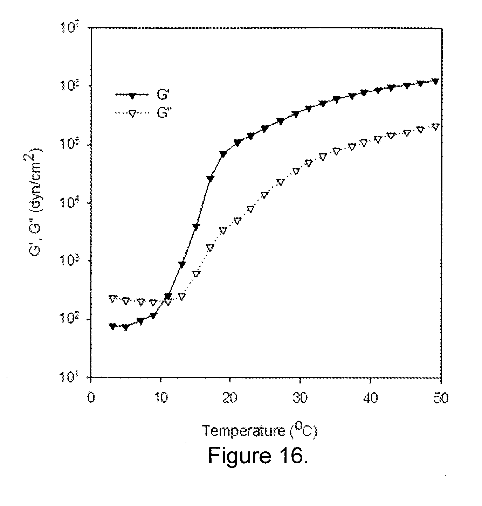

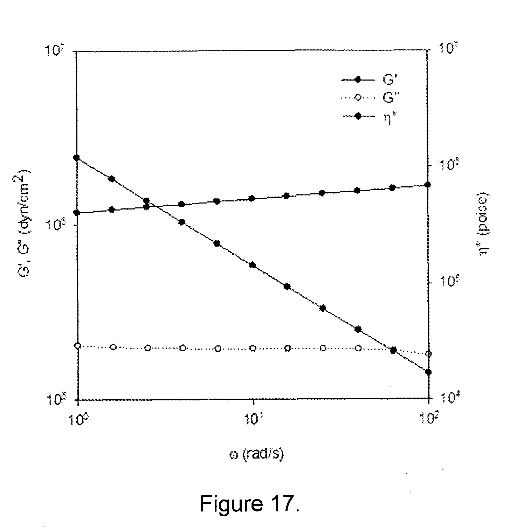

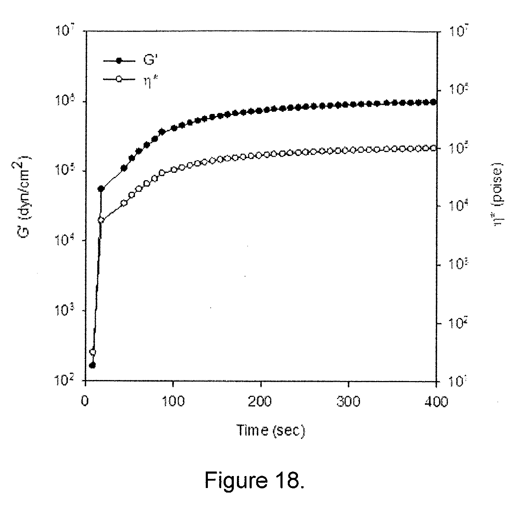

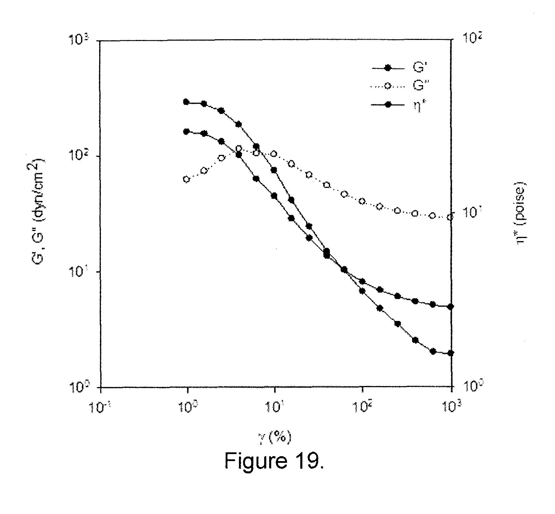

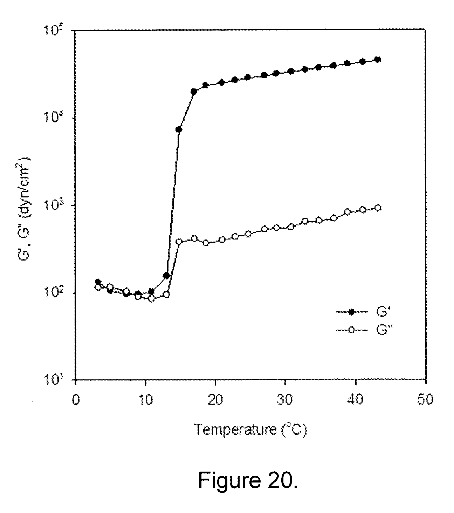

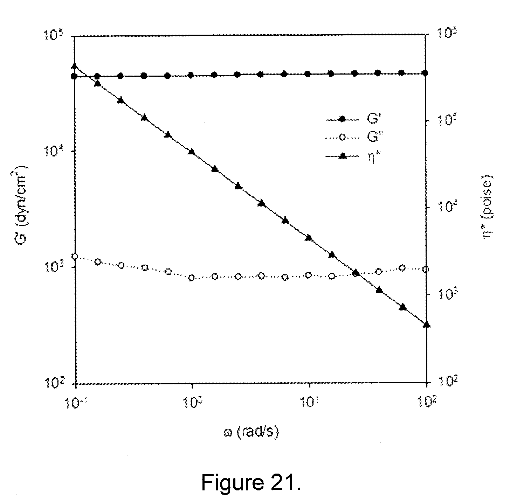

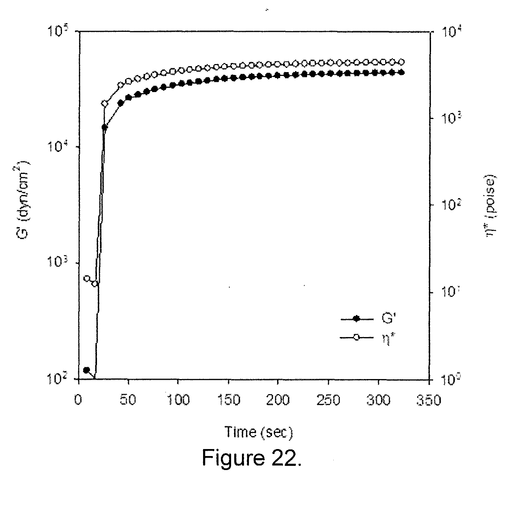

The results for the PHP and P2Asn were qualitatively similar to that of B9, and a summary is provided in Table 7. The actual data is provided in Figures 15 to 22.

**Table 7.**

| Rheological data for elastin-based protein polymers. | | | | | |
|---|---|---|---|---|---|
| Protein | Gel Point | Gel modulus (kPa) | Gel complex viscosity, eta (Poise) | Tan delta at omega=1, 37 °C | Kinetics of gelation |
| P2 | 13.2 | 280 | | 0.1428 | Instantaneous |
| C5 | 14.8 | 6.5 | 98,000 | 0.0128 | |
| B9 | 15.1 | 10.3 | 103,000 | 0.0450 | Instantaneous |
| P2Asn | 10.1 | 120 | 120,000 | 0.1730 | Gradual |
| PHP | 14.9 | 4.5 | 44,000 | 0.0178 | Instantaneous |

A range of mechanical properties can be obtained with these materials at physiologically relevant conditions since all of them gel at 37°C. Strong gels can be formed by P2 (parent homopolymer) and P2Asn, both of which are plastic materials, as compared to C5, B9 or PHP. P2 and P2Asn have higher tan delta values, however, and will likely exhibit higher mechanical hysteresis than C5, B9, or PHP. A range of mechanical properties can translate into a wide variety of applications including biomedical applications.

### Example 10. Tensile properties of protein materials.

To study the effect of chemical structure, films of the homopolymer and the triblock copolymer were cast from 10 wt% solutions in TFE and water. Although protein solutions were prepared at 5°C, solvent evaporation was performed either at 5°C or at 23°C. Since it is known that fluorinated alcohols form strong solid-state complexes with polyamides, TGA analysis was conducted to verify complete removal of solvent from film samples. After complete solvent evaporation, films were hydrated in phosphate buffered saline (pH 7.4), cut into 5mm × 15mm strips for tensile analysis, and stored in PBS for a further 24 hours prior to tensile testing. Hydrated film thickness was measured by optical microscopy using a standard image analysis protocol.

A miniature materials tester (Minimat 2000, Rheometric Scientific) was used to determine the mechanical film properties in the tensile deformation mode with a 20 N load cell, a strain rate of 5 mm/min, and a gauge length of 5 mm. Eight to ten specimens were tested and average Young's modulus, tensile strength, and elongation to break were determined. The tensile properties of these protein-based materials are summarized in Table 8 (relevant entry for P2, in water at 5°C; for B9, in water at 23°C).

**Table 8.**

| Tensile data for elastin-based protein polymers. | | | | | | | |
|---|---|---|---|---|---|---|---|
| Protein | Solvent and Temperature, degrees C | Modulus, MPa | SD* | Tensile strength, Mpa | SD | Elongation to Break (%); strain to failure | SD |
| P2 | water, 5 | 16.65 | 1.55 | 3.00 | 0.32 | 128 | 33 |
| P2 | TFE, 5 | 55.04 | 11.89 | 5.59 | 1.01 | 152 | 42 |
| B9 | TFE, 5 | 4.03 | 1.30 | 2.70 | 0.40 | 390 | 53 |
| B9 | TFE, 23 | 4.90 | 0.92 | 2.28 | 0.58 | 250 | 47 |
| B9 | water, 5 | 0.93 | 0.06 | 2.87 | 0.88 | 640 | 116 |
| B9 | water, 23 | 0.03 | 0.01 | 0.78 | 0.28 | 1084 | 67 |
| B9 | NaOH, 23 | 0.03 | 0.006 | 1.24 | 0.29 | 1330 | 64 |
| | | | | | | | |
| | | | | | | | |
| C5 | | 0.05 | 0.01 | 0.96 | 0.16 | 822 | 97 |
| PHP | | 0.043 | 0.011 | 0.305 | 0.128 | 505 | 123 |
| P2Asn | | 0.97 | 0.26 | 0.22 | 0.04 | 158 | 57 |

| | | | | | | | |
|---|---|---|---|---|---|---|---|
| *SD, standard deviation. | | | | | | | |

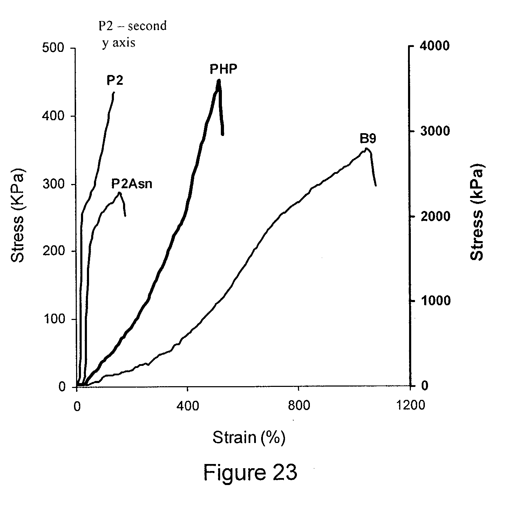

The representative stress-strain curves are shown in Figure 23. A wide range of mechanical responses ranging from plastic deformation to elastic behavior is observed depending upon the amino acid sequence employed to construct the molecule. The scale for P2 is the second y-axis, on the right. P2 has the highest modulus and tensile strength among the materials investigated. Thus protein-based materials can be chemically tailored to exhibit a wide range of mechanical responses, as shown in both tensile and shear properties. This vast range of mechanical responses includes a modulus range of two orders of magnitude and a variation of one order of magnitude variation in tensile strength and strain to failure.

The effect of processing solvent on mechanical properties was studied further. Herein it was shown that films of B9 cast from TFE show plastic deformation while those cast from water at room temperature are elastic. The same range of mechanical responses can also be obtained with B9 by simply changing the processing solvent. Here we demonstrate the ability to get properties within the range by creating an alloy of B9 with itself processed under different conditions.

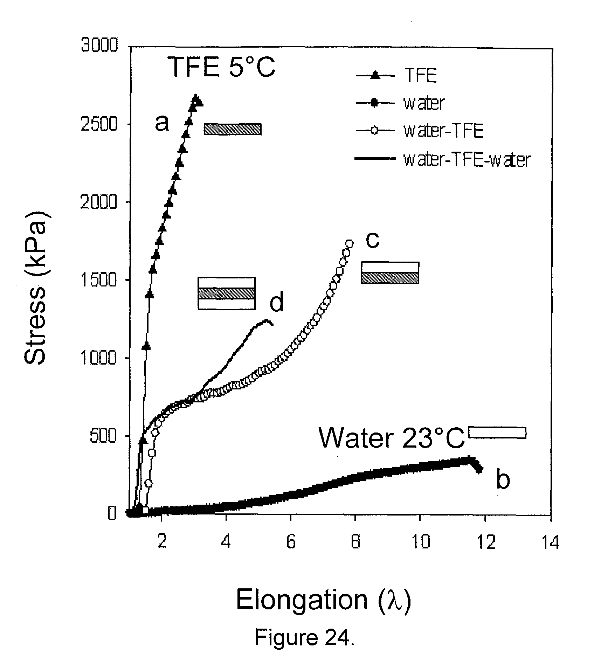

Layers of B9 were formed from TFE and water, and solvent was evaporated at room temperature. The material was subsequently rehydrated in PBS, and the mechanical properties were studied. We obtained properties intermediate to the TFE cast material or the water-cast material. Two different geometries were studied, a laminate of two layers (TFE/Water), and a laminate of three layers (Water/TFE/Water). In both geometries, the amount of B9 in water and TFE was the same, and they were cast from the solutions of the same relative concentration. Thus the primary difference was the relative geometry of construction. As shown in Fig. 24, mechanical properties of triblock copolymer materials are modulated in laminates as indicated by stress-elongation curves for B9 cast from (a) TFE, (b) water, (c) TFE/water laminate, and (d) water/TFE/water laminate. Therefore geometry variations in laminate construction can lead to mechanical property differences.

### Example 11. Fibers of P2 and B9.

In tissues such as blood vessels where shape recovery is critical for performance and to avoid fatigue, elastin networks dominate the low-strain mechanical response. In the native form the material is present as a network of elastic fibers, which provide the required resilience to tissues. In order to limit their extension these elastic fibers are found interwoven with long high-strength inelastic collagen fibrils. The main component of the elastic fiber is a highly hydrophobic protein called elastin. The protein is secreted into the extracellular space where it is assembled into fibrils. Furthermore these fibrils are crosslinked through the available lysine residues to form a network of fibers and sheets, thereby affecting the elasticity of elastin and the overall material. Both experimental and theoretical aspects of elastin's entropic elasticity have been extensively studied and reported.

Naturally occurring elastin is comprised of a diverse variety of peptide sequences. Analysis of amino acid composition of aortic elastin from various species indicates a predominance of valine, proline, glycine and alanine peptides. Moreover, these peptides exist as repeating sequences of polypenta-, polytetra- and polynanopeptides. Urry has demonstrated that a crosslinked matrix of model protein polymers based on such repeating sequences display entropic elasticity similar to that of natural elastin. Thus, from a bio-mimetic tissue engineering standpoint, it is of interest to construct model protein-based polymers containing any or all of the aforementioned repeating sequences, cast these materials into fibers and perform subsequent crosslinking to obtain a desired profile of biomechanical properties.

To generate fibers from water, a technique was developed of having the solution in water in the cold room, with fibers emerging at room temperature. This alleviates the problem of solvent evaporation and has allowed formation of B9 fibers from a water solution. When producing fibers, the rate of solvent evaporation and the nature of the non-woven construct add to the complexity of the mechanical response. Here, fibers formed from TFE and water of P2, C5 and B9 are shown along with electrospinning conditions.

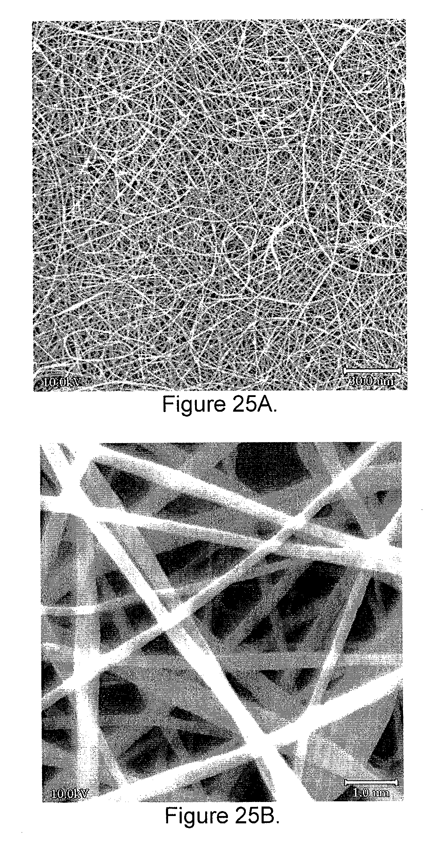

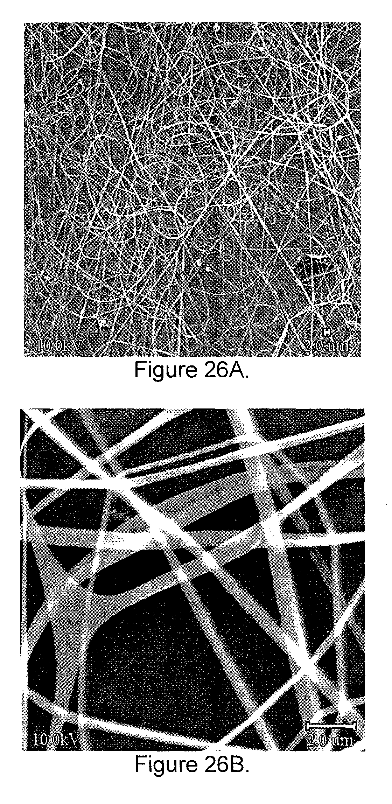

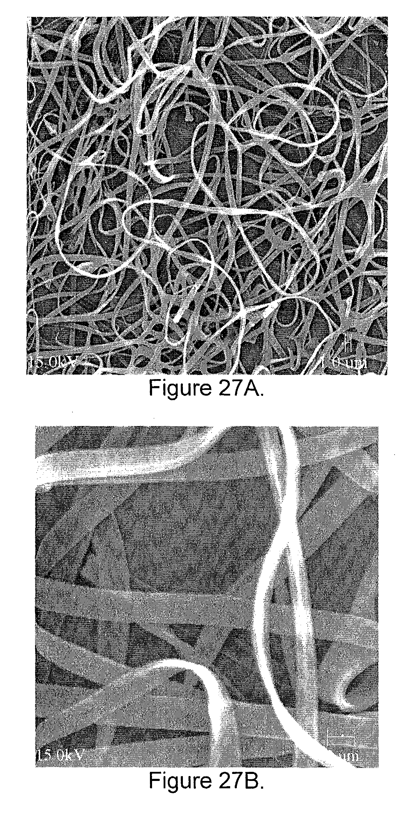

Fig. 25A shows a 10 wt% B9 triblock copolymer fiber spun from pure TFE at 350X magnification. Fiber diameters generally ranged in size from 100 to 400 nanometers (spinning conditions: 18kV, 30 microliter/min, 10 cm deposition distance, room temperature). Fig. 25B has 10kX magnification. Fig. 26A shows 10 wt% C5 triblock copolymer fiber spun from pure TFE at 300X magnification. Fiber diameters were 0.2 to 1 micrometer (spinning conditions: 18kV, 10 cm deposition distance, 30 microliter/min flow rate and room temperature). Fig. 26B has 5kX magnification. Fig. 27A shows 10 wt% B9 triblock copolymer fiber spun from pure water at 5°C with fibers emerging at room temperature (1 kX magnification). Fiber diameters were 0.8 to 3 micrometer (Spinning conditions: 18kV, 10 cm deposition distance, 50 microliter/min flow rate and room temperature). Fig. 27B has 5kX magnification.

### Example 12. Controlled release of a drug: sphingosine-1-phosphate.

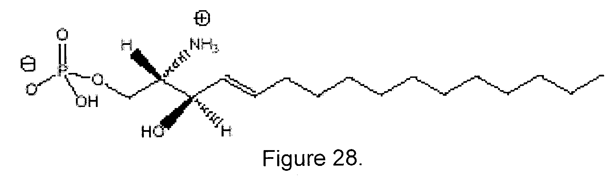

A copolymer herein has capability for application of serving as a controlled release system or matrix in the context of drug delivery. We characterized the release of a small molecule, Sphingosine-1-phosphate (S1P), from films of copolymer B9. Figure 28 indicates the structure of S1P (D-erythroSphingosine-1-Phosphate; (2S, 3R, 4E-2-aminooctadec-4-ene-1,3-diol-1-Phoshpate; abbreviated S1P; molecular weight 379.48; molecular formula C18H38NO5P). S1P is a potent, specific, and selective endothelial cell chemoattractant, and has alone been shown to induce angiogenesis in the mouse cornea. It has also been shown to promote angiogenic responses to growth factors in vivo. See English D et al., 2000, FASEB J 14:2255; Garcia JG, 2001; J Clin Invest 108:689.

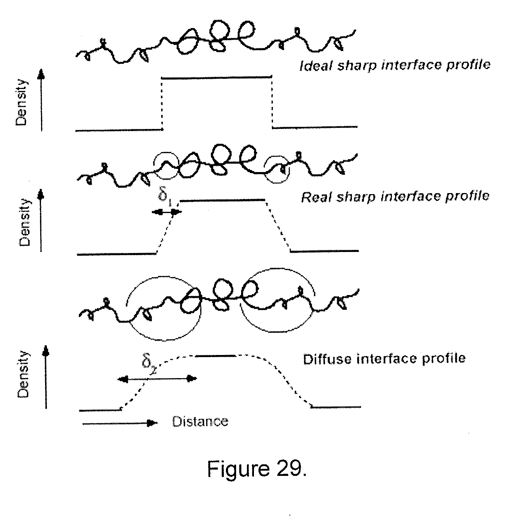

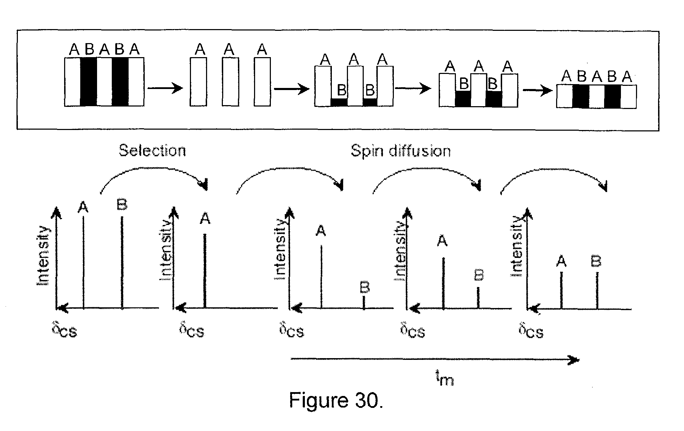

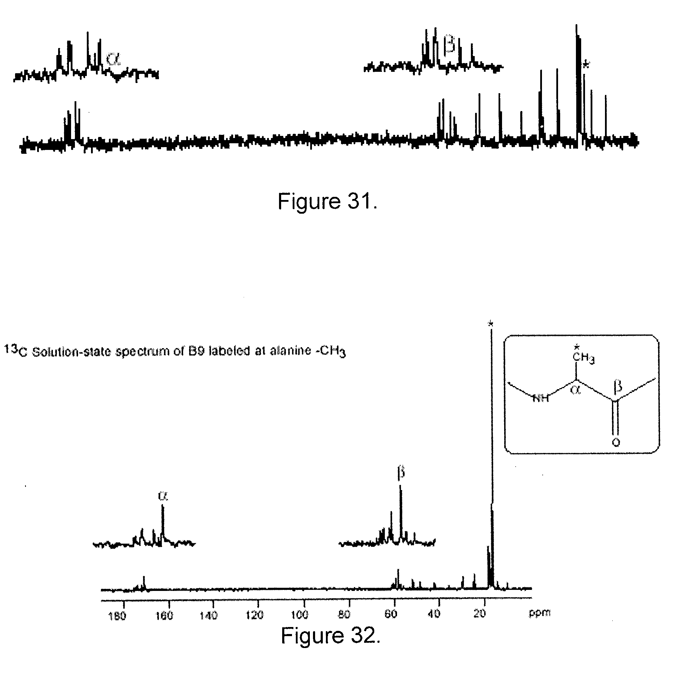

As observed earlier, B9 displays different properties in TFE relative to those in water. A possible explanation is that less or more of an interface was formed in the material between the second (middle or A block) depending upon the influence of the solvent on the block. Table 9 shows mechanical properties of protein sequences. Sequence alteration provides one tool for modifying hydrophobicity, pH response, and bioconjugation; furthermore, combinations of sequences provides a copolymer with unique properties. Table 10 indicates amino acid sequences of copolymers. Figure 29 illustrates possible interface profiles relating to domains in a B9 copolymer cast from different solvents (1H NMR dipolar filter). Figure 30 shows an evaluation of domain sizes in B9 cast from different solvents by the technique of Spin Diffusion NMR, monitoring the intensity decay in A or build-up in B. Figure 31 shows a ¹³C solution-state spectrum of unlabeled B9, and Figure 32 shows a ¹³C solution-state spectrum of B9 labeled in the end block alanine -CH3.

**Table 9.**

| Mechanical properties of protein sequences. | | |
|---|---|---|
| Protein Sequence | Mechanical State | Permitted alteration |
| (VPAVG)n; (SEQ ID NO:5)n | Plastic | 1 |
| (VPGVG)n; (SEQ ID NO:2)n | Elastic | 4 |
| (APGGVPGGAPGG)n (SEQ ID NO65)n | Fluid state-Hydrogel | |

**Table 10.**

| Amino acid sequences of proteins. | | |
|---|---|---|
| Protein Name | B block* | A block |
| P2 | | VPGVGVPGVG |
| P2E22 | | VPGVG[VPGVG(VPGIGVPGVG)₂]₁₉VPGVG; Cross reference SEQ ID NO:21 for A-block and SEQ ID NO:62 for protein |
| C5 | | VPGVG[(VPGVG)₂VPGEG(VPGVG)₂]₃₀VPGVG; Cross reference SEQ ID NO:23 for A-block and SEQ ID NO:52 for protein |
| B9 | | VPGVG[(VPGVG)₂VPGEG(VPGVG)₂]₃₈VPGVG; Cross reference SEQ ID NO:24 for A-block and SEQ ID NO:59 for protein |
| P2Asn | | VPGVG[VPGVG(VPNVG)4)]₁₂VPGVG; Cross reference SEQ ID NO:30 for A-block and SEQ ID NO:61 for protein |
| PHP | | VPGVG[(APGGVPGGAPGG)₂]₂₃VPGVG; Cross reference SEQ ID NO:33 for A-block and SEQ ID NO:60 for protein |

| | | |
|---|---|---|
| *The B block sequence is VPAVG[(IPAVG)₄(VPAVG)]₁₆IPAVG; SEQ ID NO:50. | | |

Whether or not such possible explanations provide theoretical support, the potential for differing amounts of interfacial material can have practical significance. For example, this region in between hydrophobic and hydrophilic blocks can be used to mix, integrate, or encapsulate a molecule, including amphiphilic molecules like S1P. Regardless of theory, the ability was determined for a copolymer to be used in the creation of a mixture or encapsulation unit comprising another molecule, thereby controlling or moderating the release of the molecule. A single copolymer, for example B9, can used to make a mixture of S1P or encapsulate differing amounts of S1P. Then, the S1P can be released at differing rates depending upon the solvent from which B9 is processed.

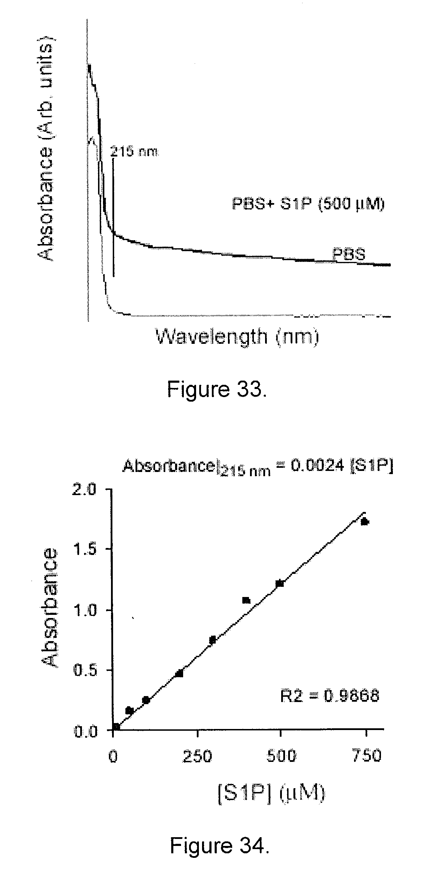

We performed release studies of S1P from films of B9 cast in TFE and water. The release was followed by UV spectroscopy. Figure 33 shows that ultraviolet light can be used to monitor the concentration of S1P in phosphate buffered saline. Figure 34 shows that absorbance of S1P scales linearly with concentration; a concentration of 10 micromolar is detectable.

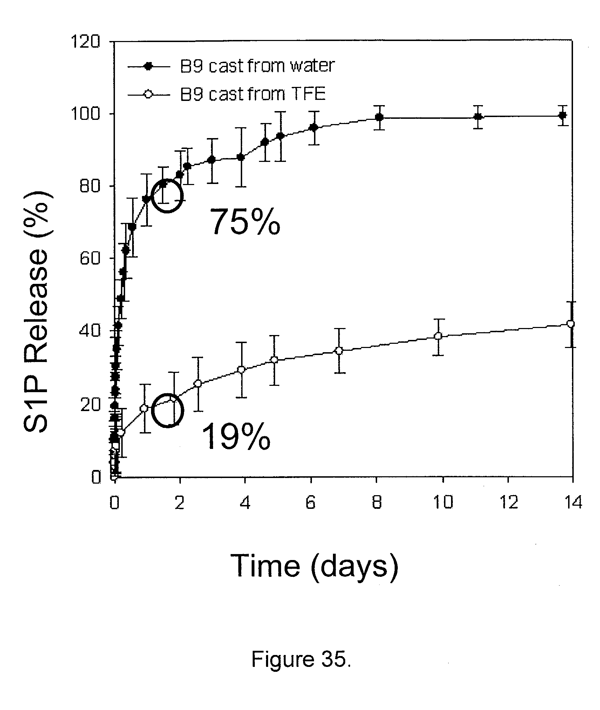

Figure 35 shows the percent of S1P released over time. In the study, a quantity of 2 mg of S1P was loaded into 100 mg of B9, and films were cast from a 10 wt% solution in either TFE or water at room temperature. The results showed that the drug eluted at a more rapid rate in B9 films cast from water than those cast from TFE. For example, almost 100% of S1P has been released from a film form of B9 cast from water within 8 days, whereas at that time only about 36% has eluted from the TFE-cast films. Moreover, even after 14 days only 42% of the drug has eluted from TFE-cast films. Approximately the same amount of drug was loaded into the polymer films in both cases. To make the release study amenable to monitoring by ultraviolet light, this study used large quantities of S1P (two percent relative to the weight of copolymer). A modulation of the release kinetics was achieved merely by varying a processing condition such as the solvent.

### Example 13. Controlled release of a growth factor molecule: Fibroblast Growth Factor 2.

Controlled release of a large molecule from a film form is achieved by mixing the large molecule with a copolymer. We performed release studies of the large molecule, Fibroblast Growth Factor 2 (FGF-2). A total of 50 mgs of B9 was dissolved in 491 microliters of ddH20 at 5°C. To this solution 9.995 micrograms of unlabeled FGF-2 and 9.04 microliters (5 ngs) of ¹²⁵I-FGF-2 were added (FGF-2: ¹²⁵I-FGF-2 = 2000:1). Film forms of B9 protein containing FGF-2 were cast and then allowed to incubate for 8 to 10 hours at room temperature for removal of solvent. Film samples of defined weight and size were immersed in 1 mL of PBS at 37°C on a shaker bath. At prescribed time intervals over a 15-day time period, films were transferred into fresh containers with 1 mL PBS, and gamma counter readings of eluted samples were used to determine FGF-2 release (n = 3).

### Example 14. Representations of copolymers.

The representations indicate possible structures, mechanisms, or explanations that may aid in understanding embodiments of the invention but do not assert an actual requirement for operation.

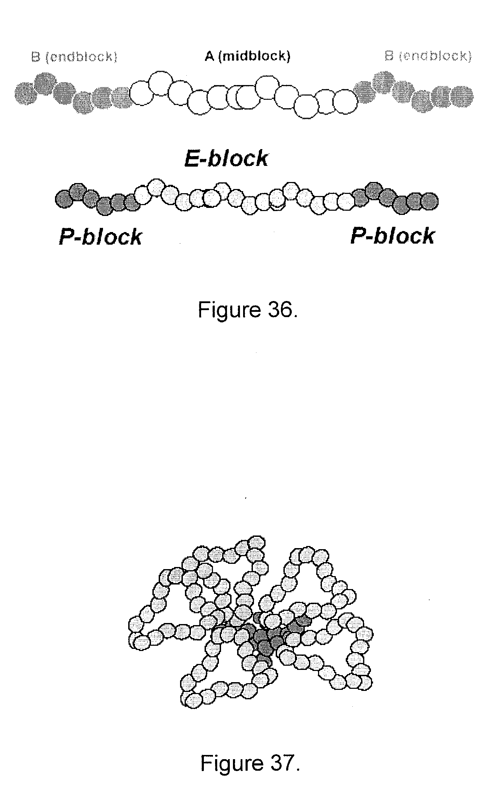

Figure 36 shows diagrams of triblock copolymers. At top is a "BAB" triblock copolymer, where A is a block segment in the middle, flanked by B block segments on the ends. At bottom is a triblock copolymer where the P-block indicates a plastic block, and an E-block indicates an elastic block. Figure 37 shows a diagram of a possible conformation upon reaching a transition temperature, Tt; the material can re-order its orientation with respect to the external environment such as the solvent.

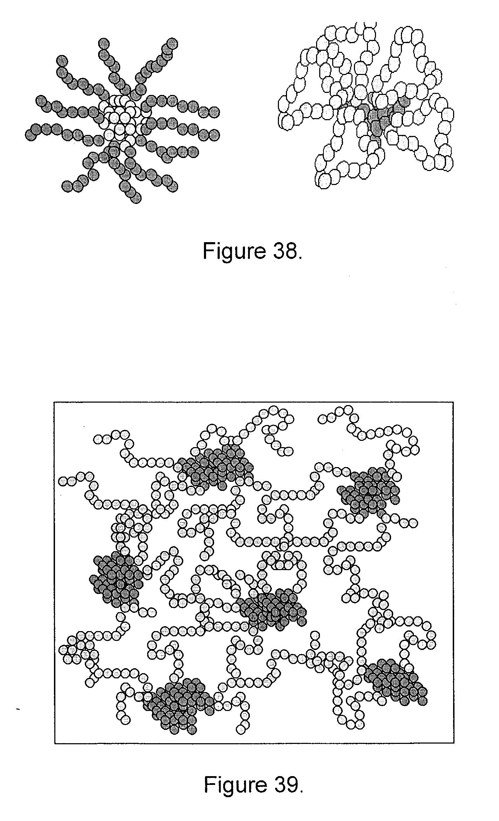

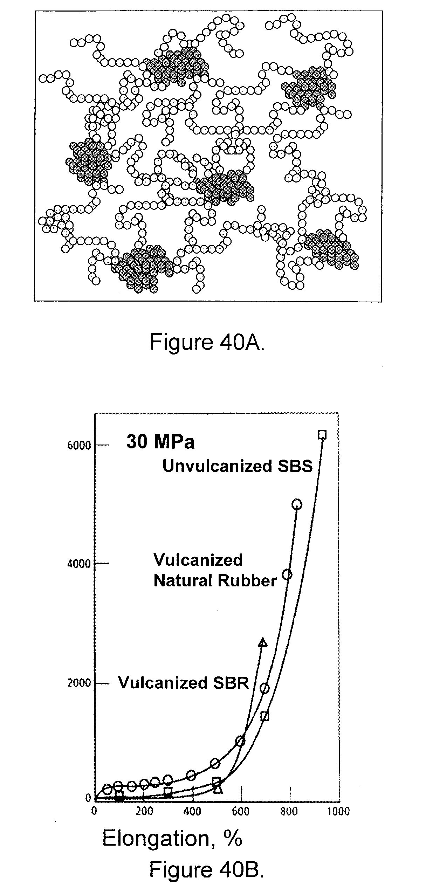

Figure 38 shows a diagram copolymer in solvent specific for the A block, at left, or for the B block, at right. Figure 39 shows a virtual or physically crosslinked network upon increased concentration of BAB triblock copolymer. Figure 40A shows synthetic thermoplastic elastomers, with a polystyrene domain shown as dark clusters (T < Tg) and an elastomer mid segment shown as quasi-linear moieties (T > Tg). Figure 40B shows a graph of stress (psi) versus elongation for synthetic thermoplastic elastomers, including Poly(Styrene-b-butadiene-styrene); see G. Holden and R. Milkovich (Shell Oil), US Patent 3,265,765. Table 11 shows properties of commercial polymers in addition to B9 under different processing conditions.

**Table 11.**

| Properties of Commercial Elastomers and B9. | | | |
|---|---|---|---|
| **Polymer** | Grade | **Modulus (MPa)** | **Strain to failure (x 100%)** |
| Natural rubber | Unfilled* vulcanisate | 1-2 | 6.5 - 9 |
| Styrene butadiene rubber (SBR) | Unfilled vulcanisate (23-25% styrene) | 1-2 | 4.5 - 6 |
| Isobutylene isoprene rubber (IIR) | Carbon black filled | 4-10 | 3-7 |
| Acrylonitrile-butadiene rubber (NBR) | Carbon black filled | 8-18 | |
| Chloroprene rubber (CR) | Unfilled vulcanisate | 1-3 | 8-10 |
| Ethylene-propylene rubber (EPDM) | Carbon black filled | 5 - 10 | 2.5 - 7.5 |
| B9 protein in water | Unfilled hydrated | 0.02-0.05 | 10-14 |
| B9 protein mixed films (TFE+ water)** | Unfilled hydrated | 1-3 | 6-8 |

| | | | |
|---|---|---|---|
| *Filler is material as known in the art such as carbon black. | | | |
| **B9 protein mixed film material: The film was prepared by dissolving B9 in vial 1 with TFE and by dissolving B9 in vial 2 with water. Solution from vial 1 was poured and solvent was allowed to evaporate. Next, solution from vial 2 was poured and solvent was allowed to evaporate. Thus a layered-type film material was formed. | | | |

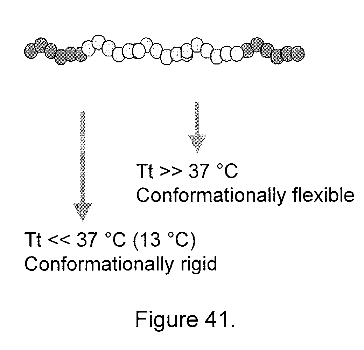

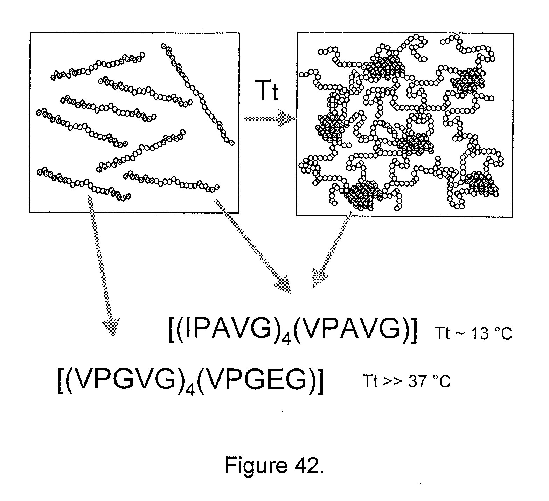

Figure 41 shows attributes of a copolymer relative to transition temperature. Figure 42 shows a diagram of block copolymers and amino acid sequences; at left, the inner lighter midblock has the sequence of [(VPGVG)₄(VPGEG)], (see SEQ ID NO:15); and the outer darker endblocks have a sequence of [(IPAVG)4(VPAVG)] (see SEQ ID NO:12). At right, the darker endblock segment tends to cluster upon reaching the transition temperature, Tt.

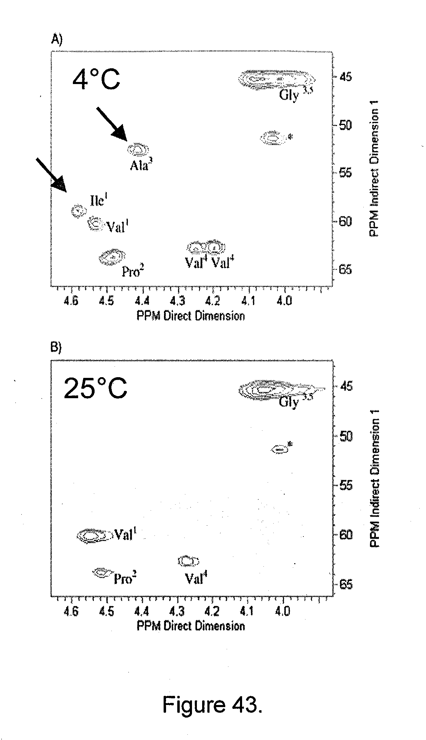

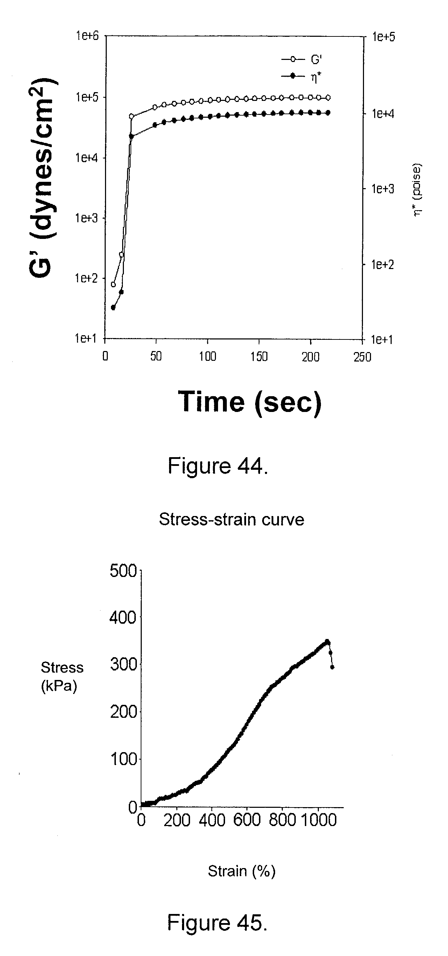

Figure 43 indicates that the technique of ¹H, ¹³C HMQC NMR Spectroscopy shows selective phase transition of hydrophobic end blocks (top, at 4°C; bottom, at a temperature of 25°C). Figure 44 examines the uniaxial stress-strain behavior of a protein triblock film. The graph indicates shear storage modulus and complex viscosity as a function of time (T = 37 degrees C, strain amplitude 5%, omega = 10 rad/sec). Figure 45 shows a stress versus strain curve.

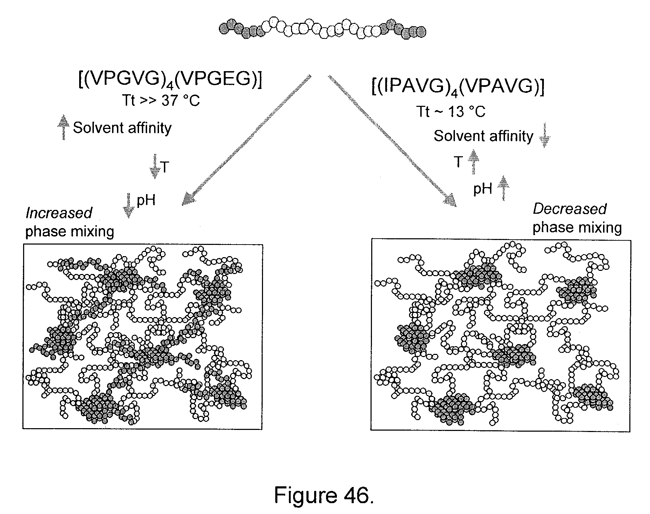

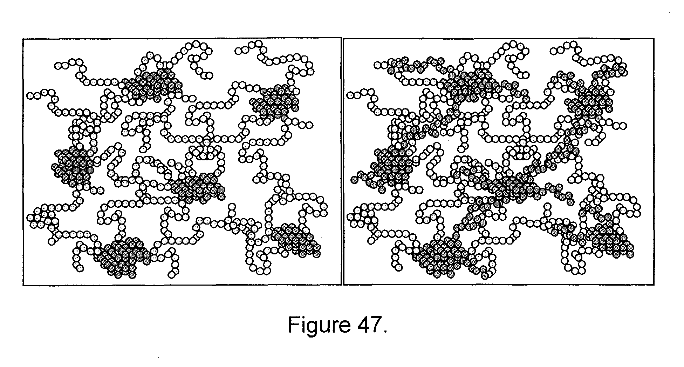

Figure 46 illustrates a possible relation of system morphology to mechanical properties of BAB triblock copolymers. Figure 47 illustrates possible solvent effects on film forms, with possible morphologies for a water cast film at left and a TFE cast film at right.

### Example 15. Rheological comparison of protein polymers.

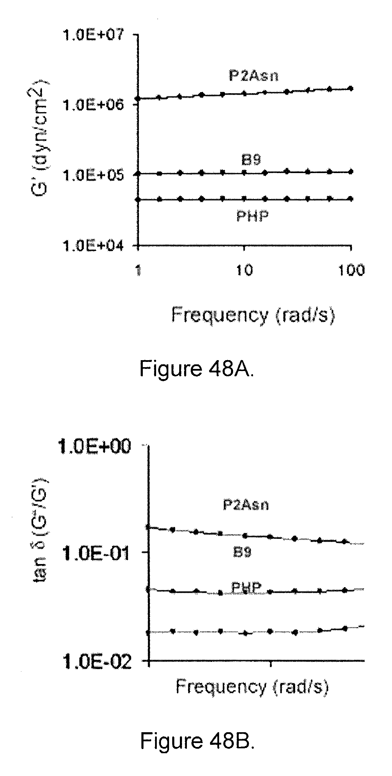

For polymers P2Asn, B9, and PHP, Figure 48A shows a graph of G' versus frequency, and Figure 48B shows a graph of tan delta versus frequency. P2Asn is made of plastic sequences and has the highest gel modulus here. In terms of relative elasticity, PHP is greater than that of B9 which is greater than that of P2Asn.

### Example 16. Small particles from copolymers.

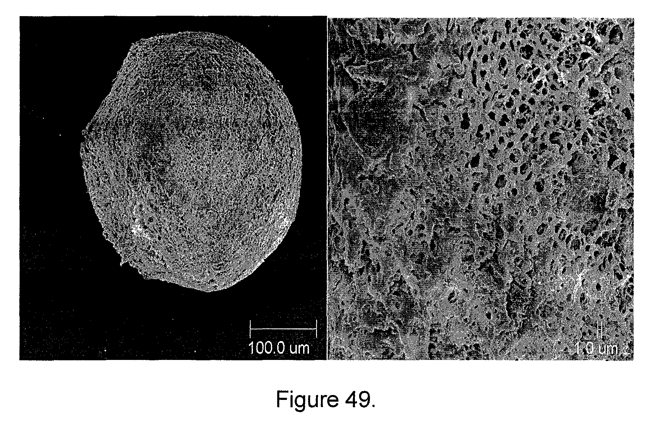

A copolymer is processed into a form thereby creating small particles. Using an oil-in-water emulsion strategy, microparticles of protein triblock copolymer B9 have been generated. An aqueous protein solution is added to corn oil maintained at a temperature below Tt. Following homogenization (20,000 rpm), the temperature of oil-in-water emulsion is raised above Tt to solidify particles, which are subsequently filtered, washed, and dried. This process yielded microparticles with an average diameter of 200 µm. Figure 49 shows an example of a roughly spherical or bead-like particle, with lower magnification at left and higher magnification at right.

For a summary of sequence listings, see Table 12.

All references throughout this application, for example publications, patents, and patent documents, are hereby incorporated by reference herein in their entireties, as though individually incorporated by reference, to the extent not inconsistent with the disclosure in this application.

The invention has been described with reference to various specific and preferred embodiments and techniques. However, it should be understood that many variations and modifications may be made while remaining within the spirit and scope of the invention. It will be apparent to one of ordinary skill in the art that methods, devices, device elements, materials, procedures and techniques other than those specifically described herein can be applied or adapted to the practice of the invention as broadly disclosed herein without resort to undue experimentation. For example, methods for recombinant expression in systems other than those specifically exemplified are known in the art, such as other prokaryotic and eukaryotic expression systems, and can be applied to the generation of protein polymers. All art-known equivalents, including functional equivalents, are intended to be encompassed by this invention; equivalents are not limited to compositions, methods, devices, device elements, materials, procedures and techniques described herein. This invention is not to be limited by the embodiments disclosed, including any shown in the drawings or exemplified in the specification, which are given by way of example and not of limitation.

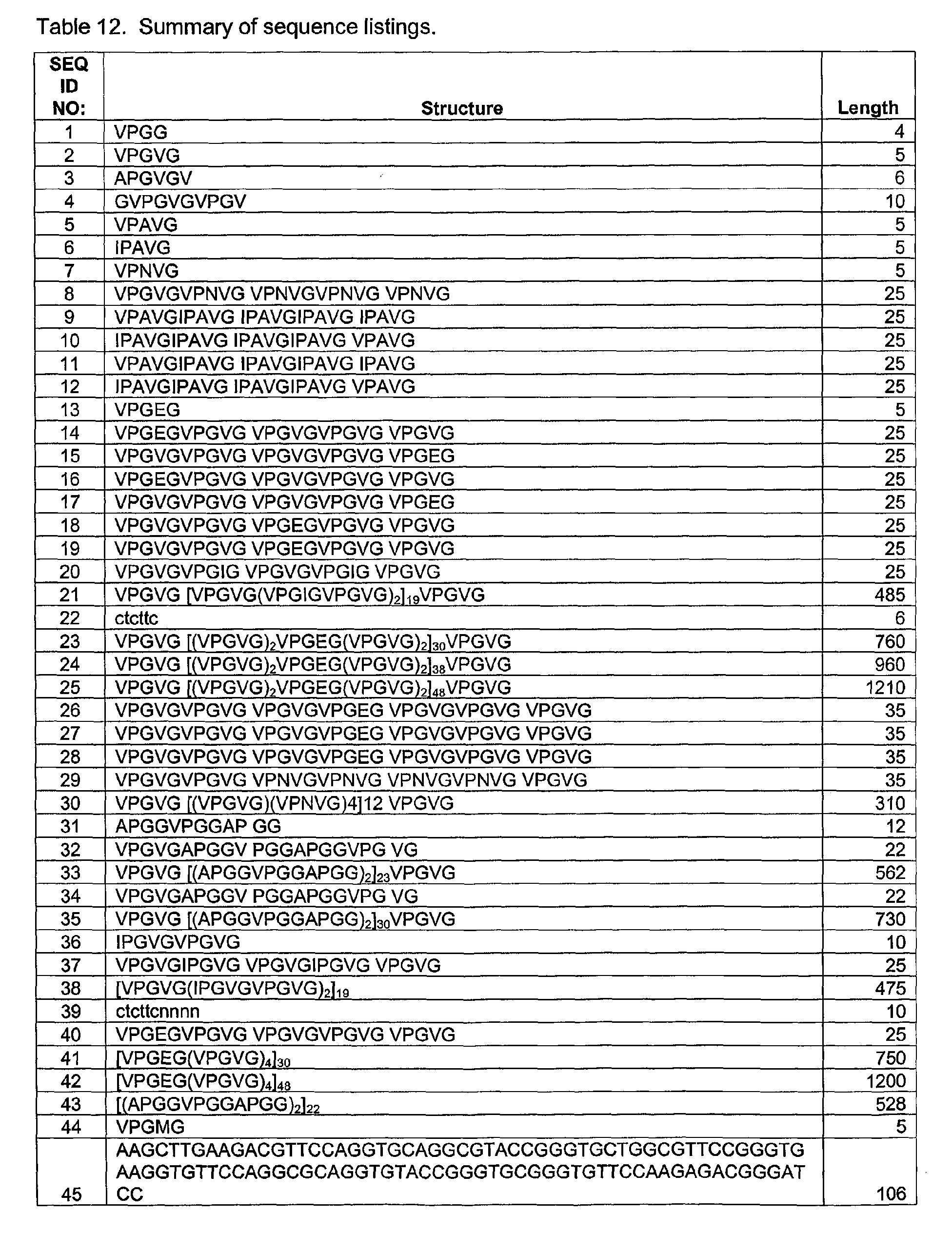

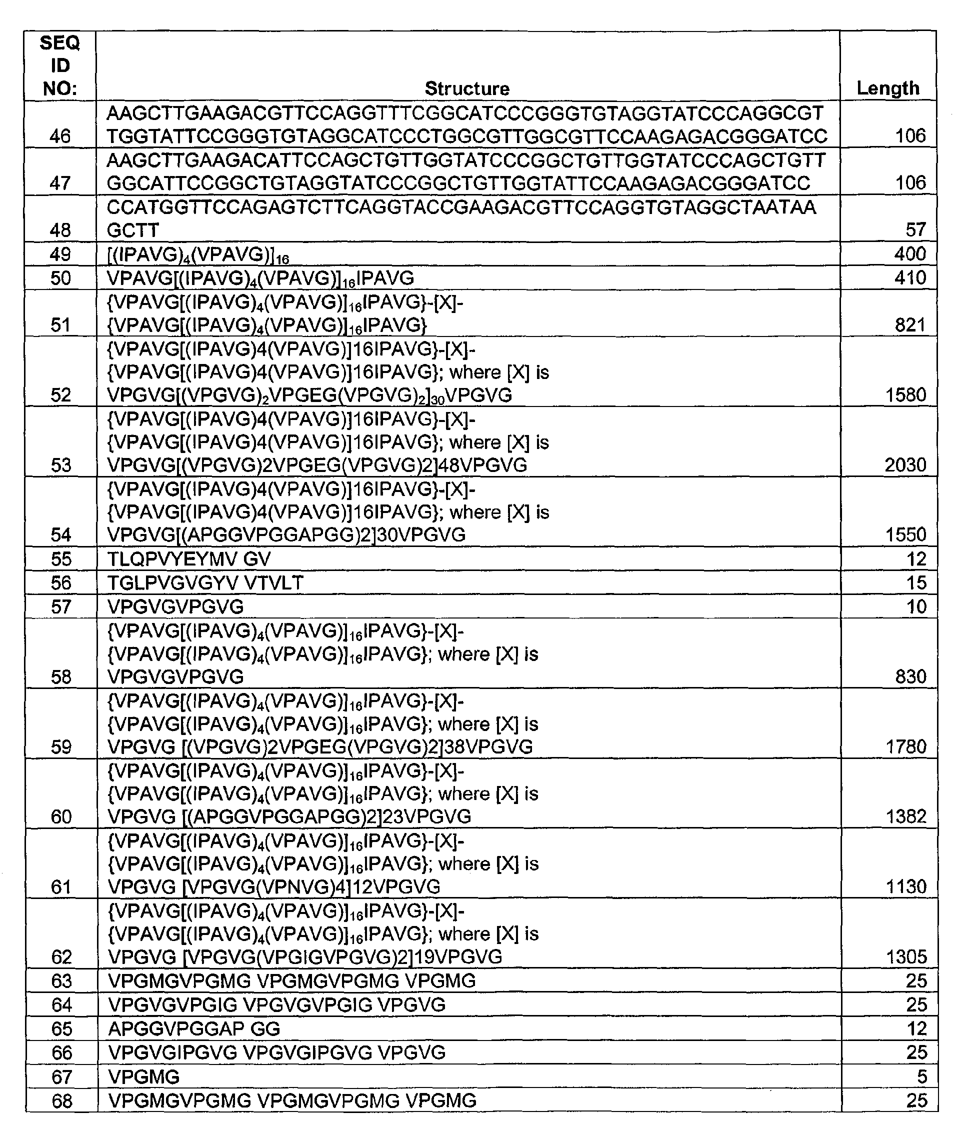

## Claims

1. A synthetic protein copolymer comprising at least one hydrophilic block and at least one hydrophobic block.

2. The protein copolymer of claim 1 having a first hydrophobic end block, a second hydrophobic end block, and a middle hydrophilic block.

3. The protein copolymer of claim 1 having a first hydrophobic end block, a second hydrophobic end block, and a middle hydrophilic block, wherein said first and second end blocks are substantially identical.

4. The protein copolymer of any of claims 1 to 3, wherein the first end block comprises an amino acid sequence of [VPAVG(IPAVG)₄]ₙ or [(IPAVG)₄(VPAVG)]ₙ ; cross-referenced as SEQ ID NO:11 and SEQ ID NO:12.

5. The protein copolymer of any of claims 2 to 4, wherein the middle block comprises an amino acid sequence selected from the group consisting of: [(VPGEG) (VPGVG)₄]ₘ , [(VPGVG)₄(VPGEG)]ₘ , and [(VPGVG)₂VPGEG(VPGVG)₂]ₘ; cross-referenced as SEQ ID NO:14, SEQ ID NO:15, and SEQ ID NO:18.

6. The protein copolymer of claim 4 or claim 5 wherein n is from about 5 to about 100 and wherein m is from about 10 to about 100.

7. The protein copolymer of claim 6 wherein n is about 16.

8. The protein copolymer of any of claims 2 to 7 wherein the middle block is selected from the group consisting of:
| STRUCTURE | SEQ ID NO: |
|---|---|
| VPGVG [VPGVG(VPGIGVPGVG)₂]₁₉VPGVG; | 21 |
| VPGVG [(VPGVG)₂VPGEG(VPGVG)₂]₃₀VPGVG; | 23 |
| VPGVG [(VPGVG)₂VPGEG(VPGVG)₂]₃₈VPGVG; | 24 |
| VPGVG [(VPGVG)₂VPGEG(VPGVG)₂]₄₈VPGVG; | 25 |
| VPGVG [VPGVG(VPNVG)₄]₁₂VPGVG; | 30 |
| VPGVG [(APGGVPGGAPGG)₂]₂₃VPGVG; | 33 |
| VPGVG [(APGGVPGGAPGG)₂]₃₀VPGVG; | 35 |
| [VPGVG(IPGVGVPGVG)₂]₁₉; | 38 |
| [VPGEG(VPGVG)₄]₃₀; | 41 |
| [VPGEG(VPGVG)₄]₄₈; | 42 |
| [(APGGVPGGAPGG)₂]₂₂; and | 43 |
| [(VPGMG)₅]ₓ, wherein x is from about 10 to about 100. | 63 |

9. The protein copolymer of any of claims 1 to 8 capable of elongation up to about 14 times its initial length.

10. A film comprising the protein copolymer of any of claims 1 to 9.

11. The film of claim 10 comprising a plurality of layers.

12. The multi-layered film of claim 11 comprising a first layer and a second layer, wherein the first layer derives from a first polymer exposed to a first solvent, and the second layer derives from a second polymer exposed to a second solvent, thereby creating a film having a desired mechanical property.

13. The multi-layered film of claim 12 wherein the first polymer and the second polymer are substantially identical.

14. The multi-layered film of claim 12 or claim 13 wherein the first solvent enhances film elasticity and the second solvent enhances film plasticity.

15. The multi-layered film of any of claims 12 to 14 wherein the first solvent is water and the second solvent is trifluoroethanol.

16. The protein copolymer of any of claims 1 to 9 in gel form.

17. The protein copolymer of claims 1 to 9 in the form of a fiber or fiber network.

18. The fiber network of claim 17 comprising a first fiber and a second fiber, wherein the first fiber derives from a polymer exposed to a first solvent and the second fiber derives from a polymer exposed to a second solvent.

19. A method of generating a medical implant comprising the step of including the fiber of claim 17 or claim 18 in the implant.

20. A method for producing a plastic elastic protein copolymer comprising the steps of
a. providing a first block of nucleic acid sequence, wherein said first block encodes a hydrophilic protein;
b. providing a second block of nucleic acid sequence, wherein said second block encodes a hydrophobic protein;
c. synthesizing a nucleic acid molecule comprising said first and second blocks; and
d. expressing said nucleic acid molecule to produce said protein copolymer.

21. The method of claim 20 further comprising solubilizing said protein copolymer in a solvent, thereby creating a solution, and bringing said solution to a temperature to cause said copolymer to agglomerate to form a non-covalently crosslinked mass.

22. The method of claim 20 or claim 21 further comprising covalently crosslinking said polymer.

23. A method of manufacture of a stent, embolization coil, vascular graft, or other implanted biomedical device comprising the method of claim 21 or 22 and further comprising the steps of
e. including a drug or biological agent in the solvent, thereby making a mixture with said copolymer; and
f. applying said mixture to said stent, embolization coil, vascular graft, or other implanted biomedical device.

24. A nucleic acid sequence comprising S1 (SEQ ID NO:45), S2 (SEQ ID NO:46), S3 (SEQ ID NO:47), or S-adaptor (SEQ ID NO:48).

25. The method of claim 20 wherein said first block or said second block of nucleic acid sequence comprise one or more sequences of claim 24.


9935 sequence listing
SEQUENCE LISTING

<110>  Emory University

<120>  PLASTIC AND ELASTIC PROTEIN COPOLYMERS

<130>  9935EP/JED

<150>  US 60/428,438
<151>  2002-11-22

<150>  CA 2,417,634
<151>  2003-01-29

<150>  JP 2003-98691
<151>  2003-04-01

<150>  AU 2003236491
<151>  2003-08-27

<160>  68

<170>  PatentIn version 3.2

<210>  1
<211>  4
<212>  PRT
<213>  Artificial

<220>
<223>  Synthetic construct.

<400>  1

Val Pro Gly Gly
1


<210>  2
<211>  5
<212>  PRT
<213>  Artificial

<220>
<223>  Synthetic construct

<400>  2

Val Pro Gly Val Gly
1               5


<210>  3
<211>  6
<212>  PRT
<213>  Artificial

<220>
<223>  Synthetic construct

<400>  3

Ala Pro Gly Val Gly Val
1               5


<210>  4
<211>  10

9935 sequence listing
<212>  PRT
<213>  Artificial

<220>
<223>  Synthetic construct


<220>
<221>  REPEAT
<222>  (2)..(6)
<223>  Repeat residues 2 to 6; total of 19 repeat units.
       G-(VPGVG)19-VPGV

<400>  4

Gly Val Pro Gly Val Gly Val Pro Gly Val
1               5                   10


<210>  5
<211>  5
<212>  PRT
<213>  Artificial

<220>
<223>  Synthetic construct

<400>  5

Val Pro Ala Val Gly
1               5


<210>  6
<211>  5
<212>  PRT
<213>  Artificial

<220>
<223>  Synthetic construct

<400>  6

Ile Pro Ala Val Gly
1               5


<210>  7
<211>  5
<212>  PRT
<213>  Artificial

<220>
<223>  Synthetic construct.

<400>  7

Val Pro Asn Val Gly
1               5


<210>  8
<211>  25
<212>  PRT
<213>  Artificial

<220>
<223>  Synthetic construct.

9935 sequence listing

<400> 8

Val Pro Gly Val Gly Val Pro Asn Val Gly Val Pro Asn Val Gly Val
1               5                   10                  15

Pro Asn Val Gly Val Pro Asn Val Gly
            20                  25

<210> 9
<211> 25
<212> PRT
<213> Artificial

<220>
<223> Synthetic construct

<220>
<221> REPEAT
<222> (1)..(25)
<223> [VPAVG(IPAVG)4]n

<400> 9

Val Pro Ala Val Gly Ile Pro Ala Val Gly Ile Pro Ala Val Gly Ile
1               5                   10                  15

Pro Ala Val Gly Ile Pro Ala Val Gly
            20                  25

<210> 10
<211> 25
<212> PRT
<213> Artificial

<220>
<223> Synthetic construct

<220>
<221> REPEAT
<222> (1)..(25)
<223> [(IPAVG)4(VPAVG)]n

<400> 10

Ile Pro Ala Val Gly Ile Pro Ala Val Gly Ile Pro Ala Val Gly Ile
1               5                   10                  15

Pro Ala Val Gly Val Pro Ala Val Gly
            20                  25

<210> 11
<211> 25
<212> PRT
<213> Artificial

<220>
<223> Synthetic construct

<400> 11

9935 sequence listing

Val Pro Ala Val Gly Ile Pro Ala Val Gly Ile Pro Ala Val Gly Ile
1               5                   10                  15

Pro Ala Val Gly Ile Pro Ala Val Gly
            20                  25

<210>  12
<211>  25
<212>  PRT
<213>  Artificial

<220>
<223>  Synthetic construct

<400>  12

Ile Pro Ala Val Gly Ile Pro Ala Val Gly Ile Pro Ala Val Gly Ile
1               5                   10                  15

Pro Ala Val Gly Val Pro Ala Val Gly
            20                  25

<210>  13
<211>  5
<212>  PRT
<213>  Artificial

<220>
<223>  Synthetic construct

<400>  13

Val Pro Gly Glu Gly
1               5

<210>  14
<211>  25
<212>  PRT
<213>  Artificial

<220>
<223>  Synthetic construct

<400>  14

Val Pro Gly Glu Gly Val Pro Gly Val Gly Val Pro Gly Val Gly Val
1               5                   10                  15

Pro Gly Val Gly Val Pro Gly Val Gly
            20                  25

<210>  15
<211>  25
<212>  PRT
<213>  Artificial

<220>
<223>  Synthetic construct

<400>  15

9935 sequence listing

Val Pro Gly Val Gly Val Pro Gly Val Gly Val Pro Gly Val Gly Val
1               5                   10                  15

Pro Gly Val Gly Val Pro Gly Glu Gly
            20                  25

<210>  16
<211>  25
<212>  PRT
<213>  Artificial

<220>
<223>  Synthetic construct

<220>
<221>  REPEAT
<222>  (1)..(25)
<223>  [(VPGEG)(VPGVG)4]m;
       alternatively [VPGEGVPGVG VPGVGVPGVG VPGVG]m

<400>  16

Val Pro Gly Glu Gly Val Pro Gly Val Gly Val Pro Gly Val Gly Val
1               5                   10                  15

Pro Gly Val Gly Val Pro Gly Val Gly
            20                  25

<210>  17
<211>  25
<212>  PRT
<213>  Artificial

<220>
<223>  Synthetic construct

<220>
<221>  REPEAT
<222>  (1)..(25)
<223>  [VPGVGVPGVG VPGVGVPGVG VPGEG]m

<400>  17

Val Pro Gly Val Gly Val Pro Gly Val Gly Val Pro Gly Val Gly Val
1               5                   10                  15

Pro Gly Val Gly Val Pro Gly Glu Gly
            20                  25

<210>  18
<211>  25
<212>  PRT
<213>  Artificial

<220>
<223>  Synthetic construct

<400>  18

9935 sequence listing
Val Pro Gly Val Gly Val Pro Gly Val Gly Val Pro Gly Glu Gly Val
1               5                   10                  15

Pro Gly Val Gly Val Pro Gly Val Gly
            20                  25

<210>  19
<211>  25
<212>  PRT
<213>  Artificial

<220>
<223>  synthetic construct

<220>
<221>  REPEAT
<222>  (1)..(25)
<223>  [(VPGVG)2 VPGEG (VPGVG)2)]m

<400>  19

Val Pro Gly Val Gly Val Pro Gly Val Gly Val Pro Gly Glu Gly Val
1               5                   10                  15

Pro Gly Val Gly Val Pro Gly Val Gly
            20                  25

<210>  20
<211>  25
<212>  PRT
<213>  Artificial

<220>
<223>  synthetic construct

<220>
<221>  REPEAT
<222>  (1)..(25)
<223>  Repeat [VPGVGVPGIG VPGVGVPGIG VPGVG] for a total of 19 units
       alternatively [VPGVG(VPGIGVPGVG)2]19

<400>  20

Val Pro Gly Val Gly Val Pro Gly Ile Gly Val Pro Gly Val Gly Val
1               5                   10                  15

Pro Gly Ile Gly Val Pro Gly Val Gly
            20                  25

<210>  21
<211>  485
<212>  PRT
<213>  Artificial

<220>
<223>  synthetic construct

<400>  21

Val Pro Gly Val Gly Val Pro Gly Val Gly Val Pro Gly Ile Gly Val

9935 sequence listing
1               5                   10                  15

Pro Gly Val Gly Val Pro Gly Ile Gly Val Pro Gly Val Gly Val Pro
            20                  25                  30

Gly Val Gly Val Pro Gly Ile Gly Val Pro Gly Val Gly Val Pro Gly
        35                  40                  45

Ile Gly Val Pro Gly Val Gly Val Pro Gly Val Gly Val Pro Gly Ile
    50                  55                  60

Gly Val Pro Gly Val Gly Val Pro Gly Ile Gly Val Pro Gly Val Gly
65                  70                  75                  80

Val Pro Gly Val Gly Val Pro Gly Ile Gly Val Pro Gly Val Gly Val
                85                  90                  95

Pro Gly Ile Gly Val Pro Gly Val Gly Val Pro Gly Val Gly Val Pro
            100                 105                 110

Gly Ile Gly Val Pro Gly Val Gly Val Pro Gly Ile Gly Val Pro Gly
        115                 120                 125

Val Gly Val Pro Gly Val Gly Val Pro Gly Ile Gly Val Pro Gly Val
    130                 135                 140

Gly Val Pro Gly Ile Gly Val Pro Gly Val Gly Val Pro Gly Val Gly
145                 150                 155                 160

Val Pro Gly Ile Gly Val Pro Gly Val Gly Val Pro Gly Ile Gly Val
                165                 170                 175

Pro Gly Val Gly Val Pro Gly Val Gly Val Pro Gly Ile Gly Val Pro
            180                 185                 190

Gly Val Gly Val Pro Gly Ile Gly Val Pro Gly Val Gly Val Pro Gly
        195                 200                 205

Val Gly Val Pro Gly Ile Gly Val Pro Gly Val Gly Val Pro Gly Ile
    210                 215                 220

Gly Val Pro Gly Val Gly Val Pro Gly Val Gly Val Pro Gly Ile Gly
225                 230                 235                 240

Val Pro Gly Val Gly Val Pro Gly Ile Gly Val Pro Gly Val Gly Val
                245                 250                 255

Pro Gly Val Gly Val Pro Gly Ile Gly Val Pro Gly Val Gly Val Pro
            260                 265                 270

Gly Ile Gly Val Pro Gly Val Gly Val Pro Gly Val Gly Val Pro Gly

9935 sequence listing
        275                         280                         285

Ile Gly Val Pro Gly Val Gly Val Pro Gly Ile Gly Val Pro Gly Val
    290                     295                     300

Gly Val Pro Gly Val Gly Val Pro Gly Ile Gly Val Pro Gly Val Gly
305                     310                     315                     320

Val Pro Gly Ile Gly Val Pro Gly Val Gly Val Pro Gly Val Gly Val
                325                     330                     335

Pro Gly Ile Gly Val Pro Gly Val Gly Val Pro Gly Ile Gly Val Pro
            340                     345                     350

Gly Val Gly Val Pro Gly Val Gly Val Pro Gly Ile Gly Val Pro Gly
        355                     360                     365

Val Gly Val Pro Gly Ile Gly Val Pro Gly Val Gly Val Pro Gly Val
    370                     375                     380

Gly Val Pro Gly Ile Gly Val Pro Gly Val Gly Val Pro Gly Ile Gly
385                     390                     395                     400

Val Pro Gly Val Gly Val Pro Gly Val Gly Val Pro Gly Ile Gly Val
                405                     410                     415

Pro Gly Val Gly Val Pro Gly Ile Gly Val Pro Gly Val Gly Val Pro
            420                     425                     430

Gly Val Gly Val Pro Gly Ile Gly Val Pro Gly Val Gly Val Pro Gly
        435                     440                     445

Ile Gly Val Pro Gly Val Gly Val Pro Gly Val Gly Val Pro Gly Ile
    450                     455                     460

Gly Val Pro Gly Val Gly Val Pro Gly Ile Gly Val Pro Gly Val Gly
465                     470                     475                     480

Val Pro Gly Val Gly
                485

<210>  22
<211>  6
<212>  DNA
<213>  Artificial

<220>
<223>  Synthetic construct

<400>  22
ctcttc                                                                  6

<210>  23

9935 sequence listing
<211>  760
<212>  PRT
<213>  Artificial

<220>
<223>  Synthetic construct

<400>  23

Val Pro Gly Val Gly Val Pro Gly Val Gly Val Pro Gly Val Gly Val
1               5                   10                  15

Pro Gly Glu Gly Val Pro Gly Val Gly Val Pro Gly Val Gly Val Pro
            20                  25                  30

Gly Val Gly Val Pro Gly Val Gly Val Pro Gly Glu Gly Val Pro Gly
        35                  40                  45

Val Gly Val Pro Gly Val Gly Val Pro Gly Val Gly Val Pro Gly Val
    50                  55                  60

Gly Val Pro Gly Glu Gly Val Pro Gly Val Gly Val Pro Gly Val Gly
65                  70                  75                  80

Val Pro Gly Val Gly Val Pro Gly Val Gly Val Pro Gly Glu Gly Val
                85                  90                  95

Pro Gly Val Gly Val Pro Gly Val Gly Val Pro Gly Val Gly Val Pro
            100                 105                 110

Gly Val Gly Val Pro Gly Glu Gly Val Pro Gly Val Gly Val Pro Gly
        115                 120                 125

Val Gly Val Pro Gly Val Gly Val Pro Gly Val Gly Val Pro Gly Glu
    130                 135                 140

Gly Val Pro Gly Val Gly Val Pro Gly Val Gly Val Pro Gly Val Gly
145                 150                 155                 160

Val Pro Gly Val Gly Val Pro Gly Glu Gly Val Pro Gly Val Gly Val
                165                 170                 175

Pro Gly Val Gly Val Pro Gly Val Gly Val Pro Gly Val Gly Val Pro
            180                 185                 190

Gly Glu Gly Val Pro Gly Val Gly Val Pro Gly Val Gly Val Pro Gly
        195                 200                 205

Val Gly Val Pro Gly Val Gly Val Pro Gly Glu Gly Val Pro Gly Val
    210                 215                 220

Gly Val Pro Gly Val Gly Val Pro Gly Val Gly Val Pro Gly Val Gly
225                 230                 235                 240

9935 sequence listing

Val Pro Gly Glu Gly Val Pro Gly Val Gly Val Pro Gly Val Gly Val
                245                 250                 255

Pro Gly Val Gly Val Pro Gly Val Gly Val Pro Gly Glu Gly Val Pro
            260                 265                 270

Gly Val Gly Val Pro Gly Val Gly Val Pro Gly Val Gly Val Pro Gly
        275                 280                 285

Val Gly Val Pro Gly Glu Gly Val Pro Gly Val Gly Val Pro Gly Val
    290                 295                 300

Gly Val Pro Gly Val Gly Val Pro Gly Val Gly Val Pro Gly Glu Gly
305                 310                 315                 320

Val Pro Gly Val Gly Val Pro Gly Val Gly Val Pro Gly Val Gly Val
                325                 330                 335

Pro Gly Val Gly Val Pro Gly Glu Gly Val Pro Gly Val Gly Val Pro
            340                 345                 350

Gly Val Gly Val Pro Gly Val Gly Val Pro Gly Val Gly Val Pro Gly
        355                 360                 365

Glu Gly Val Pro Gly Val Gly Val Pro Gly Val Gly Val Pro Gly Val
    370                 375                 380

Gly Val Pro Gly Val Gly Val Pro Gly Glu Gly Val Pro Gly Val Gly
385                 390                 395                 400

Val Pro Gly Val Gly Val Pro Gly Val Gly Val Pro Gly Val Gly Val
                405                 410                 415

Pro Gly Glu Gly Val Pro Gly Val Gly Val Pro Gly Val Gly Val Pro
            420                 425                 430

Gly Val Gly Val Pro Gly Val Gly Val Pro Gly Glu Gly Val Pro Gly
        435                 440                 445

Val Gly Val Pro Gly Val Gly Val Pro Gly Val Gly Val Pro Gly Val
    450                 455                 460

Gly Val Pro Gly Glu Gly Val Pro Gly Val Gly Val Pro Gly Val Gly
465                 470                 475                 480

Val Pro Gly Val Gly Val Pro Gly Val Gly Val Pro Gly Glu Gly Val
                485                 490                 495

Pro Gly Val Gly Val Pro Gly Val Gly Val Pro Gly Val Gly Val Pro
            500                 505                 510

9935 sequence listing

Gly Val Gly Val Pro Gly Glu Gly Val Pro Gly Val Gly Val Pro Gly
        515                 520                 525

Val Gly Val Pro Gly Val Gly Val Pro Gly Val Gly Val Pro Gly Glu
    530                 535                 540

Gly Val Pro Gly Val Gly Val Pro Gly Val Gly Val Pro Gly Val Gly
545                 550                 555                 560

Val Pro Gly Val Gly Val Pro Gly Glu Gly Val Pro Gly Val Gly Val
                565                 570                 575

Pro Gly Val Gly Val Pro Gly Val Gly Val Pro Gly Val Gly Val Pro
            580                 585                 590

Gly Glu Gly Val Pro Gly Val Gly Val Pro Gly Val Gly Val Pro Gly
        595                 600                 605

Val Gly Val Pro Gly Val Gly Val Pro Gly Glu Gly Val Pro Gly Val
    610                 615                 620

Gly Val Pro Gly Val Gly Val Pro Gly Val Gly Val Pro Gly Val Gly
625                 630                 635                 640

Val Pro Gly Glu Gly Val Pro Gly Val Gly Val Pro Gly Val Gly Val
                645                 650                 655

Pro Gly Val Gly Val Pro Gly Val Gly Val Pro Gly Glu Gly Val Pro
            660                 665                 670

Gly Val Gly Val Pro Gly Val Gly Val Pro Gly Val Gly Val Pro Gly
        675                 680                 685

Val Gly Val Pro Gly Glu Gly Val Pro Gly Val Gly Val Pro Gly Val
    690                 695                 700

Gly Val Pro Gly Val Gly Val Pro Gly Val Gly Val Pro Gly Glu Gly
705                 710                 715                 720

Val Pro Gly Val Gly Val Pro Gly Val Gly Val Pro Gly Val Gly Val
                725                 730                 735

Pro Gly Val Gly Val Pro Gly Glu Gly Val Pro Gly Val Gly Val Pro
            740                 745                 750

Gly Val Gly Val Pro Gly Val Gly
        755                 760

<210>  24
<211>  960
<212>  PRT

9935 sequence listing
<213>  Artificial

<220>
<223>  Synthetic construct

<400>  24

Val Pro Gly Val Gly Val Pro Gly Val Gly Val Pro Gly Val Gly Val
1               5                   10                  15

Pro Gly Glu Gly Val Pro Gly Val Gly Val Pro Gly Val Gly Val Pro
            20                  25                  30

Gly Val Gly Val Pro Gly Val Gly Val Pro Gly Glu Gly Val Pro Gly
        35                  40                  45

Val Gly Val Pro Gly Val Gly Val Pro Gly Val Gly Val Pro Gly Val
    50                  55                  60

Gly Val Pro Gly Glu Gly Val Pro Gly Val Gly Val Pro Gly Val Gly
65                  70                  75                  80

Val Pro Gly Val Gly Val Pro Gly Val Gly Val Pro Gly Glu Gly Val
                85                  90                  95

Pro Gly Val Gly Val Pro Gly Val Gly Val Pro Gly Val Gly Val Pro
            100                 105                 110

Gly Val Gly Val Pro Gly Glu Gly Val Pro Gly Val Gly Val Pro Gly
        115                 120                 125

Val Gly Val Pro Gly Val Gly Val Pro Gly Val Gly Val Pro Gly Glu
    130                 135                 140

Gly Val Pro Gly Val Gly Val Pro Gly Val Gly Val Pro Gly Val Gly
145                 150                 155                 160

Val Pro Gly Val Gly Val Pro Gly Glu Gly Val Pro Gly Val Gly Val
                165                 170                 175

Pro Gly Val Gly Val Pro Gly Val Gly Val Pro Gly Val Gly Val Pro
            180                 185                 190

Gly Glu Gly Val Pro Gly Val Gly Val Pro Gly Val Gly Val Pro Gly
        195                 200                 205

Val Gly Val Pro Gly Val Gly Val Pro Gly Glu Gly Val Pro Gly Val
    210                 215                 220

Gly Val Pro Gly Val Gly Val Pro Gly Val Gly Val Pro Gly Val Gly
225                 230                 235                 240

Val Pro Gly Glu Gly Val Pro Gly Val Gly Val Pro Gly Val Gly Val

9935 sequence listing
245 250 255

Pro Gly Val Gly Val Pro Gly Val Gly Val Pro Gly Glu Gly Val Pro
260 265 270

Gly Val Gly Val Pro Gly Val Gly Val Pro Gly Val Gly Val Pro Gly
275 280 285

Val Gly Val Pro Gly Glu Gly Val Pro Gly Val Gly Val Pro Gly Val
290 295 300

Gly Val Pro Gly Val Gly Val Pro Gly Val Gly Val Pro Gly Glu Gly
305 310 315 320

Val Pro Gly Val Gly Val Pro Gly Val Gly Val Pro Gly Val Gly Val
325 330 335

Pro Gly Val Gly Val Pro Gly Glu Gly Val Pro Gly Val Gly Val Pro
340 345 350

Gly Val Gly Val Pro Gly Val Gly Val Pro Gly Val Gly Val Pro Gly
355 360 365

Glu Gly Val Pro Gly Val Gly Val Pro Gly Val Gly Val Pro Gly Val
370 375 380

Gly Val Pro Gly Val Gly Val Pro Gly Glu Gly Val Pro Gly Val Gly
385 390 395 400

Val Pro Gly Val Gly Val Pro Gly Val Gly Val Pro Gly Val Gly Val
405 410 415

Pro Gly Glu Gly Val Pro Gly Val Gly Val Pro Gly Val Gly Val Pro
420 425 430

Gly Val Gly Val Pro Gly Val Gly Val Pro Gly Glu Gly Val Pro Gly
435 440 445

Val Gly Val Pro Gly Val Gly Val Pro Gly Val Gly Val Pro Gly Val
450 455 460

Gly Val Pro Gly Glu Gly Val Pro Gly Val Gly Val Pro Gly Val Gly
465 470 475 480

Val Pro Gly Val Gly Val Pro Gly Val Gly Val Pro Gly Glu Gly Val
485 490 495

Pro Gly Val Gly Val Pro Gly Val Gly Val Pro Gly Val Gly Val Pro
500 505 510

Gly Val Gly Val Pro Gly Glu Gly Val Pro Gly Val Gly Val Pro Gly

9935 sequence listing
515                    520                    525

Val Gly Val Pro Gly Val Gly Val Pro Gly Val Gly Val Pro Gly Glu
    530                    535                    540

Gly Val Pro Gly Val Gly Val Pro Gly Val Gly Val Pro Gly Val Gly
545                    550                    555                    560

Val Pro Gly Val Gly Val Pro Gly Glu Gly Val Pro Gly Val Gly Val
                565                    570                    575

Pro Gly Val Gly Val Pro Gly Val Gly Val Pro Gly Val Gly Val Pro
            580                    585                    590

Gly Glu Gly Val Pro Gly Val Gly Val Pro Gly Val Gly Val Pro Gly
        595                    600                    605

Val Gly Val Pro Gly Val Gly Val Pro Gly Glu Gly Val Pro Gly Val
    610                    615                    620

Gly Val Pro Gly Val Gly Val Pro Gly Val Gly Val Pro Gly Val Gly
625                    630                    635                    640

Val Pro Gly Glu Gly Val Pro Gly Val Gly Val Pro Gly Val Gly Val
                645                    650                    655

Pro Gly Val Gly Val Pro Gly Val Gly Val Pro Gly Glu Gly Val Pro
            660                    665                    670

Gly Val Gly Val Pro Gly Val Gly Val Pro Gly Val Gly Val Pro Gly
        675                    680                    685

Val Gly Val Pro Gly Glu Gly Val Pro Gly Val Gly Val Pro Gly Val
    690                    695                    700

Gly Val Pro Gly Val Gly Val Pro Gly Val Gly Val Pro Gly Glu Gly
705                    710                    715                    720

Val Pro Gly Val Gly Val Pro Gly Val Gly Val Pro Gly Val Gly Val
                725                    730                    735

Pro Gly Val Gly Val Pro Gly Glu Gly Val Pro Gly Val Gly Val Pro
            740                    745                    750

Gly Val Gly Val Pro Gly Val Gly Val Pro Gly Val Gly Val Pro Gly
        755                    760                    765

Glu Gly Val Pro Gly Val Gly Val Pro Gly Val Gly Val Pro Gly Val
    770                    775                    780

Gly Val Pro Gly Val Gly Val Pro Gly Glu Gly Val Pro Gly Val Gly

9935 sequence listing
785                 790                 795                 800

Val Pro Gly Val Gly Val Pro Gly Val Gly Val Pro Gly Val Gly Val
                805                 810                 815

Pro Gly Glu Gly Val Pro Gly Val Gly Val Pro Gly Val Gly Val Pro
            820                 825                 830

Gly Val Gly Val Pro Gly Val Gly Val Pro Gly Glu Gly Val Pro Gly
        835                 840                 845

Val Gly Val Pro Gly Val Gly Val Pro Gly Val Gly Val Pro Gly Val
    850                 855                 860

Gly Val Pro Gly Glu Gly Val Pro Gly Val Gly Val Pro Gly Val Gly
865                 870                 875                 880

Val Pro Gly Val Gly Val Pro Gly Val Gly Val Pro Gly Glu Gly Val
                885                 890                 895

Pro Gly Val Gly Val Pro Gly Val Gly Val Pro Gly Val Gly Val Pro
            900                 905                 910

Gly Val Gly Val Pro Gly Glu Gly Val Pro Gly Val Gly Val Pro Gly
        915                 920                 925

Val Gly Val Pro Gly Val Gly Val Pro Gly Val Gly Val Pro Gly Glu
    930                 935                 940

Gly Val Pro Gly Val Gly Val Pro Gly Val Gly Val Pro Gly Val Gly
945                 950                 955                 960

<210>  25
<211>  1210
<212>  PRT
<213>  Artificial

<220>
<223>  Synthetic construct

<400>  25

Val Pro Gly Val Gly Val Pro Gly Val Gly Val Pro Gly Val Gly Val
1               5                   10                  15

Pro Gly Glu Gly Val Pro Gly Val Gly Val Pro Gly Val Gly Val Pro
            20                  25                  30

Gly Val Gly Val Pro Gly Val Gly Val Pro Gly Glu Gly Val Pro Gly
        35                  40                  45

Val Gly Val Pro Gly Val Gly Val Pro Gly Val Gly Val Pro Gly Val
    50                  55                  60

9935 sequence listing

Gly Val Pro Gly Glu Gly Val Pro Gly Val Gly Val Pro Gly Val Gly
65                  70                  75                  80

Val Pro Gly Val Gly Val Pro Gly Val Gly Val Pro Gly Glu Gly Val
                85                  90                  95

Pro Gly Val Gly Val Pro Gly Val Gly Val Pro Gly Val Gly Val Pro
            100                 105                 110

Gly Val Gly Val Pro Gly Glu Gly Val Pro Gly Val Gly Val Pro Gly
        115                 120                 125

Val Gly Val Pro Gly Val Gly Val Pro Gly Val Gly Val Pro Gly Glu
    130                 135                 140

Gly Val Pro Gly Val Gly Val Pro Gly Val Gly Val Pro Gly Val Gly
145                 150                 155                 160

Val Pro Gly Val Gly Val Pro Gly Glu Gly Val Pro Gly Val Gly Val
                165                 170                 175

Pro Gly Val Gly Val Pro Gly Val Gly Val Pro Gly Val Gly Val Pro
            180                 185                 190

Gly Glu Gly Val Pro Gly Val Gly Val Pro Gly Val Gly Val Pro Gly
        195                 200                 205

Val Gly Val Pro Gly Val Gly Val Pro Gly Glu Gly Val Pro Gly Val
    210                 215                 220

Gly Val Pro Gly Val Gly Val Pro Gly Val Gly Val Pro Gly Val Gly
225                 230                 235                 240

Val Pro Gly Glu Gly Val Pro Gly Val Gly Val Pro Gly Val Gly Val
                245                 250                 255

Pro Gly Val Gly Val Pro Gly Val Gly Val Pro Gly Glu Gly Val Pro
            260                 265                 270

Gly Val Gly Val Pro Gly Val Gly Val Pro Gly Val Gly Val Pro Gly
        275                 280                 285

Val Gly Val Pro Gly Glu Gly Val Pro Gly Val Gly Val Pro Gly Val
    290                 295                 300

Gly Val Pro Gly Val Gly Val Pro Gly Val Gly Val Pro Gly Glu Gly
305                 310                 315                 320

Val Pro Gly Val Gly Val Pro Gly Val Gly Val Pro Gly Val Gly Val
                325                 330                 335

9935 sequence listing

Pro Gly Val Gly Val Pro Gly Glu Gly Val Pro Gly Val Gly Val Pro
            340                 345                 350

Gly Val Gly Val Pro Gly Val Gly Val Pro Gly Val Gly Val Pro Gly
        355                 360                 365

Glu Gly Val Pro Gly Val Gly Val Pro Gly Val Gly Val Pro Gly Val
    370                 375                 380

Gly Val Pro Gly Val Gly Val Pro Gly Glu Gly Val Pro Gly Val Gly
385                 390                 395                 400

Val Pro Gly Val Gly Val Pro Gly Val Gly Val Pro Gly Val Gly Val
                405                 410                 415

Pro Gly Glu Gly Val Pro Gly Val Gly Val Pro Gly Val Gly Val Pro
            420                 425                 430

Gly Val Gly Val Pro Gly Val Gly Val Pro Gly Glu Gly Val Pro Gly
        435                 440                 445

Val Gly Val Pro Gly Val Gly Val Pro Gly Val Gly Val Pro Gly Val
    450                 455                 460

Gly Val Pro Gly Glu Gly Val Pro Gly Val Gly Val Pro Gly Val Gly
465                 470                 475                 480

Val Pro Gly Val Gly Val Pro Gly Val Gly Val Pro Gly Glu Gly Val
                485                 490                 495

Pro Gly Val Gly Val Pro Gly Val Gly Val Pro Gly Val Gly Val Pro
            500                 505                 510

Gly Val Gly Val Pro Gly Glu Gly Val Pro Gly Val Gly Val Pro Gly
        515                 520                 525

Val Gly Val Pro Gly Val Gly Val Pro Gly Val Gly Val Pro Gly Glu
    530                 535                 540

Gly Val Pro Gly Val Gly Val Pro Gly Val Gly Val Pro Gly Val Gly
545                 550                 555                 560

Val Pro Gly Val Gly Val Pro Gly Glu Gly Val Pro Gly Val Gly Val
                565                 570                 575

Pro Gly Val Gly Val Pro Gly Val Gly Val Pro Gly Val Gly Val Pro
            580                 585                 590

Gly Glu Gly Val Pro Gly Val Gly Val Pro Gly Val Gly Val Pro Gly
        595                 600                 605

9935 sequence listing

Val Gly Val Pro Gly Val Gly Val Pro Gly Glu Gly Val Pro Gly Val
    610                 615                 620

Gly Val Pro Gly Val Gly Val Pro Gly Val Gly Val Pro Gly Val Gly
625                 630                 635                 640

Val Pro Gly Glu Gly Val Pro Gly Val Gly Val Pro Gly Val Gly Val
                645                 650                 655

Pro Gly Val Gly Val Pro Gly Val Gly Val Pro Gly Glu Gly Val Pro
            660                 665                 670

Gly Val Gly Val Pro Gly Val Gly Val Pro Gly Val Gly Val Pro Gly
        675                 680                 685

Val Gly Val Pro Gly Glu Gly Val Pro Gly Val Gly Val Pro Gly Val
    690                 695                 700

Gly Val Pro Gly Val Gly Val Pro Gly Val Gly Val Pro Gly Glu Gly
705                 710                 715                 720

Val Pro Gly Val Gly Val Pro Gly Val Gly Val Pro Gly Val Gly Val
                725                 730                 735

Pro Gly Val Gly Val Pro Gly Glu Gly Val Pro Gly Val Gly Val Pro
            740                 745                 750

Gly Val Gly Val Pro Gly Val Gly Val Pro Gly Val Gly Val Pro Gly
        755                 760                 765

Glu Gly Val Pro Gly Val Gly Val Pro Gly Val Gly Val Pro Gly Val
    770                 775                 780

Gly Val Pro Gly Val Gly Val Pro Gly Glu Gly Val Pro Gly Val Gly
785                 790                 795                 800

Val Pro Gly Val Gly Val Pro Gly Val Gly Val Pro Gly Val Gly Val
                805                 810                 815

Pro Gly Glu Gly Val Pro Gly Val Gly Val Pro Gly Val Gly Val Pro
            820                 825                 830

Gly Val Gly Val Pro Gly Val Gly Val Pro Gly Glu Gly Val Pro Gly
        835                 840                 845

Val Gly Val Pro Gly Val Gly Val Pro Gly Val Gly Val Pro Gly Val
    850                 855                 860

Gly Val Pro Gly Glu Gly Val Pro Gly Val Gly Val Pro Gly Val Gly
865                 870                 875                 880

9935 sequence listing

Val Pro Gly Val Gly Val Pro Gly Val Gly Val Pro Gly Glu Gly Val
                885                 890                 895

Pro Gly Val Gly Val Pro Gly Val Gly Val Pro Gly Val Gly Val Pro
            900                 905                 910

Gly Val Gly Val Pro Gly Glu Gly Val Pro Gly Val Gly Val Pro Gly
        915                 920                 925

Val Gly Val Pro Gly Val Gly Val Pro Gly Val Gly Val Pro Gly Glu
    930                 935                 940

Gly Val Pro Gly Val Gly Val Pro Gly Val Gly Val Pro Gly Val Gly
945                 950                 955                 960

Val Pro Gly Val Gly Val Pro Gly Glu Gly Val Pro Gly Val Gly Val
                965                 970                 975

Pro Gly Val Gly Val Pro Gly Val Gly Val Pro Gly Val Gly Val Pro
            980                 985                 990

Gly Glu Gly Val Pro Gly Val Gly  Val Pro Gly Val Gly  Val Pro Gly
        995                 1000                 1005

Val Gly  Val Pro Gly Val Gly  Val Pro Gly Glu Gly  Val Pro Gly
    1010                 1015                 1020

Val Gly  Val Pro Gly Val Gly  Val Pro Gly Val Gly  Val Pro Gly
    1025                 1030                 1035

Val Gly  Val Pro Gly Glu Gly  Val Pro Gly Val Gly  Val Pro Gly
    1040                 1045                 1050

Val Gly  Val Pro Gly Val Gly  Val Pro Gly Val Gly  Val Pro Gly
    1055                 1060                 1065

Glu Gly  Val Pro Gly Val Gly  Val Pro Gly Val Gly  Val Pro Gly
    1070                 1075                 1080

Val Gly  Val Pro Gly Val Gly  Val Pro Gly Glu Gly  Val Pro Gly
    1085                 1090                 1095

Val Gly  Val Pro Gly Val Gly  Val Pro Gly Val Gly  Val Pro Gly
    1100                 1105                 1110

Val Gly  Val Pro Gly Glu Gly  Val Pro Gly Val Gly  Val Pro Gly
    1115                 1120                 1125

Val Gly  Val Pro Gly Val Gly  Val Pro Gly Val Gly  Val Pro Gly
    1130                 1135                 1140

9935 sequence listing

Glu Gly Val Pro Gly Val Gly Val Pro Gly Val Gly Val Pro Gly
    1145                1150                1155

Val Gly Val Pro Gly Val Gly Val Pro Gly Glu Gly Val Pro Gly
    1160                1165                1170

Val Gly Val Pro Gly Val Gly Val Pro Gly Val Gly Val Pro Gly
    1175                1180                1185

Val Gly Val Pro Gly Glu Gly Val Pro Gly Val Gly Val Pro Gly
    1190                1195                1200

Val Gly Val Pro Gly Val Gly
    1205                1210

<210> 26
<211> 35
<212> PRT
<213> Artificial

<220>
<223> Synthetic construct

<220>
<221> REPEAT
<222> (6)..(30)
<223> Repeat residues 6 to 30; total of 30 repeat units.
      VPGVG[(VPGVG)2 VPGEG (VPGVG)2]30 VPGVG

<400> 26

Val Pro Gly Val Gly Val Pro Gly Val Gly Val Pro Gly Val Gly Val
1               5                   10                  15

Pro Gly Glu Gly Val Pro Gly Val Gly Val Pro Gly Val Gly Val Pro
            20                  25                  30

Gly Val Gly
        35

<210> 27
<211> 35
<212> PRT
<213> Artificial

<220>
<223> Synthetic construct

<220>
<221> REPEAT
<222> (6)..(30)
<223> Repeat residues 6 to 30; total of 38 units.
      VPGVG[(VPGVG)2 VPGEG (VPGVG)2]38 VPGVG

<400> 27

Val Pro Gly Val Gly Val Pro Gly Val Gly Val Pro Gly Val Gly Val

9935 sequence listing
1               5                   10                  15

Pro Gly Glu Gly Val Pro Gly Val Gly Val Pro Gly Val Gly Val Pro
            20                  25                  30

Gly Val Gly
        35

<210>  28
<211>  35
<212>  PRT
<213>  Artificial

<220>
<223>  synthetic construct

<220>
<221>  REPEAT
<222>  (6)..(30)
<223>  Repeat residues 6 to 30; total of 48 units.
       VPGVG[(VPGVG)2 VPGEG (VPGVG)2]48 VPGVG

<400>  28

Val Pro Gly Val Gly Val Pro Gly Val Gly Val Pro Gly Val Gly Val
1               5                   10                  15

Pro Gly Glu Gly Val Pro Gly Val Gly Val Pro Gly Val Gly Val Pro
            20                  25                  30

Gly Val Gly
        35

<210>  29
<211>  35
<212>  PRT
<213>  Artificial

<220>
<223>  synthetic construct.

<220>
<221>  REPEAT
<222>  (6)..(30)
<223>  Repeat residues 6 to 30; total of 12 units.
       VPGVG [(VPGVG)(VPNVG)4]12 VPGVG

<400>  29

Val Pro Gly Val Gly Val Pro Gly Val Gly Val Pro Asn Val Gly Val
1               5                   10                  15

Pro Asn Val Gly Val Pro Asn Val Gly Val Pro Asn Val Gly Val Pro
            20                  25                  30

Gly Val Gly
        35

9935 sequence listing

<210> 30
<211> 310
<212> PRT
<213> Artificial

<220>
<223> Synthetic construct.

<400> 30

Val Pro Gly Val Gly Val Pro Gly Val Gly Val Pro Asn Val Gly Val
1               5                   10                  15

Pro Asn Val Gly Val Pro Asn Val Gly Val Pro Asn Val Gly Val Pro
            20                  25                  30

Gly Val Gly Val Pro Asn Val Gly Val Pro Asn Val Gly Val Pro Asn
        35                  40                  45

Val Gly Val Pro Asn Val Gly Val Pro Gly Val Gly Val Pro Asn Val
    50                  55                  60

Gly Val Pro Asn Val Gly Val Pro Asn Val Gly Val Pro Asn Val Gly
65                  70                  75                  80

Val Pro Gly Val Gly Val Pro Asn Val Gly Val Pro Asn Val Gly Val
                85                  90                  95

Pro Asn Val Gly Val Pro Asn Val Gly Val Pro Gly Val Gly Val Pro
            100                 105                 110

Asn Val Gly Val Pro Asn Val Gly Val Pro Asn Val Gly Val Pro Asn
        115                 120                 125

Val Gly Val Pro Gly Val Gly Val Pro Asn Val Gly Val Pro Asn Val
    130                 135                 140

Gly Val Pro Asn Val Gly Val Pro Asn Val Gly Val Pro Gly Val Gly
145                 150                 155                 160

Val Pro Asn Val Gly Val Pro Asn Val Gly Val Pro Asn Val Gly Val
                165                 170                 175

Pro Asn Val Gly Val Pro Gly Val Gly Val Pro Asn Val Gly Val Pro
            180                 185                 190

Asn Val Gly Val Pro Asn Val Gly Val Pro Asn Val Gly Val Pro Gly
        195                 200                 205

Val Gly Val Pro Asn Val Gly Val Pro Asn Val Gly Val Pro Asn Val
    210                 215                 220

Gly Val Pro Asn Val Gly Val Pro Gly Val Gly Val Pro Asn Val Gly

9935 sequence listing
225                 230                 235                 240

Val Pro Asn Val Gly Val Pro Asn Val Gly Val Pro Asn Val Gly Val
                245                 250                 255

Pro Gly Val Gly Val Pro Asn Val Gly Val Pro Asn Val Gly Val Pro
            260                 265                 270

Asn Val Gly Val Pro Asn Val Gly Val Pro Gly Val Gly Val Pro Asn
        275                 280                 285

Val Gly Val Pro Asn Val Gly Val Pro Asn Val Gly Val Pro Asn Val
    290                 295                 300

Gly Val Pro Gly Val Gly
305                 310

<210>  31
<211>  12
<212>  PRT
<213>  Artificial

<220>
<223>  Synthetic construct.

<220>
<221>  REPEAT
<222>  (1)..(12)

<400>  31

Ala Pro Gly Gly Val Pro Gly Gly Ala Pro Gly Gly
1               5                   10

<210>  32
<211>  22
<212>  PRT
<213>  Artificial

<220>
<223>  Synthetic construct.

<220>
<221>  REPEAT
<222>  (6)..(17)
<223>  Repeat residues 6 to 17; total of 2 x 23 = 46 units.
       VPGVG [(APGGVPGGAPGG)2]23 VPGVG

<400>  32

Val Pro Gly Val Gly Ala Pro Gly Gly Val Pro Gly Gly Ala Pro Gly
1               5                   10                  15

Gly Val Pro Gly Val Gly
            20

<210>  33

9935 sequence listing
<211>  562
<212>  PRT
<213>  Artificial

<220>
<223>  synthetic construct.

<400>  33

Val Pro Gly Val Gly Ala Pro Gly Gly Val Pro Gly Gly Ala Pro Gly
1               5                   10                  15

Gly Ala Pro Gly Gly Val Pro Gly Gly Ala Pro Gly Gly Ala Pro Gly
            20                  25                  30

Gly Val Pro Gly Gly Ala Pro Gly Gly Ala Pro Gly Gly Val Pro Gly
        35                  40                  45

Gly Ala Pro Gly Gly Ala Pro Gly Gly Val Pro Gly Gly Ala Pro Gly
    50                  55                  60

Gly Ala Pro Gly Gly Val Pro Gly Gly Ala Pro Gly Gly Ala Pro Gly
65                  70                  75                  80

Gly Val Pro Gly Gly Ala Pro Gly Gly Ala Pro Gly Gly Val Pro Gly
                85                  90                  95

Gly Ala Pro Gly Gly Ala Pro Gly Gly Val Pro Gly Gly Ala Pro Gly
            100                 105                 110

Gly Ala Pro Gly Gly Val Pro Gly Gly Ala Pro Gly Gly Ala Pro Gly
        115                 120                 125

Gly Val Pro Gly Gly Ala Pro Gly Gly Ala Pro Gly Gly Val Pro Gly
    130                 135                 140

Gly Ala Pro Gly Gly Ala Pro Gly Gly Val Pro Gly Gly Ala Pro Gly
145                 150                 155                 160

Gly Ala Pro Gly Gly Val Pro Gly Gly Ala Pro Gly Gly Ala Pro Gly
                165                 170                 175

Gly Val Pro Gly Gly Ala Pro Gly Gly Ala Pro Gly Gly Val Pro Gly
            180                 185                 190

Gly Ala Pro Gly Gly Ala Pro Gly Gly Val Pro Gly Gly Ala Pro Gly
        195                 200                 205

Gly Ala Pro Gly Gly Val Pro Gly Gly Ala Pro Gly Gly Ala Pro Gly
    210                 215                 220

Gly Val Pro Gly Gly Ala Pro Gly Gly Ala Pro Gly Gly Val Pro Gly
225                 230                 235                 240

9935 sequence listing

Gly Ala Pro Gly Gly Ala Pro Gly Gly Val Pro Gly Gly Ala Pro Gly
                245                 250                 255

Gly Ala Pro Gly Gly Val Pro Gly Gly Ala Pro Gly Gly Ala Pro Gly
            260                 265                 270

Gly Val Pro Gly Gly Ala Pro Gly Gly Ala Pro Gly Gly Val Pro Gly
        275                 280                 285

Gly Ala Pro Gly Gly Ala Pro Gly Gly Val Pro Gly Gly Ala Pro Gly
    290                 295                 300

Gly Ala Pro Gly Gly Val Pro Gly Gly Ala Pro Gly Gly Ala Pro Gly
305                 310                 315                 320

Gly Val Pro Gly Gly Ala Pro Gly Gly Ala Pro Gly Gly Val Pro Gly
                325                 330                 335

Gly Ala Pro Gly Gly Ala Pro Gly Gly Val Pro Gly Gly Ala Pro Gly
            340                 345                 350

Gly Ala Pro Gly Gly Val Pro Gly Gly Ala Pro Gly Gly Ala Pro Gly
        355                 360                 365

Gly Val Pro Gly Gly Ala Pro Gly Gly Ala Pro Gly Gly Val Pro Gly
    370                 375                 380

Gly Ala Pro Gly Gly Ala Pro Gly Gly Val Pro Gly Gly Ala Pro Gly
385                 390                 395                 400

Gly Ala Pro Gly Gly Val Pro Gly Gly Ala Pro Gly Gly Ala Pro Gly
                405                 410                 415

Gly Val Pro Gly Gly Ala Pro Gly Gly Ala Pro Gly Gly Val Pro Gly
            420                 425                 430

Gly Ala Pro Gly Gly Ala Pro Gly Gly Val Pro Gly Gly Ala Pro Gly
        435                 440                 445

Gly Ala Pro Gly Gly Val Pro Gly Gly Ala Pro Gly Gly Ala Pro Gly
    450                 455                 460

Gly Val Pro Gly Gly Ala Pro Gly Gly Ala Pro Gly Gly Val Pro Gly
465                 470                 475                 480

Gly Ala Pro Gly Gly Ala Pro Gly Gly Val Pro Gly Gly Ala Pro Gly
                485                 490                 495

Gly Ala Pro Gly Gly Val Pro Gly Gly Ala Pro Gly Gly Ala Pro Gly
            500                 505                 510

9935 sequence listing

Gly Val Pro Gly Gly Ala Pro Gly Gly Ala Pro Gly Gly Val Pro Gly
        515                 520                 525

Gly Ala Pro Gly Gly Ala Pro Gly Gly Val Pro Gly Gly Ala Pro Gly
    530                 535                 540

Gly Ala Pro Gly Gly Val Pro Gly Gly Ala Pro Gly Gly Val Pro Gly
545                 550                 555                 560

Val Gly

<210>  34
<211>  22
<212>  PRT
<213>  Artificial

<220>
<223>  Synthetic construct.

<220>
<221>  REPEAT
<222>  (6)..(17)
<223>  Repeat residues 6 to 17; total of 2 x 30 = 60 units.
       VPGVG [(APGGVPGGAPGG)2]30 VPGVG

<400>  34

Val Pro Gly Val Gly Ala Pro Gly Gly Val Pro Gly Gly Ala Pro Gly
1               5                   10                  15

Gly Val Pro Gly Val Gly
            20

<210>  35
<211>  730
<212>  PRT
<213>  Artificial

<220>
<223>  Synthetic construct.

<400>  35

Val Pro Gly Val Gly Ala Pro Gly Gly Val Pro Gly Gly Ala Pro Gly
1               5                   10                  15

Gly Ala Pro Gly Gly Val Pro Gly Gly Ala Pro Gly Gly Ala Pro Gly
            20                  25                  30

Gly Val Pro Gly Gly Ala Pro Gly Gly Ala Pro Gly Gly Val Pro Gly
        35                  40                  45

Gly Ala Pro Gly Gly Ala Pro Gly Gly Val Pro Gly Gly Ala Pro Gly
    50                  55                  60

9935 sequence listing
Gly Ala Pro Gly Gly Val Pro Gly Gly Ala Pro Gly Gly Ala Pro Gly
65                  70                  75                  80

Gly Val Pro Gly Gly Ala Pro Gly Gly Ala Pro Gly Gly Val Pro Gly
                85                  90                  95

Gly Ala Pro Gly Gly Ala Pro Gly Gly Val Pro Gly Gly Ala Pro Gly
            100                 105                 110

Gly Ala Pro Gly Gly Val Pro Gly Gly Ala Pro Gly Gly Ala Pro Gly
        115                 120                 125

Gly Val Pro Gly Gly Ala Pro Gly Gly Ala Pro Gly Gly Val Pro Gly
    130                 135                 140

Gly Ala Pro Gly Gly Ala Pro Gly Gly Val Pro Gly Gly Ala Pro Gly
145                 150                 155                 160

Gly Ala Pro Gly Gly Val Pro Gly Gly Ala Pro Gly Gly Ala Pro Gly
                165                 170                 175

Gly Val Pro Gly Gly Ala Pro Gly Gly Ala Pro Gly Gly Val Pro Gly
            180                 185                 190

Gly Ala Pro Gly Gly Ala Pro Gly Gly Val Pro Gly Gly Ala Pro Gly
        195                 200                 205

Gly Ala Pro Gly Gly Val Pro Gly Gly Ala Pro Gly Gly Ala Pro Gly
    210                 215                 220

Gly Val Pro Gly Gly Ala Pro Gly Gly Ala Pro Gly Gly Val Pro Gly
225                 230                 235                 240

Gly Ala Pro Gly Gly Ala Pro Gly Gly Val Pro Gly Gly Ala Pro Gly
                245                 250                 255

Gly Ala Pro Gly Gly Val Pro Gly Gly Ala Pro Gly Gly Ala Pro Gly
            260                 265                 270

Gly Val Pro Gly Gly Ala Pro Gly Gly Ala Pro Gly Gly Val Pro Gly
        275                 280                 285

Gly Ala Pro Gly Gly Ala Pro Gly Gly Val Pro Gly Gly Ala Pro Gly
    290                 295                 300

Gly Ala Pro Gly Gly Val Pro Gly Gly Ala Pro Gly Gly Ala Pro Gly
305                 310                 315                 320

Gly Val Pro Gly Gly Ala Pro Gly Gly Ala Pro Gly Gly Val Pro Gly
                325                 330                 335

9935 sequence listing
Gly Ala Pro Gly Gly Ala Pro Gly Gly Val Pro Gly Gly Ala Pro Gly
            340                 345                 350

Gly Ala Pro Gly Gly Val Pro Gly Gly Ala Pro Gly Gly Ala Pro Gly
        355                 360                 365

Gly Val Pro Gly Gly Ala Pro Gly Gly Ala Pro Gly Gly Val Pro Gly
    370                 375                 380

Gly Ala Pro Gly Gly Ala Pro Gly Gly Val Pro Gly Gly Ala Pro Gly
385                 390                 395                 400

Gly Ala Pro Gly Gly Val Pro Gly Gly Ala Pro Gly Gly Ala Pro Gly
                405                 410                 415

Gly Val Pro Gly Gly Ala Pro Gly Gly Ala Pro Gly Gly Val Pro Gly
            420                 425                 430

Gly Ala Pro Gly Gly Ala Pro Gly Gly Val Pro Gly Gly Ala Pro Gly
        435                 440                 445

Gly Ala Pro Gly Gly Val Pro Gly Gly Ala Pro Gly Gly Ala Pro Gly
    450                 455                 460

Gly Val Pro Gly Gly Ala Pro Gly Gly Ala Pro Gly Gly Val Pro Gly
465                 470                 475                 480

Gly Ala Pro Gly Gly Ala Pro Gly Gly Val Pro Gly Gly Ala Pro Gly
                485                 490                 495

Gly Ala Pro Gly Gly Val Pro Gly Gly Ala Pro Gly Gly Ala Pro Gly
            500                 505                 510

Gly Val Pro Gly Gly Ala Pro Gly Gly Ala Pro Gly Gly Val Pro Gly
        515                 520                 525

Gly Ala Pro Gly Gly Ala Pro Gly Gly Val Pro Gly Gly Ala Pro Gly
    530                 535                 540

Gly Ala Pro Gly Gly Val Pro Gly Gly Ala Pro Gly Gly Ala Pro Gly
545                 550                 555                 560

Gly Val Pro Gly Gly Ala Pro Gly Gly Ala Pro Gly Gly Val Pro Gly
                565                 570                 575

Gly Ala Pro Gly Gly Ala Pro Gly Gly Val Pro Gly Gly Ala Pro Gly
            580                 585                 590

Gly Ala Pro Gly Gly Val Pro Gly Gly Ala Pro Gly Gly Ala Pro Gly
        595                 600                 605

9935 sequence listing
Gly Val Pro Gly Gly Ala Pro Gly Gly Ala Pro Gly Gly Val Pro Gly
    610                 615                 620

Gly Ala Pro Gly Gly Ala Pro Gly Gly Val Pro Gly Gly Ala Pro Gly
625                 630                 635                 640

Gly Ala Pro Gly Gly Val Pro Gly Gly Ala Pro Gly Gly Ala Pro Gly
                645                 650                 655

Gly Val Pro Gly Gly Ala Pro Gly Gly Ala Pro Gly Gly Val Pro Gly
            660                 665                 670

Gly Ala Pro Gly Gly Ala Pro Gly Gly Val Pro Gly Gly Ala Pro Gly
        675                 680                 685

Gly Ala Pro Gly Gly Val Pro Gly Gly Ala Pro Gly Gly Ala Pro Gly
    690                 695                 700

Gly Val Pro Gly Gly Ala Pro Gly Gly Ala Pro Gly Gly Val Pro Gly
705                 710                 715                 720

Gly Ala Pro Gly Gly Val Pro Gly Val Gly
                725                 730

<210>  36
<211>  10
<212>  PRT
<213>  Artificial

<220>
<223>  Synthetic construct.

<400>  36

Ile Pro Gly Val Gly Val Pro Gly Val Gly
1               5                   10

<210>  37
<211>  25
<212>  PRT
<213>  Artificial

<220>
<223>  Synthetic construct.

<220>
<221>  REPEAT
<222>  (1)..(25)

<400>  37

Val Pro Gly Val Gly Ile Pro Gly Val Gly Val Pro Gly Val Gly Ile
1               5                   10                  15

Pro Gly Val Gly Val Pro Gly Val Gly
            20                  25

9935 sequence listing

<210>  38
<211>  475
<212>  PRT
<213>  Artificial

<220>
<223>  Synthetic construct.

<400>  38

Val Pro Gly Val Gly Ile Pro Gly Val Gly Val Pro Gly Val Gly Ile
1               5                   10                  15

Pro Gly Val Gly Val Pro Gly Val Gly Val Pro Gly Val Gly Ile Pro
            20                  25                  30

Gly Val Gly Val Pro Gly Val Gly Ile Pro Gly Val Gly Val Pro Gly
        35                  40                  45

Val Gly Val Pro Gly Val Gly Ile Pro Gly Val Gly Val Pro Gly Val
    50                  55                  60

Gly Ile Pro Gly Val Gly Val Pro Gly Val Gly Val Pro Gly Val Gly
65                  70                  75                  80

Ile Pro Gly Val Gly Val Pro Gly Val Gly Ile Pro Gly Val Gly Val
                85                  90                  95

Pro Gly Val Gly Val Pro Gly Val Gly Ile Pro Gly Val Gly Val Pro
            100                 105                 110

Gly Val Gly Ile Pro Gly Val Gly Val Pro Gly Val Gly Val Pro Gly
        115                 120                 125

Val Gly Ile Pro Gly Val Gly Val Pro Gly Val Gly Ile Pro Gly Val
    130                 135                 140

Gly Val Pro Gly Val Gly Val Pro Gly Val Gly Ile Pro Gly Val Gly
145                 150                 155                 160

Val Pro Gly Val Gly Ile Pro Gly Val Gly Val Pro Gly Val Gly Val
                165                 170                 175

Pro Gly Val Gly Ile Pro Gly Val Gly Val Pro Gly Val Gly Ile Pro
            180                 185                 190

Gly Val Gly Val Pro Gly Val Gly Val Pro Gly Val Gly Ile Pro Gly
        195                 200                 205

Val Gly Val Pro Gly Val Gly Ile Pro Gly Val Gly Val Pro Gly Val
    210                 215                 220

Gly Val Pro Gly Val Gly Ile Pro Gly Val Gly Val Pro Gly Val Gly

9935 sequence listing
225                 230                 235                 240

Ile Pro Gly Val Gly Val Pro Gly Val Gly Val Pro Gly Val Gly Ile
                245                 250                 255

Pro Gly Val Gly Val Pro Gly Val Gly Ile Pro Gly Val Gly Val Pro
            260                 265                 270

Gly Val Gly Val Pro Gly Val Gly Ile Pro Gly Val Gly Val Pro Gly
        275                 280                 285

Val Gly Ile Pro Gly Val Gly Val Pro Gly Val Gly Val Pro Gly Val
    290                 295                 300

Gly Ile Pro Gly Val Gly Val Pro Gly Val Gly Ile Pro Gly Val Gly
305                 310                 315                 320

Val Pro Gly Val Gly Val Pro Gly Val Gly Ile Pro Gly Val Gly Val
                325                 330                 335

Pro Gly Val Gly Ile Pro Gly Val Gly Val Pro Gly Val Gly Val Pro
            340                 345                 350

Gly Val Gly Ile Pro Gly Val Gly Val Pro Gly Val Gly Ile Pro Gly
        355                 360                 365

Val Gly Val Pro Gly Val Gly Val Pro Gly Val Gly Ile Pro Gly Val
    370                 375                 380

Gly Val Pro Gly Val Gly Ile Pro Gly Val Gly Val Pro Gly Val Gly
385                 390                 395                 400

Val Pro Gly Val Gly Ile Pro Gly Val Gly Val Pro Gly Val Gly Ile
                405                 410                 415

Pro Gly Val Gly Val Pro Gly Val Gly Val Pro Gly Val Gly Ile Pro
            420                 425                 430

Gly Val Gly Val Pro Gly Val Gly Ile Pro Gly Val Gly Val Pro Gly
        435                 440                 445

Val Gly Val Pro Gly Val Gly Ile Pro Gly Val Gly Val Pro Gly Val
    450                 455                 460

Gly Ile Pro Gly Val Gly Val Pro Gly Val Gly
465                 470                 475

<210>  39
<211>  10
<212>  DNA
<213>  Artificial

9935 sequence listing
<220>
<223>  Synthetic construct

<220>
<221>  misc_feature
<222>  (7)..(10)
<223>  n is a, c, g, or t

<400>  39
ctcttcnnnn                                                              10

<210>  40
<211>  25
<212>  PRT
<213>  Artificial

<220>
<223>  Synthetic construct.

<220>
<221>  REPEAT
<222>  (1)..(25)

<400>  40

Val Pro Gly Glu Gly Val Pro Gly Val Gly Val Pro Gly Val Gly Val
1               5                   10                  15

Pro Gly Val Gly Val Pro Gly Val Gly
            20                  25

<210>  41
<211>  750
<212>  PRT
<213>  Artificial

<220>
<223>  Synthetic construct.

<400>  41

Val Pro Gly Glu Gly Val Pro Gly Val Gly Val Pro Gly Val Gly Val
1               5                   10                  15

Pro Gly Val Gly Val Pro Gly Val Gly Val Pro Gly Glu Gly Val Pro
            20                  25                  30

Gly Val Gly Val Pro Gly Val Gly Val Pro Gly Val Gly Val Pro Gly
        35                  40                  45

Val Gly Val Pro Gly Glu Gly Val Pro Gly Val Gly Val Pro Gly Val
    50                  55                  60

Gly Val Pro Gly Val Gly Val Pro Gly Val Gly Val Pro Gly Glu Gly
65                  70                  75                  80

Val Pro Gly Val Gly Val Pro Gly Val Gly Val Pro Gly Val Gly Val
                85                  90                  95

9935 sequence listing

Pro Gly Val Gly Val Pro Gly Glu Gly Val Pro Gly Val Gly Val Pro
            100                 105                 110

Gly Val Gly Val Pro Gly Val Gly Val Pro Gly Val Gly Val Pro Gly
        115                 120                 125

Glu Gly Val Pro Gly Val Gly Val Pro Gly Val Gly Val Pro Gly Val
    130                 135                 140

Gly Val Pro Gly Val Gly Val Pro Gly Glu Gly Val Pro Gly Val Gly
145                 150                 155                 160

Val Pro Gly Val Gly Val Pro Gly Val Gly Val Pro Gly Val Gly Val
                165                 170                 175

Pro Gly Glu Gly Val Pro Gly Val Gly Val Pro Gly Val Gly Val Pro
            180                 185                 190

Gly Val Gly Val Pro Gly Val Gly Val Pro Gly Glu Gly Val Pro Gly
        195                 200                 205

Val Gly Val Pro Gly Val Gly Val Pro Gly Val Gly Val Pro Gly Val
    210                 215                 220

Gly Val Pro Gly Glu Gly Val Pro Gly Val Gly Val Pro Gly Val Gly
225                 230                 235                 240

Val Pro Gly Val Gly Val Pro Gly Val Gly Val Pro Gly Glu Gly Val
                245                 250                 255

Pro Gly Val Gly Val Pro Gly Val Gly Val Pro Gly Val Gly Val Pro
            260                 265                 270

Gly Val Gly Val Pro Gly Glu Gly Val Pro Gly Val Gly Val Pro Gly
        275                 280                 285

Val Gly Val Pro Gly Val Gly Val Pro Gly Val Gly Val Pro Gly Glu
    290                 295                 300

Gly Val Pro Gly Val Gly Val Pro Gly Val Gly Val Pro Gly Val Gly
305                 310                 315                 320

Val Pro Gly Val Gly Val Pro Gly Glu Gly Val Pro Gly Val Gly Val
                325                 330                 335

Pro Gly Val Gly Val Pro Gly Val Gly Val Pro Gly Val Gly Val Pro
            340                 345                 350

Gly Glu Gly Val Pro Gly Val Gly Val Pro Gly Val Gly Val Pro Gly
        355                 360                 365

9935 sequence listing

Val Gly Val Pro Gly Val Gly Val Pro Gly Glu Gly Val Pro Gly Val
    370                 375                 380

Gly Val Pro Gly Val Gly Val Pro Gly Val Gly Val Pro Gly Val Gly
385                 390                 395                 400

Val Pro Gly Glu Gly Val Pro Gly Val Gly Val Pro Gly Val Gly Val
                405                 410                 415

Pro Gly Val Gly Val Pro Gly Val Gly Val Pro Gly Glu Gly Val Pro
            420                 425                 430

Gly Val Gly Val Pro Gly Val Gly Val Pro Gly Val Gly Val Pro Gly
        435                 440                 445

Val Gly Val Pro Gly Glu Gly Val Pro Gly Val Gly Val Pro Gly Val
    450                 455                 460

Gly Val Pro Gly Val Gly Val Pro Gly Val Gly Val Pro Gly Glu Gly
465                 470                 475                 480

Val Pro Gly Val Gly Val Pro Gly Val Gly Val Pro Gly Val Gly Val
                485                 490                 495

Pro Gly Val Gly Val Pro Gly Glu Gly Val Pro Gly Val Gly Val Pro
            500                 505                 510

Gly Val Gly Val Pro Gly Val Gly Val Pro Gly Val Gly Val Pro Gly
        515                 520                 525

Glu Gly Val Pro Gly Val Gly Val Pro Gly Val Gly Val Pro Gly Val
    530                 535                 540

Gly Val Pro Gly Val Gly Val Pro Gly Glu Gly Val Pro Gly Val Gly
545                 550                 555                 560

Val Pro Gly Val Gly Val Pro Gly Val Gly Val Pro Gly Val Gly Val
                565                 570                 575

Pro Gly Glu Gly Val Pro Gly Val Gly Val Pro Gly Val Gly Val Pro
            580                 585                 590

Gly Val Gly Val Pro Gly Val Gly Val Pro Gly Glu Gly Val Pro Gly
        595                 600                 605

Val Gly Val Pro Gly Val Gly Val Pro Gly Val Gly Val Pro Gly Val
    610                 615                 620

Gly Val Pro Gly Glu Gly Val Pro Gly Val Gly Val Pro Gly Val Gly
625                 630                 635                 640

9935 sequence listing

Val Pro Gly Val Gly Val Pro Gly Val Gly Val Pro Gly Glu Gly Val
                645                 650                 655

Pro Gly Val Gly Val Pro Gly Val Gly Val Pro Gly Val Gly Val Pro
            660                 665                 670

Gly Val Gly Val Pro Gly Glu Gly Val Pro Gly Val Gly Val Pro Gly
        675                 680                 685

Val Gly Val Pro Gly Val Gly Val Pro Gly Val Gly Val Pro Gly Glu
    690                 695                 700

Gly Val Pro Gly Val Gly Val Pro Gly Val Gly Val Pro Gly Val Gly
705                 710                 715                 720

Val Pro Gly Val Gly Val Pro Gly Glu Gly Val Pro Gly Val Gly Val
                725                 730                 735

Pro Gly Val Gly Val Pro Gly Val Gly Val Pro Gly Val Gly
            740                 745                 750

<210>  42
<211>  1200
<212>  PRT
<213>  Artificial

<220>
<223>  Synthetic construct.

<400>  42

Val Pro Gly Glu Gly Val Pro Gly Val Gly Val Pro Gly Val Gly Val
1               5                   10                  15

Pro Gly Val Gly Val Pro Gly Val Gly Val Pro Gly Glu Gly Val Pro
            20                  25                  30

Gly Val Gly Val Pro Gly Val Gly Val Pro Gly Val Gly Val Pro Gly
        35                  40                  45

Val Gly Val Pro Gly Glu Gly Val Pro Gly Val Gly Val Pro Gly Val
    50                  55                  60

Gly Val Pro Gly Val Gly Val Pro Gly Val Gly Val Pro Gly Glu Gly
65                  70                  75                  80

Val Pro Gly Val Gly Val Pro Gly Val Gly Val Pro Gly Val Gly Val
                85                  90                  95

Pro Gly Val Gly Val Pro Gly Glu Gly Val Pro Gly Val Gly Val Pro
            100                 105                 110

9935 sequence listing
Gly Val Gly Val Pro Gly Val Gly Val Pro Gly Val Gly Val Pro Gly
        115                 120                 125

Glu Gly Val Pro Gly Val Gly Val Pro Gly Val Gly Val Pro Gly Val
    130                 135                 140

Gly Val Pro Gly Val Gly Val Pro Gly Glu Gly Val Pro Gly Val Gly
145                 150                 155                 160

Val Pro Gly Val Gly Val Pro Gly Val Gly Val Pro Gly Val Gly Val
                165                 170                 175

Pro Gly Glu Gly Val Pro Gly Val Gly Val Pro Gly Val Gly Val Pro
            180                 185                 190

Gly Val Gly Val Pro Gly Val Gly Val Pro Gly Glu Gly Val Pro Gly
        195                 200                 205

Val Gly Val Pro Gly Val Gly Val Pro Gly Val Gly Val Pro Gly Val
    210                 215                 220

Gly Val Pro Gly Glu Gly Val Pro Gly Val Gly Val Pro Gly Val Gly
225                 230                 235                 240

Val Pro Gly Val Gly Val Pro Gly Val Gly Val Pro Gly Glu Gly Val
                245                 250                 255

Pro Gly Val Gly Val Pro Gly Val Gly Val Pro Gly Val Gly Val Pro
            260                 265                 270

Gly Val Gly Val Pro Gly Glu Gly Val Pro Gly Val Gly Val Pro Gly
        275                 280                 285

Val Gly Val Pro Gly Val Gly Val Pro Gly Val Gly Val Pro Gly Glu
    290                 295                 300

Gly Val Pro Gly Val Gly Val Pro Gly Val Gly Val Pro Gly Val Gly
305                 310                 315                 320

Val Pro Gly Val Gly Val Pro Gly Glu Gly Val Pro Gly Val Gly Val
                325                 330                 335

Pro Gly Val Gly Val Pro Gly Val Gly Val Pro Gly Val Gly Val Pro
            340                 345                 350

Gly Glu Gly Val Pro Gly Val Gly Val Pro Gly Val Gly Val Pro Gly
        355                 360                 365

Val Gly Val Pro Gly Val Gly Val Pro Gly Glu Gly Val Pro Gly Val
    370                 375                 380

9935 sequence listing
Gly Val Pro Gly Val Gly Val Pro Gly Val Gly Val Pro Gly Val Gly
385                 390                 395                 400

Val Pro Gly Glu Gly Val Pro Gly Val Gly Val Pro Gly Val Gly Val
                405                 410                 415

Pro Gly Val Gly Val Pro Gly Val Gly Val Pro Gly Glu Gly Val Pro
            420                 425                 430

Gly Val Gly Val Pro Gly Val Gly Val Pro Gly Val Gly Val Pro Gly
        435                 440                 445

Val Gly Val Pro Gly Glu Gly Val Pro Gly Val Gly Val Pro Gly Val
    450                 455                 460

Gly Val Pro Gly Val Gly Val Pro Gly Val Gly Val Pro Gly Glu Gly
465                 470                 475                 480

Val Pro Gly Val Gly Val Pro Gly Val Gly Val Pro Gly Val Gly Val
                485                 490                 495

Pro Gly Val Gly Val Pro Gly Glu Gly Val Pro Gly Val Gly Val Pro
            500                 505                 510

Gly Val Gly Val Pro Gly Val Gly Val Pro Gly Val Gly Val Pro Gly
        515                 520                 525

Glu Gly Val Pro Gly Val Gly Val Pro Gly Val Gly Val Pro Gly Val
    530                 535                 540

Gly Val Pro Gly Val Gly Val Pro Gly Glu Gly Val Pro Gly Val Gly
545                 550                 555                 560

Val Pro Gly Val Gly Val Pro Gly Val Gly Val Pro Gly Val Gly Val
                565                 570                 575

Pro Gly Glu Gly Val Pro Gly Val Gly Val Pro Gly Val Gly Val Pro
            580                 585                 590

Gly Val Gly Val Pro Gly Val Gly Val Pro Gly Glu Gly Val Pro Gly
        595                 600                 605

Val Gly Val Pro Gly Val Gly Val Pro Gly Val Gly Val Pro Gly Val
    610                 615                 620

Gly Val Pro Gly Glu Gly Val Pro Gly Val Gly Val Pro Gly Val Gly
625                 630                 635                 640

Val Pro Gly Val Gly Val Pro Gly Val Gly Val Pro Gly Glu Gly Val
                645                 650                 655

9935 sequence listing
Pro Gly Val Gly Val Pro Gly Val Gly Val Pro Gly Val Gly Val Pro
            660                 665                 670

Gly Val Gly Val Pro Gly Glu Gly Val Pro Gly Val Gly Val Pro Gly
        675                 680                 685

Val Gly Val Pro Gly Val Gly Val Pro Gly Val Gly Val Pro Gly Glu
    690                 695                 700

Gly Val Pro Gly Val Gly Val Pro Gly Val Gly Val Pro Gly Val Gly
705                 710                 715                 720

Val Pro Gly Val Gly Val Pro Gly Glu Gly Val Pro Gly Val Gly Val
                725                 730                 735

Pro Gly Val Gly Val Pro Gly Val Gly Val Pro Gly Val Gly Val Pro
            740                 745                 750

Gly Glu Gly Val Pro Gly Val Gly Val Pro Gly Val Gly Val Pro Gly
        755                 760                 765

Val Gly Val Pro Gly Val Gly Val Pro Gly Glu Gly Val Pro Gly Val
    770                 775                 780

Gly Val Pro Gly Val Gly Val Pro Gly Val Gly Val Pro Gly Val Gly
785                 790                 795                 800

Val Pro Gly Glu Gly Val Pro Gly Val Gly Val Pro Gly Val Gly Val
                805                 810                 815

Pro Gly Val Gly Val Pro Gly Val Gly Val Pro Gly Glu Gly Val Pro
            820                 825                 830

Gly Val Gly Val Pro Gly Val Gly Val Pro Gly Val Gly Val Pro Gly
        835                 840                 845

Val Gly Val Pro Gly Glu Gly Val Pro Gly Val Gly Val Pro Gly Val
    850                 855                 860

Gly Val Pro Gly Val Gly Val Pro Gly Val Gly Val Pro Gly Glu Gly
865                 870                 875                 880

Val Pro Gly Val Gly Val Pro Gly Val Gly Val Pro Gly Val Gly Val
                885                 890                 895

Pro Gly Val Gly Val Pro Gly Glu Gly Val Pro Gly Val Gly Val Pro
            900                 905                 910

Gly Val Gly Val Pro Gly Val Gly Val Pro Gly Val Gly Val Pro Gly
        915                 920                 925

9935 sequence listing
Glu Gly Val Pro Gly Val Gly Val Pro Gly Val Gly Val Pro Gly Val
    930                 935                 940

Gly Val Pro Gly Val Gly Val Pro Gly Glu Gly Val Pro Gly Val Gly
945                 950                 955                 960

Val Pro Gly Val Gly Val Pro Gly Val Gly Val Pro Gly Val Gly Val
                965                 970                 975

Pro Gly Glu Gly Val Pro Gly Val Gly Val Pro Gly Val Gly Val Pro
            980                 985                 990

Gly Val Gly Val Pro Gly Val Gly  Val Pro Gly Glu Gly  Val Pro Gly
        995                 1000                 1005

Val Gly  Val Pro Gly Val Gly  Val Pro Gly Val Gly  Val Pro Gly
    1010                 1015                 1020

Val Gly  Val Pro Gly Glu Gly  Val Pro Gly Val Gly  Val Pro Gly
    1025                 1030                 1035

Val Gly  Val Pro Gly Val Gly  Val Pro Gly Val Gly  Val Pro Gly
    1040                 1045                 1050

Glu Gly  Val Pro Gly Val Gly  Val Pro Gly Val Gly  Val Pro Gly
    1055                 1060                 1065

Val Gly  Val Pro Gly Val Gly  Val Pro Gly Glu Gly  Val Pro Gly
    1070                 1075                 1080

Val Gly  Val Pro Gly Val Gly  Val Pro Gly Val Gly  Val Pro Gly
    1085                 1090                 1095

Val Gly  Val Pro Gly Glu Gly  Val Pro Gly Val Gly  Val Pro Gly
    1100                 1105                 1110

Val Gly  Val Pro Gly Val Gly  Val Pro Gly Val Gly  Val Pro Gly
    1115                 1120                 1125

Glu Gly  Val Pro Gly Val Gly  Val Pro Gly Val Gly  Val Pro Gly
    1130                 1135                 1140

Val Gly  Val Pro Gly Val Gly  Val Pro Gly Glu Gly  Val Pro Gly
    1145                 1150                 1155

Val Gly  Val Pro Gly Val Gly  Val Pro Gly Val Gly  Val Pro Gly
    1160                 1165                 1170

Val Gly  Val Pro Gly Glu Gly  Val Pro Gly Val Gly  Val Pro Gly
    1175                 1180                 1185

9935 sequence listing
Val Gly Val Pro Gly Val Gly Val Pro Gly Val Gly
1190 1195 1200

<210> 43
<211> 528
<212> PRT
<213> Artificial

<220>
<223> Synthetic construct.

<400> 43

Ala Pro Gly Gly Val Pro Gly Gly Ala Pro Gly Gly Ala Pro Gly Gly
1               5                   10                  15

Val Pro Gly Gly Ala Pro Gly Gly Ala Pro Gly Gly Val Pro Gly Gly
            20                  25                  30

Ala Pro Gly Gly Ala Pro Gly Gly Val Pro Gly Gly Ala Pro Gly Gly
        35                  40                  45

Ala Pro Gly Gly Val Pro Gly Gly Ala Pro Gly Gly Ala Pro Gly Gly
    50                  55                  60

Val Pro Gly Gly Ala Pro Gly Gly Ala Pro Gly Gly Val Pro Gly Gly
65                  70                  75                  80

Ala Pro Gly Gly Ala Pro Gly Gly Val Pro Gly Gly Ala Pro Gly Gly
                85                  90                  95

Ala Pro Gly Gly Val Pro Gly Gly Ala Pro Gly Gly Ala Pro Gly Gly
            100                 105                 110

Val Pro Gly Gly Ala Pro Gly Gly Ala Pro Gly Gly Val Pro Gly Gly
        115                 120                 125

Ala Pro Gly Gly Ala Pro Gly Gly Val Pro Gly Gly Ala Pro Gly Gly
    130                 135                 140

Ala Pro Gly Gly Val Pro Gly Gly Ala Pro Gly Gly Ala Pro Gly Gly
145                 150                 155                 160

Val Pro Gly Gly Ala Pro Gly Gly Ala Pro Gly Gly Val Pro Gly Gly
                165                 170                 175

Ala Pro Gly Gly Ala Pro Gly Gly Val Pro Gly Gly Ala Pro Gly Gly
            180                 185                 190

Ala Pro Gly Gly Val Pro Gly Gly Ala Pro Gly Gly Ala Pro Gly Gly
        195                 200                 205

Val Pro Gly Gly Ala Pro Gly Gly Ala Pro Gly Gly Val Pro Gly Gly
    210                 215                 220

9935 sequence listing

Ala Pro Gly Gly Ala Pro Gly Gly Val Pro Gly Gly Ala Pro Gly Gly
225                 230                 235                 240

Ala Pro Gly Gly Val Pro Gly Gly Ala Pro Gly Gly Ala Pro Gly Gly
                245                 250                 255

Val Pro Gly Gly Ala Pro Gly Gly Ala Pro Gly Gly Val Pro Gly Gly
            260                 265                 270

Ala Pro Gly Gly Ala Pro Gly Gly Val Pro Gly Gly Ala Pro Gly Gly
        275                 280                 285

Ala Pro Gly Gly Val Pro Gly Gly Ala Pro Gly Gly Ala Pro Gly Gly
    290                 295                 300

Val Pro Gly Gly Ala Pro Gly Gly Ala Pro Gly Gly Val Pro Gly Gly
305                 310                 315                 320

Ala Pro Gly Gly Ala Pro Gly Gly Val Pro Gly Gly Ala Pro Gly Gly
                325                 330                 335

Ala Pro Gly Gly Val Pro Gly Gly Ala Pro Gly Gly Ala Pro Gly Gly
            340                 345                 350

Val Pro Gly Gly Ala Pro Gly Gly Ala Pro Gly Gly Val Pro Gly Gly
        355                 360                 365

Ala Pro Gly Gly Ala Pro Gly Gly Val Pro Gly Gly Ala Pro Gly Gly
    370                 375                 380

Ala Pro Gly Gly Val Pro Gly Gly Ala Pro Gly Gly Ala Pro Gly Gly
385                 390                 395                 400

Val Pro Gly Gly Ala Pro Gly Gly Ala Pro Gly Gly Val Pro Gly Gly
                405                 410                 415

Ala Pro Gly Gly Ala Pro Gly Gly Val Pro Gly Gly Ala Pro Gly Gly
            420                 425                 430

Ala Pro Gly Gly Val Pro Gly Gly Ala Pro Gly Gly Ala Pro Gly Gly
        435                 440                 445

Val Pro Gly Gly Ala Pro Gly Gly Ala Pro Gly Gly Val Pro Gly Gly
    450                 455                 460

Ala Pro Gly Gly Ala Pro Gly Gly Val Pro Gly Gly Ala Pro Gly Gly
465                 470                 475                 480

Ala Pro Gly Gly Val Pro Gly Gly Ala Pro Gly Gly Ala Pro Gly Gly
                485                 490                 495

9935 sequence listing

Val Pro Gly Gly Ala Pro Gly Gly Ala Pro Gly Gly Val Pro Gly Gly
            500                 505                 510

Ala Pro Gly Gly Ala Pro Gly Gly Val Pro Gly Gly Ala Pro Gly Gly
        515                 520                 525

<210>  44
<211>  5
<212>  PRT
<213>  Artificial

<220>
<223>  synthetic construct.

<220>
<221>  REPEAT
<222>  (1)..(5)
<223>  [(VPGMG)5]x; wherein x is from about 10 to about 100

<400>  44

Val Pro Gly Met Gly
1               5

<210>  45
<211>  106
<212>  DNA
<213>  Artificial

<220>
<223>  synthetic construct.

<400>  45
aagcttgaag acgttccagg tgcaggcgta ccgggtgctg gcgttccggg tgaaggtgtt      60

ccaggcgcag gtgtaccggg tgcgggtgtt ccaagagacg ggatcc                    106

<210>  46
<211>  106
<212>  DNA
<213>  Artificial

<220>
<223>  synthetic construct.

<400>  46
aagcttgaag acgttccagg tttcggcatc ccgggtgtag gtatcccagg cgttggtatt      60

ccgggtgtag gcatccctgg cgttggcgtt ccaagagacg ggatcc                    106

<210>  47
<211>  106
<212>  DNA
<213>  Artificial

<220>
<223>  synthetic construct.

<400>  47
aagcttgaag acattccagc tgttggtatc ccggctgttg gtatcccagc tgttggcatt      60

9935 sequence listing

ccggctgtag gtatcccggc tgttggtatt ccaagagacg ggatcc                    106


<210>  48
<211>  57
<212>  DNA
<213>  Artificial

<220>
<223>  Synthetic construct.

<400>  48
ccatggttcc agagtcttca ggtaccgaag acgttccagg tgtaggctaa taagctt        57


<210>  49
<211>  400
<212>  PRT
<213>  Artificial

<220>
<223>  Synthetic construct.

<400>  49

Ile Pro Ala Val Gly Ile Pro Ala Val Gly Ile Pro Ala Val Gly Ile
1               5                   10                  15


Pro Ala Val Gly Val Pro Ala Val Gly Ile Pro Ala Val Gly Ile Pro
            20                  25                  30


Ala Val Gly Ile Pro Ala Val Gly Ile Pro Ala Val Gly Val Pro Ala
        35                  40                  45


Val Gly Ile Pro Ala Val Gly Ile Pro Ala Val Gly Ile Pro Ala Val
    50                  55                  60


Gly Ile Pro Ala Val Gly Val Pro Ala Val Gly Ile Pro Ala Val Gly
65                  70                  75                  80


Ile Pro Ala Val Gly Ile Pro Ala Val Gly Ile Pro Ala Val Gly Val
                85                  90                  95


Pro Ala Val Gly Ile Pro Ala Val Gly Ile Pro Ala Val Gly Ile Pro
            100                 105                 110


Ala Val Gly Ile Pro Ala Val Gly Val Pro Ala Val Gly Ile Pro Ala
        115                 120                 125


Val Gly Ile Pro Ala Val Gly Ile Pro Ala Val Gly Ile Pro Ala Val
    130                 135                 140


Gly Val Pro Ala Val Gly Ile Pro Ala Val Gly Ile Pro Ala Val Gly
145                 150                 155                 160


Ile Pro Ala Val Gly Ile Pro Ala Val Gly Val Pro Ala Val Gly Ile
                165                 170                 175

9935 sequence listing

Pro Ala Val Gly Ile Pro Ala Val Gly Ile Pro Ala Val Gly Ile Pro
            180                 185                 190

Ala Val Gly Val Pro Ala Val Gly Ile Pro Ala Val Gly Ile Pro Ala
        195                 200                 205

Val Gly Ile Pro Ala Val Gly Ile Pro Ala Val Gly Val Pro Ala Val
    210                 215                 220

Gly Ile Pro Ala Val Gly Ile Pro Ala Val Gly Ile Pro Ala Val Gly
225                 230                 235                 240

Ile Pro Ala Val Gly Val Pro Ala Val Gly Ile Pro Ala Val Gly Ile
                245                 250                 255

Pro Ala Val Gly Ile Pro Ala Val Gly Ile Pro Ala Val Gly Val Pro
            260                 265                 270

Ala Val Gly Ile Pro Ala Val Gly Ile Pro Ala Val Gly Ile Pro Ala
        275                 280                 285

Val Gly Ile Pro Ala Val Gly Val Pro Ala Val Gly Ile Pro Ala Val
    290                 295                 300

Gly Ile Pro Ala Val Gly Ile Pro Ala Val Gly Ile Pro Ala Val Gly
305                 310                 315                 320

Val Pro Ala Val Gly Ile Pro Ala Val Gly Ile Pro Ala Val Gly Ile
                325                 330                 335

Pro Ala Val Gly Ile Pro Ala Val Gly Val Pro Ala Val Gly Ile Pro
            340                 345                 350

Ala Val Gly Ile Pro Ala Val Gly Ile Pro Ala Val Gly Ile Pro Ala
        355                 360                 365

Val Gly Val Pro Ala Val Gly Ile Pro Ala Val Gly Ile Pro Ala Val
    370                 375                 380

Gly Ile Pro Ala Val Gly Ile Pro Ala Val Gly Val Pro Ala Val Gly
385                 390                 395                 400

<210>  50
<211>  410
<212>  PRT
<213>  Artificial

<220>
<223>  Synthetic construct.

<400>  50

9935 sequence listing
Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

9935 sequence listing
Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly
                405                 410

<210>  51
<211>  821
<212>  PRT
<213>  Artificial

<220>
<223>  Synthetic construct.

<220>
<221>  MISC_FEATURE
<222>  (411)..(411)
<223>  X at position 411 represents an optionally selected midblock
       structure.

<400>  51

Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

9935 sequence listing

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

9935 sequence listing

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Xaa Val Pro Ala Val Gly
                405                 410                 415

Ile Pro Ala Val Gly Ile Pro Ala Val Gly Ile Pro Ala Val Gly Ile
            420                 425                 430

Pro Ala Val Gly Val Pro Ala Val Gly Ile Pro Ala Val Gly Ile Pro
        435                 440                 445

Ala Val Gly Ile Pro Ala Val Gly Ile Pro Ala Val Gly Val Pro Ala
    450                 455                 460

Val Gly Ile Pro Ala Val Gly Ile Pro Ala Val Gly Ile Pro Ala Val
465                 470                 475                 480

Gly Ile Pro Ala Val Gly Val Pro Ala Val Gly Ile Pro Ala Val Gly
                485                 490                 495

Ile Pro Ala Val Gly Ile Pro Ala Val Gly Ile Pro Ala Val Gly Val
            500                 505                 510

Pro Ala Val Gly Ile Pro Ala Val Gly Ile Pro Ala Val Gly Ile Pro
        515                 520                 525

Ala Val Gly Ile Pro Ala Val Gly Val Pro Ala Val Gly Ile Pro Ala
    530                 535                 540

Val Gly Ile Pro Ala Val Gly Ile Pro Ala Val Gly Ile Pro Ala Val
545                 550                 555                 560

Gly Val Pro Ala Val Gly Ile Pro Ala Val Gly Ile Pro Ala Val Gly
                565                 570                 575

Ile Pro Ala Val Gly Ile Pro Ala Val Gly Val Pro Ala Val Gly Ile
            580                 585                 590

Pro Ala Val Gly Ile Pro Ala Val Gly Ile Pro Ala Val Gly Ile Pro
        595                 600                 605

9935 sequence listing

Ala Val Gly Val Pro Ala Val Gly Ile Pro Ala Val Gly Ile Pro Ala
    610                 615                 620

Val Gly Ile Pro Ala Val Gly Ile Pro Ala Val Gly Val Pro Ala Val
625                 630                 635                 640

Gly Ile Pro Ala Val Gly Ile Pro Ala Val Gly Ile Pro Ala Val Gly
                645                 650                 655

Ile Pro Ala Val Gly Val Pro Ala Val Gly Ile Pro Ala Val Gly Ile
            660                 665                 670

Pro Ala Val Gly Ile Pro Ala Val Gly Ile Pro Ala Val Gly Val Pro
        675                 680                 685

Ala Val Gly Ile Pro Ala Val Gly Ile Pro Ala Val Gly Ile Pro Ala
    690                 695                 700

Val Gly Ile Pro Ala Val Gly Val Pro Ala Val Gly Ile Pro Ala Val
705                 710                 715                 720

Gly Ile Pro Ala Val Gly Ile Pro Ala Val Gly Ile Pro Ala Val Gly
                725                 730                 735

Val Pro Ala Val Gly Ile Pro Ala Val Gly Ile Pro Ala Val Gly Ile
            740                 745                 750

Pro Ala Val Gly Ile Pro Ala Val Gly Val Pro Ala Val Gly Ile Pro
        755                 760                 765

Ala Val Gly Ile Pro Ala Val Gly Ile Pro Ala Val Gly Ile Pro Ala
    770                 775                 780

Val Gly Val Pro Ala Val Gly Ile Pro Ala Val Gly Ile Pro Ala Val
785                 790                 795                 800

Gly Ile Pro Ala Val Gly Ile Pro Ala Val Gly Val Pro Ala Val Gly
                805                 810                 815

Ile Pro Ala Val Gly
            820

<210>  52
<211>  1580
<212>  PRT
<213>  Artificial

<220>
<223>  Synthetic construct.

<400>  52

Val Pro Ala Val Gly Ile Pro Ala Val Gly Ile Pro Ala Val Gly Ile

9935 sequence listing
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala

9935 sequence listing
275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Val Pro Gly Val Gly Val
                405                 410                 415

Pro Gly Val Gly Val Pro Gly Val Gly Val Pro Gly Glu Gly Val Pro
            420                 425                 430

Gly Val Gly Val Pro Gly Val Gly Val Pro Gly Val Gly Val Pro Gly
        435                 440                 445

Val Gly Val Pro Gly Glu Gly Val Pro Gly Val Gly Val Pro Gly Val
    450                 455                 460

Gly Val Pro Gly Val Gly Val Pro Gly Val Gly Val Pro Gly Glu Gly
465                 470                 475                 480

Val Pro Gly Val Gly Val Pro Gly Val Gly Val Pro Gly Val Gly Val
                485                 490                 495

Pro Gly Val Gly Val Pro Gly Glu Gly Val Pro Gly Val Gly Val Pro
            500                 505                 510

Gly Val Gly Val Pro Gly Val Gly Val Pro Gly Val Gly Val Pro Gly
        515                 520                 525

Glu Gly Val Pro Gly Val Gly Val Pro Gly Val Gly Val Pro Gly Val
    530                 535                 540

Gly Val Pro Gly Val Gly Val Pro Gly Glu Gly Val Pro Gly Val Gly

9935 sequence listing
545                 550                 555                 560

Val Pro Gly Val Gly Val Pro Gly Val Gly Val Pro Gly Val Gly Val
                565                 570                 575

Pro Gly Glu Gly Val Pro Gly Val Gly Val Pro Gly Val Gly Val Pro
            580                 585                 590

Gly Val Gly Val Pro Gly Val Gly Val Pro Gly Glu Gly Val Pro Gly
        595                 600                 605

Val Gly Val Pro Gly Val Gly Val Pro Gly Val Gly Val Pro Gly Val
    610                 615                 620

Gly Val Pro Gly Glu Gly Val Pro Gly Val Gly Val Pro Gly Val Gly
625                 630                 635                 640

Val Pro Gly Val Gly Val Pro Gly Val Gly Val Pro Gly Glu Gly Val
                645                 650                 655

Pro Gly Val Gly Val Pro Gly Val Gly Val Pro Gly Val Gly Val Pro
            660                 665                 670

Gly Val Gly Val Pro Gly Glu Gly Val Pro Gly Val Gly Val Pro Gly
        675                 680                 685

Val Gly Val Pro Gly Val Gly Val Pro Gly Val Gly Val Pro Gly Glu
    690                 695                 700

Gly Val Pro Gly Val Gly Val Pro Gly Val Gly Val Pro Gly Val Gly
705                 710                 715                 720

Val Pro Gly Val Gly Val Pro Gly Glu Gly Val Pro Gly Val Gly Val
                725                 730                 735

Pro Gly Val Gly Val Pro Gly Val Gly Val Pro Gly Val Gly Val Pro
            740                 745                 750

Gly Glu Gly Val Pro Gly Val Gly Val Pro Gly Val Gly Val Pro Gly
        755                 760                 765

Val Gly Val Pro Gly Val Gly Val Pro Gly Glu Gly Val Pro Gly Val
    770                 775                 780

Gly Val Pro Gly Val Gly Val Pro Gly Val Gly Val Pro Gly Val Gly
785                 790                 795                 800

Val Pro Gly Glu Gly Val Pro Gly Val Gly Val Pro Gly Val Gly Val
                805                 810                 815

Pro Gly Val Gly Val Pro Gly Val Gly Val Pro Gly Glu Gly Val Pro

9935 sequence listing
820                    825                    830

Gly Val Gly Val Pro Gly Val Gly Val Pro Gly Val Gly Val Pro Gly
        835                 840                 845

Val Gly Val Pro Gly Glu Gly Val Pro Gly Val Gly Val Pro Gly Val
    850                 855                 860

Gly Val Pro Gly Val Gly Val Pro Gly Val Gly Val Pro Gly Glu Gly
865                 870                 875                 880

Val Pro Gly Val Gly Val Pro Gly Val Gly Val Pro Gly Val Gly Val
                885                 890                 895

Pro Gly Val Gly Val Pro Gly Glu Gly Val Pro Gly Val Gly Val Pro
            900                 905                 910

Gly Val Gly Val Pro Gly Val Gly Val Pro Gly Val Gly Val Pro Gly
        915                 920                 925

Glu Gly Val Pro Gly Val Gly Val Pro Gly Val Gly Val Pro Gly Val
    930                 935                 940

Gly Val Pro Gly Val Gly Val Pro Gly Glu Gly Val Pro Gly Val Gly
945                 950                 955                 960

Val Pro Gly Val Gly Val Pro Gly Val Gly Val Pro Gly Val Gly Val
                965                 970                 975

Pro Gly Glu Gly Val Pro Gly Val Gly Val Pro Gly Val Gly Val Pro
            980                 985                 990

Gly Val Gly Val Pro Gly Val Gly  Val Pro Gly Glu Gly  Val Pro Gly
        995                 1000                 1005

Val Gly  Val Pro Gly Val Gly  Val Pro Gly Val Gly  Val Pro Gly
    1010                 1015                 1020

Val Gly  Val Pro Gly Glu Gly  Val Pro Gly Val Gly  Val Pro Gly
    1025                 1030                 1035

Val Gly  Val Pro Gly Val Gly  Val Pro Gly Val Gly  Val Pro Gly
    1040                 1045                 1050

Glu Gly  Val Pro Gly Val Gly  Val Pro Gly Val Gly  Val Pro Gly
    1055                 1060                 1065

Val Gly  Val Pro Gly Val Gly  Val Pro Gly Glu Gly  Val Pro Gly
    1070                 1075                 1080

Val Gly  Val Pro Gly Val Gly  Val Pro Gly Val Gly  Val Pro Gly

9935 sequence listing
1085                    1090                    1095

Val Gly Val Pro Gly Glu Gly Val Pro Gly Val Gly Val Pro Gly
    1100                    1105                    1110

Val Gly Val Pro Gly Val Gly Val Pro Gly Val Gly Val Pro Gly
    1115                    1120                    1125

Glu Gly Val Pro Gly Val Gly Val Pro Gly Val Gly Val Pro Gly
    1130                    1135                    1140

Val Gly Val Pro Gly Val Gly Val Pro Gly Glu Gly Val Pro Gly
    1145                    1150                    1155

Val Gly Val Pro Gly Val Gly Val Pro Gly Val Gly Val Pro Ala
    1160                    1165                    1170

Val Gly Ile Pro Ala Val Gly Ile Pro Ala Val Gly Ile Pro Ala
    1175                    1180                    1185

Val Gly Ile Pro Ala Val Gly Val Pro Ala Val Gly Ile Pro Ala
    1190                    1195                    1200

Val Gly Ile Pro Ala Val Gly Ile Pro Ala Val Gly Ile Pro Ala
    1205                    1210                    1215

Val Gly Val Pro Ala Val Gly Ile Pro Ala Val Gly Ile Pro Ala
    1220                    1225                    1230

Val Gly Ile Pro Ala Val Gly Ile Pro Ala Val Gly Val Pro Ala
    1235                    1240                    1245

Val Gly Ile Pro Ala Val Gly Ile Pro Ala Val Gly Ile Pro Ala
    1250                    1255                    1260

Val Gly Ile Pro Ala Val Gly Val Pro Ala Val Gly Ile Pro Ala
    1265                    1270                    1275

Val Gly Ile Pro Ala Val Gly Ile Pro Ala Val Gly Ile Pro Ala
    1280                    1285                    1290

Val Gly Val Pro Ala Val Gly Ile Pro Ala Val Gly Ile Pro Ala
    1295                    1300                    1305

Val Gly Ile Pro Ala Val Gly Ile Pro Ala Val Gly Val Pro Ala
    1310                    1315                    1320

Val Gly Ile Pro Ala Val Gly Ile Pro Ala Val Gly Ile Pro Ala
    1325                    1330                    1335

Val Gly Ile Pro Ala Val Gly Val Pro Ala Val Gly Ile Pro Ala

9935 sequence listing
1340                    1345                    1350

Val Gly Ile Pro Ala Val Gly Ile Pro Ala Val Gly Ile Pro Ala
    1355                    1360                    1365

Val Gly Val Pro Ala Val Gly Ile Pro Ala Val Gly Ile Pro Ala
    1370                    1375                    1380

Val Gly Ile Pro Ala Val Gly Ile Pro Ala Val Gly Val Pro Ala
    1385                    1390                    1395

Val Gly Ile Pro Ala Val Gly Ile Pro Ala Val Gly Ile Pro Ala
    1400                    1405                    1410

Val Gly Ile Pro Ala Val Gly Val Pro Ala Val Gly Ile Pro Ala
    1415                    1420                    1425

Val Gly Ile Pro Ala Val Gly Ile Pro Ala Val Gly Ile Pro Ala
    1430                    1435                    1440

Val Gly Val Pro Ala Val Gly Ile Pro Ala Val Gly Ile Pro Ala
    1445                    1450                    1455

Val Gly Ile Pro Ala Val Gly Ile Pro Ala Val Gly Val Pro Ala
    1460                    1465                    1470

Val Gly Ile Pro Ala Val Gly Ile Pro Ala Val Gly Ile Pro Ala
    1475                    1480                    1485

Val Gly Ile Pro Ala Val Gly Val Pro Ala Val Gly Ile Pro Ala
    1490                    1495                    1500

Val Gly Ile Pro Ala Val Gly Ile Pro Ala Val Gly Ile Pro Ala
    1505                    1510                    1515

Val Gly Val Pro Ala Val Gly Ile Pro Ala Val Gly Ile Pro Ala
    1520                    1525                    1530

Val Gly Ile Pro Ala Val Gly Ile Pro Ala Val Gly Val Pro Ala
    1535                    1540                    1545

Val Gly Ile Pro Ala Val Gly Ile Pro Ala Val Gly Ile Pro Ala
    1550                    1555                    1560

Val Gly Ile Pro Ala Val Gly Val Pro Ala Val Gly Ile Pro Ala
    1565                    1570                    1575

Val Gly
    1580

<210>  53

9935 sequence listing
<211>  2030
<212>  PRT
<213>  Artificial

<220>
<223>  Synthetic construct.

<400>  53

Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

9935 sequence listing

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Val Pro Gly Val Gly Val
                405                 410                 415

Pro Gly Val Gly Val Pro Gly Val Gly Val Pro Gly Glu Gly Val Pro
            420                 425                 430

Gly Val Gly Val Pro Gly Val Gly Val Pro Gly Val Gly Val Pro Gly
        435                 440                 445

Val Gly Val Pro Gly Glu Gly Val Pro Gly Val Gly Val Pro Gly Val
    450                 455                 460

Gly Val Pro Gly Val Gly Val Pro Gly Val Gly Val Pro Gly Glu Gly
465                 470                 475                 480

Val Pro Gly Val Gly Val Pro Gly Val Gly Val Pro Gly Val Gly Val
                485                 490                 495

Pro Gly Val Gly Val Pro Gly Glu Gly Val Pro Gly Val Gly Val Pro
            500                 505                 510

9935 sequence listing

Gly Val Gly Val Pro Gly Val Gly Val Pro Gly Val Gly Val Pro Gly
        515                 520                 525

Glu Gly Val Pro Gly Val Gly Val Pro Gly Val Gly Val Pro Gly Val
    530                 535                 540

Gly Val Pro Gly Val Gly Val Pro Gly Glu Gly Val Pro Gly Val Gly
545                 550                 555                 560

Val Pro Gly Val Gly Val Pro Gly Val Gly Val Pro Gly Val Gly Val
                565                 570                 575

Pro Gly Glu Gly Val Pro Gly Val Gly Val Pro Gly Val Gly Val Pro
            580                 585                 590

Gly Val Gly Val Pro Gly Val Gly Val Pro Gly Glu Gly Val Pro Gly
        595                 600                 605

Val Gly Val Pro Gly Val Gly Val Pro Gly Val Gly Val Pro Gly Val
    610                 615                 620

Gly Val Pro Gly Glu Gly Val Pro Gly Val Gly Val Pro Gly Val Gly
625                 630                 635                 640

Val Pro Gly Val Gly Val Pro Gly Val Gly Val Pro Gly Glu Gly Val
                645                 650                 655

Pro Gly Val Gly Val Pro Gly Val Gly Val Pro Gly Val Gly Val Pro
            660                 665                 670

Gly Val Gly Val Pro Gly Glu Gly Val Pro Gly Val Gly Val Pro Gly
        675                 680                 685

Val Gly Val Pro Gly Val Gly Val Pro Gly Val Gly Val Pro Gly Glu
    690                 695                 700

Gly Val Pro Gly Val Gly Val Pro Gly Val Gly Val Pro Gly Val Gly
705                 710                 715                 720

Val Pro Gly Val Gly Val Pro Gly Glu Gly Val Pro Gly Val Gly Val
                725                 730                 735

Pro Gly Val Gly Val Pro Gly Val Gly Val Pro Gly Val Gly Val Pro
            740                 745                 750

Gly Glu Gly Val Pro Gly Val Gly Val Pro Gly Val Gly Val Pro Gly
        755                 760                 765

Val Gly Val Pro Gly Val Gly Val Pro Gly Glu Gly Val Pro Gly Val
    770                 775                 780

9935 sequence listing

Gly Val Pro Gly Val Gly Val Pro Gly Val Gly Val Pro Gly Val Gly
785                 790                 795                 800

Val Pro Gly Glu Gly Val Pro Gly Val Gly Val Pro Gly Val Gly Val
                805                 810                 815

Pro Gly Val Gly Val Pro Gly Val Gly Val Pro Gly Glu Gly Val Pro
            820                 825                 830

Gly Val Gly Val Pro Gly Val Gly Val Pro Gly Val Gly Val Pro Gly
        835                 840                 845

Val Gly Val Pro Gly Glu Gly Val Pro Gly Val Gly Val Pro Gly Val
    850                 855                 860

Gly Val Pro Gly Val Gly Val Pro Gly Val Gly Val Pro Gly Glu Gly
865                 870                 875                 880

Val Pro Gly Val Gly Val Pro Gly Val Gly Val Pro Gly Val Gly Val
                885                 890                 895

Pro Gly Val Gly Val Pro Gly Glu Gly Val Pro Gly Val Gly Val Pro
            900                 905                 910

Gly Val Gly Val Pro Gly Val Gly Val Pro Gly Val Gly Val Pro Gly
        915                 920                 925

Glu Gly Val Pro Gly Val Gly Val Pro Gly Val Gly Val Pro Gly Val
    930                 935                 940

Gly Val Pro Gly Val Gly Val Pro Gly Glu Gly Val Pro Gly Val Gly
945                 950                 955                 960

Val Pro Gly Val Gly Val Pro Gly Val Gly Val Pro Gly Val Gly Val
                965                 970                 975

Pro Gly Glu Gly Val Pro Gly Val Gly Val Pro Gly Val Gly Val Pro
            980                 985                 990

Gly Val Gly Val Pro Gly Val Gly  Val Pro Gly Glu Gly  Val Pro Gly
        995                 1000                1005

Val Gly  Val Pro Gly Val Gly  Val Pro Gly Val Gly  Val Pro Gly
    1010                1015                1020

Val Gly  Val Pro Gly Glu Gly  Val Pro Gly Val Gly  Val Pro Gly
    1025                1030                1035

Val Gly  Val Pro Gly Val Gly  Val Pro Gly Val Gly  Val Pro Gly
    1040                1045                1050

9935 sequence listing

Glu Gly Val Pro Gly Val Gly Val Pro Gly Val Gly Val Pro Gly
    1055                1060                1065

Val Gly Val Pro Gly Val Gly Val Pro Gly Glu Gly Val Pro Gly
    1070                1075                1080

Val Gly Val Pro Gly Val Gly Val Pro Gly Val Gly Val Pro Gly
    1085                1090                1095

Val Gly Val Pro Gly Glu Gly Val Pro Gly Val Gly Val Pro Gly
    1100                1105                1110

Val Gly Val Pro Gly Val Gly Val Pro Gly Val Gly Val Pro Gly
    1115                1120                1125

Glu Gly Val Pro Gly Val Gly Val Pro Gly Val Gly Val Pro Gly
    1130                1135                1140

Val Gly Val Pro Gly Val Gly Val Pro Gly Glu Gly Val Pro Gly
    1145                1150                1155

Val Gly Val Pro Gly Val Gly Val Pro Gly Val Gly Val Pro Gly
    1160                1165                1170

Val Gly Val Pro Gly Glu Gly Val Pro Gly Val Gly Val Pro Gly
    1175                1180                1185

Val Gly Val Pro Gly Val Gly Val Pro Gly Val Gly Val Pro Gly
    1190                1195                1200

Glu Gly Val Pro Gly Val Gly Val Pro Gly Val Gly Val Pro Gly
    1205                1210                1215

Val Gly Val Pro Gly Val Gly Val Pro Gly Glu Gly Val Pro Gly
    1220                1225                1230

Val Gly Val Pro Gly Val Gly Val Pro Gly Val Gly Val Pro Gly
    1235                1240                1245

Val Gly Val Pro Gly Glu Gly Val Pro Gly Val Gly Val Pro Gly
    1250                1255                1260

Val Gly Val Pro Gly Val Gly Val Pro Gly Val Gly Val Pro Gly
    1265                1270                1275

Glu Gly Val Pro Gly Val Gly Val Pro Gly Val Gly Val Pro Gly
    1280                1285                1290

Val Gly Val Pro Gly Val Gly Val Pro Gly Glu Gly Val Pro Gly
    1295                1300                1305

9935 sequence listing

Val Gly Val Pro Gly Val Gly Val Pro Gly Val Gly Val Pro Gly
    1310                1315                1320

Val Gly Val Pro Gly Glu Gly Val Pro Gly Val Gly Val Pro Gly
    1325                1330                1335

Val Gly Val Pro Gly Val Gly Val Pro Gly Val Gly Val Pro Gly
    1340                1345                1350

Glu Gly Val Pro Gly Val Gly Val Pro Gly Val Gly Val Pro Gly
    1355                1360                1365

Val Gly Val Pro Gly Val Gly Val Pro Gly Glu Gly Val Pro Gly
    1370                1375                1380

Val Gly Val Pro Gly Val Gly Val Pro Gly Val Gly Val Pro Gly
    1385                1390                1395

Val Gly Val Pro Gly Glu Gly Val Pro Gly Val Gly Val Pro Gly
    1400                1405                1410

Val Gly Val Pro Gly Val Gly Val Pro Gly Val Gly Val Pro Gly
    1415                1420                1425

Glu Gly Val Pro Gly Val Gly Val Pro Gly Val Gly Val Pro Gly
    1430                1435                1440

Val Gly Val Pro Gly Val Gly Val Pro Gly Glu Gly Val Pro Gly
    1445                1450                1455

Val Gly Val Pro Gly Val Gly Val Pro Gly Val Gly Val Pro Gly
    1460                1465                1470

Val Gly Val Pro Gly Glu Gly Val Pro Gly Val Gly Val Pro Gly
    1475                1480                1485

Val Gly Val Pro Gly Val Gly Val Pro Gly Val Gly Val Pro Gly
    1490                1495                1500

Glu Gly Val Pro Gly Val Gly Val Pro Gly Val Gly Val Pro Gly
    1505                1510                1515

Val Gly Val Pro Gly Val Gly Val Pro Gly Glu Gly Val Pro Gly
    1520                1525                1530

Val Gly Val Pro Gly Val Gly Val Pro Gly Val Gly Val Pro Gly
    1535                1540                1545

Val Gly Val Pro Gly Glu Gly Val Pro Gly Val Gly Val Pro Gly
    1550                1555                1560

9935 sequence listing

Val Gly Val Pro Gly Val Gly Val Pro Gly Val Gly Val Pro Gly
    1565                1570                1575

Glu Gly Val Pro Gly Val Gly Val Pro Gly Val Gly Val Pro Gly
    1580                1585                1590

Val Gly Val Pro Gly Val Gly Val Pro Gly Glu Gly Val Pro Gly
    1595                1600                1605

Val Gly Val Pro Gly Val Gly Val Pro Gly Val Gly Val Pro Ala
    1610                1615                1620

Val Gly Ile Pro Ala Val Gly Ile Pro Ala Val Gly Ile Pro Ala
    1625                1630                1635

Val Gly Ile Pro Ala Val Gly Val Pro Ala Val Gly Ile Pro Ala
    1640                1645                1650

Val Gly Ile Pro Ala Val Gly Ile Pro Ala Val Gly Ile Pro Ala
    1655                1660                1665

Val Gly Val Pro Ala Val Gly Ile Pro Ala Val Gly Ile Pro Ala
    1670                1675                1680

Val Gly Ile Pro Ala Val Gly Ile Pro Ala Val Gly Val Pro Ala
    1685                1690                1695

Val Gly Ile Pro Ala Val Gly Ile Pro Ala Val Gly Ile Pro Ala
    1700                1705                1710

Val Gly Ile Pro Ala Val Gly Val Pro Ala Val Gly Ile Pro Ala
    1715                1720                1725

Val Gly Ile Pro Ala Val Gly Ile Pro Ala Val Gly Ile Pro Ala
    1730                1735                1740

Val Gly Val Pro Ala Val Gly Ile Pro Ala Val Gly Ile Pro Ala
    1745                1750                1755

Val Gly Ile Pro Ala Val Gly Ile Pro Ala Val Gly Val Pro Ala
    1760                1765                1770

Val Gly Ile Pro Ala Val Gly Ile Pro Ala Val Gly Ile Pro Ala
    1775                1780                1785

Val Gly Ile Pro Ala Val Gly Val Pro Ala Val Gly Ile Pro Ala
    1790                1795                1800

Val Gly Ile Pro Ala Val Gly Ile Pro Ala Val Gly Ile Pro Ala
    1805                1810                1815

9935 sequence listing

Val Gly Val Pro Ala Val Gly Ile Pro Ala Val Gly Ile Pro Ala
    1820                1825                1830

Val Gly Ile Pro Ala Val Gly Ile Pro Ala Val Gly Val Pro Ala
    1835                1840                1845

Val Gly Ile Pro Ala Val Gly Ile Pro Ala Val Gly Ile Pro Ala
    1850                1855                1860

Val Gly Ile Pro Ala Val Gly Val Pro Ala Val Gly Ile Pro Ala
    1865                1870                1875

Val Gly Ile Pro Ala Val Gly Ile Pro Ala Val Gly Ile Pro Ala
    1880                1885                1890

Val Gly Val Pro Ala Val Gly Ile Pro Ala Val Gly Ile Pro Ala
    1895                1900                1905

Val Gly Ile Pro Ala Val Gly Ile Pro Ala Val Gly Val Pro Ala
    1910                1915                1920

Val Gly Ile Pro Ala Val Gly Ile Pro Ala Val Gly Ile Pro Ala
    1925                1930                1935

Val Gly Ile Pro Ala Val Gly Val Pro Ala Val Gly Ile Pro Ala
    1940                1945                1950

Val Gly Ile Pro Ala Val Gly Ile Pro Ala Val Gly Ile Pro Ala
    1955                1960                1965

Val Gly Val Pro Ala Val Gly Ile Pro Ala Val Gly Ile Pro Ala
    1970                1975                1980

Val Gly Ile Pro Ala Val Gly Ile Pro Ala Val Gly Val Pro Ala
    1985                1990                1995

Val Gly Ile Pro Ala Val Gly Ile Pro Ala Val Gly Ile Pro Ala
    2000                2005                2010

Val Gly Ile Pro Ala Val Gly Val Pro Ala Val Gly Ile Pro Ala
    2015                2020                2025

Val Gly
    2030

<210>  54
<211>  1550
<212>  PRT
<213>  Artificial

<220>
<223>  Synthetic construct.

9935 sequence listing

<400> 54

Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro

9935 sequence listing
260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Val Pro Gly Val Gly Ala
                405                 410                 415

Pro Gly Gly Val Pro Gly Gly Ala Pro Gly Gly Ala Pro Gly Gly Val
            420                 425                 430

Pro Gly Gly Ala Pro Gly Gly Ala Pro Gly Gly Val Pro Gly Gly Ala
        435                 440                 445

Pro Gly Gly Ala Pro Gly Gly Val Pro Gly Gly Ala Pro Gly Gly Ala
    450                 455                 460

Pro Gly Gly Val Pro Gly Gly Ala Pro Gly Gly Ala Pro Gly Gly Val
465                 470                 475                 480

Pro Gly Gly Ala Pro Gly Gly Ala Pro Gly Gly Val Pro Gly Gly Ala
                485                 490                 495

Pro Gly Gly Ala Pro Gly Gly Val Pro Gly Gly Ala Pro Gly Gly Ala
            500                 505                 510

Pro Gly Gly Val Pro Gly Gly Ala Pro Gly Gly Ala Pro Gly Gly Val
        515                 520                 525

Pro Gly Gly Ala Pro Gly Gly Ala Pro Gly Gly Val Pro Gly Gly Ala

9935 sequence listing
530                 535                 540

Pro Gly Gly Ala Pro Gly Gly Val Pro Gly Gly Ala Pro Gly Gly Ala
545                 550                 555                 560

Pro Gly Gly Val Pro Gly Gly Ala Pro Gly Gly Ala Pro Gly Gly Val
                565                 570                 575

Pro Gly Gly Ala Pro Gly Gly Ala Pro Gly Gly Val Pro Gly Gly Ala
            580                 585                 590

Pro Gly Gly Ala Pro Gly Gly Val Pro Gly Gly Ala Pro Gly Gly Ala
        595                 600                 605

Pro Gly Gly Val Pro Gly Gly Ala Pro Gly Gly Ala Pro Gly Gly Val
    610                 615                 620

Pro Gly Gly Ala Pro Gly Gly Ala Pro Gly Gly Val Pro Gly Gly Ala
625                 630                 635                 640

Pro Gly Gly Ala Pro Gly Gly Val Pro Gly Gly Ala Pro Gly Gly Ala
                645                 650                 655

Pro Gly Gly Val Pro Gly Gly Ala Pro Gly Gly Ala Pro Gly Gly Val
            660                 665                 670

Pro Gly Gly Ala Pro Gly Gly Ala Pro Gly Gly Val Pro Gly Gly Ala
        675                 680                 685

Pro Gly Gly Ala Pro Gly Gly Val Pro Gly Gly Ala Pro Gly Gly Ala
    690                 695                 700

Pro Gly Gly Val Pro Gly Gly Ala Pro Gly Gly Ala Pro Gly Gly Val
705                 710                 715                 720

Pro Gly Gly Ala Pro Gly Gly Ala Pro Gly Gly Val Pro Gly Gly Ala
                725                 730                 735

Pro Gly Gly Ala Pro Gly Gly Val Pro Gly Gly Ala Pro Gly Gly Ala
            740                 745                 750

Pro Gly Gly Val Pro Gly Gly Ala Pro Gly Gly Ala Pro Gly Gly Val
        755                 760                 765

Pro Gly Gly Ala Pro Gly Gly Ala Pro Gly Gly Val Pro Gly Gly Ala
    770                 775                 780

Pro Gly Gly Ala Pro Gly Gly Val Pro Gly Gly Ala Pro Gly Gly Ala
785                 790                 795                 800

Pro Gly Gly Val Pro Gly Gly Ala Pro Gly Gly Ala Pro Gly Gly Val

9935 sequence listing
805                    810                    815

Pro Gly Gly Ala Pro Gly Gly Ala Pro Gly Gly Val Pro Gly Gly Ala
            820                    825                    830

Pro Gly Gly Ala Pro Gly Gly Val Pro Gly Gly Ala Pro Gly Gly Ala
        835                    840                    845

Pro Gly Gly Val Pro Gly Gly Ala Pro Gly Gly Ala Pro Gly Gly Val
    850                    855                    860

Pro Gly Gly Ala Pro Gly Gly Ala Pro Gly Gly Val Pro Gly Gly Ala
865                    870                    875                    880

Pro Gly Gly Ala Pro Gly Gly Val Pro Gly Gly Ala Pro Gly Gly Ala
                885                    890                    895

Pro Gly Gly Val Pro Gly Gly Ala Pro Gly Gly Ala Pro Gly Gly Val
            900                    905                    910

Pro Gly Gly Ala Pro Gly Gly Ala Pro Gly Gly Val Pro Gly Gly Ala
        915                    920                    925

Pro Gly Gly Ala Pro Gly Gly Val Pro Gly Gly Ala Pro Gly Gly Ala
    930                    935                    940

Pro Gly Gly Val Pro Gly Gly Ala Pro Gly Gly Ala Pro Gly Gly Val
945                    950                    955                    960

Pro Gly Gly Ala Pro Gly Gly Ala Pro Gly Gly Val Pro Gly Gly Ala
                965                    970                    975

Pro Gly Gly Ala Pro Gly Gly Val Pro Gly Gly Ala Pro Gly Gly Ala
            980                    985                    990

Pro Gly Gly Val Pro Gly Gly Ala  Pro Gly Gly Ala Pro  Gly Gly Val
        995                    1000                    1005

Pro Gly  Gly Ala Pro Gly Gly  Ala Pro Gly Gly Val  Pro Gly Gly
    1010                    1015                    1020

Ala Pro  Gly Gly Ala Pro Gly  Gly Val  Pro Gly Gly  Ala Pro Gly
    1025                    1030                    1035

Gly Ala  Pro Gly Gly Val  Pro  Gly Gly Ala Pro Gly  Gly Ala Pro
    1040                    1045                    1050

Gly Gly  Val Pro Gly Gly Ala  Pro Gly Gly Ala Pro  Gly Gly Val
    1055                    1060                    1065

Pro Gly  Gly Ala Pro Gly Gly  Ala Pro Gly Gly Val  Pro Gly Gly

9935 sequence listing
1070                    1075                    1080

Ala Pro Gly Gly Ala Pro Gly Gly Val Pro Gly Gly Ala Pro Gly
    1085                    1090                    1095

Gly Ala Pro Gly Gly Val Pro Gly Gly Ala Pro Gly Gly Ala Pro
    1100                    1105                    1110

Gly Gly Val Pro Gly Gly Ala Pro Gly Gly Ala Pro Gly Gly Val
    1115                    1120                    1125

Pro Gly Gly Ala Pro Gly Gly Val Pro Gly Val Gly Val Pro Ala
    1130                    1135                    1140

Val Gly Ile Pro Ala Val Gly Ile Pro Ala Val Gly Ile Pro Ala
    1145                    1150                    1155

Val Gly Ile Pro Ala Val Gly Val Pro Ala Val Gly Ile Pro Ala
    1160                    1165                    1170

Val Gly Ile Pro Ala Val Gly Ile Pro Ala Val Gly Ile Pro Ala
    1175                    1180                    1185

Val Gly Val Pro Ala Val Gly Ile Pro Ala Val Gly Ile Pro Ala
    1190                    1195                    1200

Val Gly Ile Pro Ala Val Gly Ile Pro Ala Val Gly Val Pro Ala
    1205                    1210                    1215

Val Gly Ile Pro Ala Val Gly Ile Pro Ala Val Gly Ile Pro Ala
    1220                    1225                    1230

Val Gly Ile Pro Ala Val Gly Val Pro Ala Val Gly Ile Pro Ala
    1235                    1240                    1245

Val Gly Ile Pro Ala Val Gly Ile Pro Ala Val Gly Ile Pro Ala
    1250                    1255                    1260

Val Gly Val Pro Ala Val Gly Ile Pro Ala Val Gly Ile Pro Ala
    1265                    1270                    1275

Val Gly Ile Pro Ala Val Gly Ile Pro Ala Val Gly Val Pro Ala
    1280                    1285                    1290

Val Gly Ile Pro Ala Val Gly Ile Pro Ala Val Gly Ile Pro Ala
    1295                    1300                    1305

Val Gly Ile Pro Ala Val Gly Val Pro Ala Val Gly Ile Pro Ala
    1310                    1315                    1320

Val Gly Ile Pro Ala Val Gly Ile Pro Ala Val Gly Ile Pro Ala

9935 sequence listing
    1325                        1330                        1335

Val Gly Val Pro Ala Val Gly Ile Pro Ala Val Gly Ile Pro Ala
    1340                        1345                        1350

Val Gly Ile Pro Ala Val Gly Ile Pro Ala Val Gly Val Pro Ala
    1355                        1360                        1365

Val Gly Ile Pro Ala Val Gly Ile Pro Ala Val Gly Ile Pro Ala
    1370                        1375                        1380

Val Gly Ile Pro Ala Val Gly Val Pro Ala Val Gly Ile Pro Ala
    1385                        1390                        1395

Val Gly Ile Pro Ala Val Gly Ile Pro Ala Val Gly Ile Pro Ala
    1400                        1405                        1410

Val Gly Val Pro Ala Val Gly Ile Pro Ala Val Gly Ile Pro Ala
    1415                        1420                        1425

Val Gly Ile Pro Ala Val Gly Ile Pro Ala Val Gly Val Pro Ala
    1430                        1435                        1440

Val Gly Ile Pro Ala Val Gly Ile Pro Ala Val Gly Ile Pro Ala
    1445                        1450                        1455

Val Gly Ile Pro Ala Val Gly Val Pro Ala Val Gly Ile Pro Ala
    1460                        1465                        1470

Val Gly Ile Pro Ala Val Gly Ile Pro Ala Val Gly Ile Pro Ala
    1475                        1480                        1485

Val Gly Val Pro Ala Val Gly Ile Pro Ala Val Gly Ile Pro Ala
    1490                        1495                        1500

Val Gly Ile Pro Ala Val Gly Ile Pro Ala Val Gly Val Pro Ala
    1505                        1510                        1515

Val Gly Ile Pro Ala Val Gly Ile Pro Ala Val Gly Ile Pro Ala
    1520                        1525                        1530

Val Gly Ile Pro Ala Val Gly Val Pro Ala Val Gly Ile Pro Ala
    1535                        1540                        1545

Val Gly
    1550

<210>  55
<211>  12
<212>  PRT
<213>  Artificial

9935 sequence listing
<220>
<223>  Synthetic construct.

<400>  55

Thr Leu Gln Pro Val Tyr Glu Tyr Met Val Gly Val
1               5                   10


<210>  56
<211>  15
<212>  PRT
<213>  Artificial

<220>
<223>  Synthetic construct.

<400>  56

Thr Gly Leu Pro Val Gly Val Gly Tyr Val Val Thr Val Leu Thr
1               5                   10                  15


<210>  57
<211>  10
<212>  PRT
<213>  Artificial

<220>
<223>  Synthetic construct.

<400>  57

Val Pro Gly Val Gly Val Pro Gly Val Gly
1               5                   10


<210>  58
<211>  830
<212>  PRT
<213>  Artificial

<220>
<223>  Synthetic construct.

<400>  58

Val Pro Ala Val Gly Ile Pro Ala Val Gly Ile Pro Ala Val Gly Ile
1               5                   10                  15


Pro Ala Val Gly Ile Pro Ala Val Gly Val Pro Ala Val Gly Ile Pro
            20                  25                  30


Ala Val Gly Ile Pro Ala Val Gly Ile Pro Ala Val Gly Ile Pro Ala
        35                  40                  45


Val Gly Val Pro Ala Val Gly Ile Pro Ala Val Gly Ile Pro Ala Val
    50                  55                  60


Gly Ile Pro Ala Val Gly Ile Pro Ala Val Gly Val Pro Ala Val Gly
65                  70                  75                  80


Ile Pro Ala Val Gly Ile Pro Ala Val Gly Ile Pro Ala Val Gly Ile

9935 sequence listing
85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala

9935 sequence listing
355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Val Pro Gly Val Gly Val
                405                 410                 415

Pro Gly Val Gly Val Pro Ala Val Gly Ile Pro Ala Val Gly Ile Pro
            420                 425                 430

Ala Val Gly Ile Pro Ala Val Gly Ile Pro Ala Val Gly Val Pro Ala
        435                 440                 445

Val Gly Ile Pro Ala Val Gly Ile Pro Ala Val Gly Ile Pro Ala Val
    450                 455                 460

Gly Ile Pro Ala Val Gly Val Pro Ala Val Gly Ile Pro Ala Val Gly
465                 470                 475                 480

Ile Pro Ala Val Gly Ile Pro Ala Val Gly Ile Pro Ala Val Gly Val
                485                 490                 495

Pro Ala Val Gly Ile Pro Ala Val Gly Ile Pro Ala Val Gly Ile Pro
            500                 505                 510

Ala Val Gly Ile Pro Ala Val Gly Val Pro Ala Val Gly Ile Pro Ala
        515                 520                 525

Val Gly Ile Pro Ala Val Gly Ile Pro Ala Val Gly Ile Pro Ala Val
    530                 535                 540

Gly Val Pro Ala Val Gly Ile Pro Ala Val Gly Ile Pro Ala Val Gly
545                 550                 555                 560

Ile Pro Ala Val Gly Ile Pro Ala Val Gly Val Pro Ala Val Gly Ile
                565                 570                 575

Pro Ala Val Gly Ile Pro Ala Val Gly Ile Pro Ala Val Gly Ile Pro
            580                 585                 590

Ala Val Gly Val Pro Ala Val Gly Ile Pro Ala Val Gly Ile Pro Ala
        595                 600                 605

Val Gly Ile Pro Ala Val Gly Ile Pro Ala Val Gly Val Pro Ala Val
    610                 615                 620

Gly Ile Pro Ala Val Gly Ile Pro Ala Val Gly Ile Pro Ala Val Gly

9935 sequence listing
625                 630                 635                 640

Ile Pro Ala Val Gly Val Pro Ala Val Gly Ile Pro Ala Val Gly Ile
                645                 650                 655

Pro Ala Val Gly Ile Pro Ala Val Gly Ile Pro Ala Val Gly Val Pro
            660                 665                 670

Ala Val Gly Ile Pro Ala Val Gly Ile Pro Ala Val Gly Ile Pro Ala
        675                 680                 685

Val Gly Ile Pro Ala Val Gly Val Pro Ala Val Gly Ile Pro Ala Val
    690                 695                 700

Gly Ile Pro Ala Val Gly Ile Pro Ala Val Gly Ile Pro Ala Val Gly
705                 710                 715                 720

Val Pro Ala Val Gly Ile Pro Ala Val Gly Ile Pro Ala Val Gly Ile
                725                 730                 735

Pro Ala Val Gly Ile Pro Ala Val Gly Val Pro Ala Val Gly Ile Pro
            740                 745                 750

Ala Val Gly Ile Pro Ala Val Gly Ile Pro Ala Val Gly Ile Pro Ala
        755                 760                 765

Val Gly Val Pro Ala Val Gly Ile Pro Ala Val Gly Ile Pro Ala Val
    770                 775                 780

Gly Ile Pro Ala Val Gly Ile Pro Ala Val Gly Val Pro Ala Val Gly
785                 790                 795                 800

Ile Pro Ala Val Gly Ile Pro Ala Val Gly Ile Pro Ala Val Gly Ile
                805                 810                 815

Pro Ala Val Gly Val Pro Ala Val Gly Ile Pro Ala Val Gly
            820                 825                 830

<210>  59
<211>  1780
<212>  PRT
<213>  Artificial

<220>
<223>  Synthetic construct.

<400>  59

Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

9935 sequence listing

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

9935 sequence listing

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Val Pro Gly Val Gly Val
                405                 410                 415

Pro Gly Val Gly Val Pro Gly Val Gly Val Pro Gly Glu Gly Val Pro
            420                 425                 430

Gly Val Gly Val Pro Gly Val Gly Val Pro Gly Val Gly Val Pro Gly
        435                 440                 445

Val Gly Val Pro Gly Glu Gly Val Pro Gly Val Gly Val Pro Gly Val
    450                 455                 460

Gly Val Pro Gly Val Gly Val Pro Gly Val Gly Val Pro Gly Glu Gly
465                 470                 475                 480

Val Pro Gly Val Gly Val Pro Gly Val Gly Val Pro Gly Val Gly Val
                485                 490                 495

Pro Gly Val Gly Val Pro Gly Glu Gly Val Pro Gly Val Gly Val Pro
            500                 505                 510

Gly Val Gly Val Pro Gly Val Gly Val Pro Gly Val Gly Val Pro Gly
        515                 520                 525

Glu Gly Val Pro Gly Val Gly Val Pro Gly Val Gly Val Pro Gly Val
    530                 535                 540

Gly Val Pro Gly Val Gly Val Pro Gly Glu Gly Val Pro Gly Val Gly
545                 550                 555                 560

Val Pro Gly Val Gly Val Pro Gly Val Gly Val Pro Gly Val Gly Val
                565                 570                 575

9935 sequence listing

Pro Gly Glu Gly Val Pro Gly Val Gly Val Pro Gly Val Gly Val Pro
            580                 585                 590

Gly Val Gly Val Pro Gly Val Gly Val Pro Gly Glu Gly Val Pro Gly
        595                 600                 605

Val Gly Val Pro Gly Val Gly Val Pro Gly Val Gly Val Pro Gly Val
    610                 615                 620

Gly Val Pro Gly Glu Gly Val Pro Gly Val Gly Val Pro Gly Val Gly
625                 630                 635                 640

Val Pro Gly Val Gly Val Pro Gly Val Gly Val Pro Gly Glu Gly Val
                645                 650                 655

Pro Gly Val Gly Val Pro Gly Val Gly Val Pro Gly Val Gly Val Pro
            660                 665                 670

Gly Val Gly Val Pro Gly Glu Gly Val Pro Gly Val Gly Val Pro Gly
        675                 680                 685

Val Gly Val Pro Gly Val Gly Val Pro Gly Val Gly Val Pro Gly Glu
    690                 695                 700

Gly Val Pro Gly Val Gly Val Pro Gly Val Gly Val Pro Gly Val Gly
705                 710                 715                 720

Val Pro Gly Val Gly Val Pro Gly Glu Gly Val Pro Gly Val Gly Val
                725                 730                 735

Pro Gly Val Gly Val Pro Gly Val Gly Val Pro Gly Val Gly Val Pro
            740                 745                 750

Gly Glu Gly Val Pro Gly Val Gly Val Pro Gly Val Gly Val Pro Gly
        755                 760                 765

Val Gly Val Pro Gly Val Gly Val Pro Gly Glu Gly Val Pro Gly Val
    770                 775                 780

Gly Val Pro Gly Val Gly Val Pro Gly Val Gly Val Pro Gly Val Gly
785                 790                 795                 800

Val Pro Gly Glu Gly Val Pro Gly Val Gly Val Pro Gly Val Gly Val
                805                 810                 815

Pro Gly Val Gly Val Pro Gly Val Gly Val Pro Gly Glu Gly Val Pro
            820                 825                 830

Gly Val Gly Val Pro Gly Val Gly Val Pro Gly Val Gly Val Pro Gly
        835                 840                 845

9935 sequence listing

Val Gly Val Pro Gly Glu Gly Val Pro Gly Val Gly Val Pro Gly Val
    850                 855                 860

Gly Val Pro Gly Val Gly Val Pro Gly Val Gly Val Pro Gly Glu Gly
865                 870                 875                 880

Val Pro Gly Val Gly Val Pro Gly Val Gly Val Pro Gly Val Gly Val
                885                 890                 895

Pro Gly Val Gly Val Pro Gly Glu Gly Val Pro Gly Val Gly Val Pro
            900                 905                 910

Gly Val Gly Val Pro Gly Val Gly Val Pro Gly Val Gly Val Pro Gly
        915                 920                 925

Glu Gly Val Pro Gly Val Gly Val Pro Gly Val Gly Val Pro Gly Val
    930                 935                 940

Gly Val Pro Gly Val Gly Val Pro Gly Glu Gly Val Pro Gly Val Gly
945                 950                 955                 960

Val Pro Gly Val Gly Val Pro Gly Val Gly Val Pro Gly Val Gly Val
                965                 970                 975

Pro Gly Glu Gly Val Pro Gly Val Gly Val Pro Gly Val Gly Val Pro
            980                 985                 990

Gly Val Gly Val Pro Gly Val Gly  Val Pro Gly Glu Gly  Val Pro Gly
        995                 1000                 1005

Val Gly  Val Pro Gly Val Gly  Val Pro Gly Val Gly  Val Pro Gly
    1010                 1015                 1020

Val Gly  Val Pro Gly Glu Gly  Val Pro Gly Val Gly  Val Pro Gly
    1025                 1030                 1035

Val Gly  Val Pro Gly Val Gly  Val Pro Gly Val Gly  Val Pro Gly
    1040                 1045                 1050

Glu Gly  Val Pro Gly Val Gly  Val Pro Gly Val Gly  Val Pro Gly
    1055                 1060                 1065

Val Gly  Val Pro Gly Val Gly  Val Pro Gly Glu Gly  Val Pro Gly
    1070                 1075                 1080

Val Gly  Val Pro Gly Val Gly  Val Pro Gly Val Gly  Val Pro Gly
    1085                 1090                 1095

Val Gly  Val Pro Gly Glu Gly  Val Pro Gly Val Gly  Val Pro Gly
    1100                 1105                 1110

9935 sequence listing

Val Gly Val Pro Gly Val Gly Val Pro Gly Val Gly Val Pro Gly
    1115                1120                1125

Glu Gly Val Pro Gly Val Gly Val Pro Gly Val Gly Val Pro Gly
    1130                1135                1140

Val Gly Val Pro Gly Val Gly Val Pro Gly Glu Gly Val Pro Gly
    1145                1150                1155

Val Gly Val Pro Gly Val Gly Val Pro Gly Val Gly Val Pro Gly
    1160                1165                1170

Val Gly Val Pro Gly Glu Gly Val Pro Gly Val Gly Val Pro Gly
    1175                1180                1185

Val Gly Val Pro Gly Val Gly Val Pro Gly Val Gly Val Pro Gly
    1190                1195                1200

Glu Gly Val Pro Gly Val Gly Val Pro Gly Val Gly Val Pro Gly
    1205                1210                1215

Val Gly Val Pro Gly Val Gly Val Pro Gly Glu Gly Val Pro Gly
    1220                1225                1230

Val Gly Val Pro Gly Val Gly Val Pro Gly Val Gly Val Pro Gly
    1235                1240                1245

Val Gly Val Pro Gly Glu Gly Val Pro Gly Val Gly Val Pro Gly
    1250                1255                1260

Val Gly Val Pro Gly Val Gly Val Pro Gly Val Gly Val Pro Gly
    1265                1270                1275

Glu Gly Val Pro Gly Val Gly Val Pro Gly Val Gly Val Pro Gly
    1280                1285                1290

Val Gly Val Pro Gly Val Gly Val Pro Gly Glu Gly Val Pro Gly
    1295                1300                1305

Val Gly Val Pro Gly Val Gly Val Pro Gly Val Gly Val Pro Gly
    1310                1315                1320

Val Gly Val Pro Gly Glu Gly Val Pro Gly Val Gly Val Pro Gly
    1325                1330                1335

Val Gly Val Pro Gly Val Gly Val Pro Gly Val Gly Val Pro Gly
    1340                1345                1350

Glu Gly Val Pro Gly Val Gly Val Pro Gly Val Gly Val Pro Gly
    1355                1360                1365

9935 sequence listing

Val Gly Val Pro Ala Val Gly Ile Pro Ala Val Gly Ile Pro Ala
1370 1375 1380

Val Gly Ile Pro Ala Val Gly Ile Pro Ala Val Gly Val Pro Ala
1385 1390 1395

Val Gly Ile Pro Ala Val Gly Ile Pro Ala Val Gly Ile Pro Ala
1400 1405 1410

Val Gly Ile Pro Ala Val Gly Val Pro Ala Val Gly Ile Pro Ala
1415 1420 1425

Val Gly Ile Pro Ala Val Gly Ile Pro Ala Val Gly Ile Pro Ala
1430 1435 1440

Val Gly Val Pro Ala Val Gly Ile Pro Ala Val Gly Ile Pro Ala
1445 1450 1455

Val Gly Ile Pro Ala Val Gly Ile Pro Ala Val Gly Val Pro Ala
1460 1465 1470

Val Gly Ile Pro Ala Val Gly Ile Pro Ala Val Gly Ile Pro Ala
1475 1480 1485

Val Gly Ile Pro Ala Val Gly Val Pro Ala Val Gly Ile Pro Ala
1490 1495 1500

Val Gly Ile Pro Ala Val Gly Ile Pro Ala Val Gly Ile Pro Ala
1505 1510 1515

Val Gly Val Pro Ala Val Gly Ile Pro Ala Val Gly Ile Pro Ala
1520 1525 1530

Val Gly Ile Pro Ala Val Gly Ile Pro Ala Val Gly Val Pro Ala
1535 1540 1545

Val Gly Ile Pro Ala Val Gly Ile Pro Ala Val Gly Ile Pro Ala
1550 1555 1560

Val Gly Ile Pro Ala Val Gly Val Pro Ala Val Gly Ile Pro Ala
1565 1570 1575

Val Gly Ile Pro Ala Val Gly Ile Pro Ala Val Gly Ile Pro Ala
1580 1585 1590

Val Gly Val Pro Ala Val Gly Ile Pro Ala Val Gly Ile Pro Ala
1595 1600 1605

Val Gly Ile Pro Ala Val Gly Ile Pro Ala Val Gly Val Pro Ala
1610 1615 1620

9935 sequence listing

Val Gly Ile Pro Ala Val Gly Ile Pro Ala Val Gly Ile Pro Ala
    1625                1630                1635

Val Gly Ile Pro Ala Val Gly Val Pro Ala Val Gly Ile Pro Ala
    1640                1645                1650

Val Gly Ile Pro Ala Val Gly Ile Pro Ala Val Gly Ile Pro Ala
    1655                1660                1665

Val Gly Val Pro Ala Val Gly Ile Pro Ala Val Gly Ile Pro Ala
    1670                1675                1680

Val Gly Ile Pro Ala Val Gly Ile Pro Ala Val Gly Val Pro Ala
    1685                1690                1695

Val Gly Ile Pro Ala Val Gly Ile Pro Ala Val Gly Ile Pro Ala
    1700                1705                1710

Val Gly Ile Pro Ala Val Gly Val Pro Ala Val Gly Ile Pro Ala
    1715                1720                1725

Val Gly Ile Pro Ala Val Gly Ile Pro Ala Val Gly Ile Pro Ala
    1730                1735                1740

Val Gly Val Pro Ala Val Gly Ile Pro Ala Val Gly Ile Pro Ala
    1745                1750                1755

Val Gly Ile Pro Ala Val Gly Ile Pro Ala Val Gly Val Pro Ala
    1760                1765                1770

Val Gly Ile Pro Ala Val Gly
    1775                1780

<210>  60
<211>  1382
<212>  PRT
<213>  Artificial

<220>
<223>  Synthetic construct.

<400>  60

Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val

9935 sequence listing
50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile

9935 sequence listing
325                    330                    335

Pro Ala Val Gly Ile Pro Ala Val Gly Ile Pro Ala Val Gly Val Pro
            340                    345                    350

Ala Val Gly Ile Pro Ala Val Gly Ile Pro Ala Val Gly Ile Pro Ala
        355                    360                    365

Val Gly Ile Pro Ala Val Gly Val Pro Ala Val Gly Ile Pro Ala Val
    370                    375                    380

Gly Ile Pro Ala Val Gly Ile Pro Ala Val Gly Ile Pro Ala Val Gly
385                    390                    395                    400

Val Pro Ala Val Gly Ile Pro Ala Val Gly Val Pro Gly Val Gly Ala
                405                    410                    415

Pro Gly Gly Val Pro Gly Gly Ala Pro Gly Gly Ala Pro Gly Gly Val
            420                    425                    430

Pro Gly Gly Ala Pro Gly Gly Ala Pro Gly Gly Val Pro Gly Gly Ala
        435                    440                    445

Pro Gly Gly Ala Pro Gly Gly Val Pro Gly Gly Ala Pro Gly Gly Ala
    450                    455                    460

Pro Gly Gly Val Pro Gly Gly Ala Pro Gly Gly Ala Pro Gly Gly Val
465                    470                    475                    480

Pro Gly Gly Ala Pro Gly Gly Ala Pro Gly Gly Val Pro Gly Gly Ala
                485                    490                    495

Pro Gly Gly Ala Pro Gly Gly Val Pro Gly Gly Ala Pro Gly Gly Ala
            500                    505                    510

Pro Gly Gly Val Pro Gly Gly Ala Pro Gly Gly Ala Pro Gly Gly Val
        515                    520                    525

Pro Gly Gly Ala Pro Gly Gly Ala Pro Gly Gly Val Pro Gly Gly Ala
    530                    535                    540

Pro Gly Gly Ala Pro Gly Gly Val Pro Gly Gly Ala Pro Gly Gly Ala
545                    550                    555                    560

Pro Gly Gly Val Pro Gly Gly Ala Pro Gly Gly Ala Pro Gly Gly Val
                565                    570                    575

Pro Gly Gly Ala Pro Gly Gly Ala Pro Gly Gly Val Pro Gly Gly Ala
            580                    585                    590

Pro Gly Gly Ala Pro Gly Gly Val Pro Gly Gly Ala Pro Gly Gly Ala

9935 sequence listing
        595                     600                     605

Pro Gly Gly Val Pro Gly Gly Ala Pro Gly Gly Ala Pro Gly Gly Val
    610                     615                     620

Pro Gly Gly Ala Pro Gly Gly Ala Pro Gly Gly Val Pro Gly Gly Ala
625                     630                     635                     640

Pro Gly Gly Ala Pro Gly Gly Val Pro Gly Gly Ala Pro Gly Gly Ala
                645                     650                     655

Pro Gly Gly Val Pro Gly Gly Ala Pro Gly Gly Ala Pro Gly Gly Val
            660                     665                     670

Pro Gly Gly Ala Pro Gly Gly Ala Pro Gly Gly Val Pro Gly Gly Ala
        675                     680                     685

Pro Gly Gly Ala Pro Gly Gly Val Pro Gly Gly Ala Pro Gly Gly Ala
    690                     695                     700

Pro Gly Gly Val Pro Gly Gly Ala Pro Gly Gly Ala Pro Gly Gly Val
705                     710                     715                     720

Pro Gly Gly Ala Pro Gly Gly Ala Pro Gly Gly Val Pro Gly Gly Ala
                725                     730                     735

Pro Gly Gly Ala Pro Gly Gly Val Pro Gly Gly Ala Pro Gly Gly Ala
            740                     745                     750

Pro Gly Gly Val Pro Gly Gly Ala Pro Gly Gly Ala Pro Gly Gly Val
        755                     760                     765

Pro Gly Gly Ala Pro Gly Gly Ala Pro Gly Gly Val Pro Gly Gly Ala
    770                     775                     780

Pro Gly Gly Ala Pro Gly Gly Val Pro Gly Gly Ala Pro Gly Gly Ala
785                     790                     795                     800

Pro Gly Gly Val Pro Gly Gly Ala Pro Gly Gly Ala Pro Gly Gly Val
                805                     810                     815

Pro Gly Gly Ala Pro Gly Gly Ala Pro Gly Gly Val Pro Gly Gly Ala
            820                     825                     830

Pro Gly Gly Ala Pro Gly Gly Val Pro Gly Gly Ala Pro Gly Gly Ala
        835                     840                     845

Pro Gly Gly Val Pro Gly Gly Ala Pro Gly Gly Ala Pro Gly Gly Val
    850                     855                     860

Pro Gly Gly Ala Pro Gly Gly Ala Pro Gly Gly Val Pro Gly Gly Ala

9935 sequence listing
865                 870                 875                 880

Pro Gly Gly Ala Pro Gly Gly Val Pro Gly Gly Ala Pro Gly Gly Ala
                885                 890                 895

Pro Gly Gly Val Pro Gly Gly Ala Pro Gly Gly Ala Pro Gly Gly Val
            900                 905                 910

Pro Gly Gly Ala Pro Gly Gly Ala Pro Gly Gly Val Pro Gly Gly Ala
        915                 920                 925

Pro Gly Gly Ala Pro Gly Gly Val Pro Gly Gly Ala Pro Gly Gly Ala
    930                 935                 940

Pro Gly Gly Val Pro Gly Gly Ala Pro Gly Gly Ala Pro Gly Gly Val
945                 950                 955                 960

Pro Gly Gly Ala Pro Gly Gly Val Pro Gly Val Gly Val Pro Ala Val
                965                 970                 975

Gly Ile Pro Ala Val Gly Ile Pro Ala Val Gly Ile Pro Ala Val Gly
            980                 985                 990

Ile Pro Ala Val Gly Val Pro Ala  Val Gly Ile Pro Ala  Val Gly Ile
        995                 1000                 1005

Pro Ala  Val Gly Ile Pro Ala  Val Gly Ile Pro Ala  Val Gly Val
    1010                 1015                 1020

Pro Ala  Val Gly Ile Pro Ala  Val Gly Ile Pro Ala  Val Gly Ile
    1025                 1030                 1035

Pro Ala  Val Gly Ile Pro Ala  Val Gly Val Pro Ala  Val Gly Ile
    1040                 1045                 1050

Pro Ala  Val Gly Ile Pro Ala  Val Gly Ile Pro Ala  Val Gly Ile
    1055                 1060                 1065

Pro Ala  Val Gly Val Pro Ala  Val Gly Ile Pro Ala  Val Gly Ile
    1070                 1075                 1080

Pro Ala  Val Gly Ile Pro Ala  Val Gly Ile Pro Ala  Val Gly Val
    1085                 1090                 1095

Pro Ala  Val Gly Ile Pro Ala  Val Gly Ile Pro Ala  Val Gly Ile
    1100                 1105                 1110

Pro Ala  Val Gly Ile Pro Ala  Val Gly Val Pro Ala  Val Gly Ile
    1115                 1120                 1125

Pro Ala  Val Gly Ile Pro Ala  Val Gly Ile Pro Ala  Val Gly Ile

9935 sequence listing
1130                    1135                    1140

Pro Ala Val Gly Val Pro Ala Val Gly Ile Pro Ala Val Gly Ile
1145                    1150                    1155

Pro Ala Val Gly Ile Pro Ala Val Gly Ile Pro Ala Val Gly Val
1160                    1165                    1170

Pro Ala Val Gly Ile Pro Ala Val Gly Ile Pro Ala Val Gly Ile
1175                    1180                    1185

Pro Ala Val Gly Ile Pro Ala Val Gly Val Pro Ala Val Gly Ile
1190                    1195                    1200

Pro Ala Val Gly Ile Pro Ala Val Gly Ile Pro Ala Val Gly Ile
1205                    1210                    1215

Pro Ala Val Gly Val Pro Ala Val Gly Ile Pro Ala Val Gly Ile
1220                    1225                    1230

Pro Ala Val Gly Ile Pro Ala Val Gly Ile Pro Ala Val Gly Val
1235                    1240                    1245

Pro Ala Val Gly Ile Pro Ala Val Gly Ile Pro Ala Val Gly Ile
1250                    1255                    1260

Pro Ala Val Gly Ile Pro Ala Val Gly Val Pro Ala Val Gly Ile
1265                    1270                    1275

Pro Ala Val Gly Ile Pro Ala Val Gly Ile Pro Ala Val Gly Ile
1280                    1285                    1290

Pro Ala Val Gly Val Pro Ala Val Gly Ile Pro Ala Val Gly Ile
1295                    1300                    1305

Pro Ala Val Gly Ile Pro Ala Val Gly Ile Pro Ala Val Gly Val
1310                    1315                    1320

Pro Ala Val Gly Ile Pro Ala Val Gly Ile Pro Ala Val Gly Ile
1325                    1330                    1335

Pro Ala Val Gly Ile Pro Ala Val Gly Val Pro Ala Val Gly Ile
1340                    1345                    1350

Pro Ala Val Gly Ile Pro Ala Val Gly Ile Pro Ala Val Gly Ile
1355                    1360                    1365

Pro Ala Val Gly Val Pro Ala Val Gly Ile Pro Ala Val Gly
1370                    1375                    1380

<210>  61

9935 sequence listing
<211>  1130
<212>  PRT
<213>  Artificial

<220>
<223>  Synthetic construct.

<400>  61

Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val
    130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

9935 sequence listing

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Val Pro Gly Val Gly Val
                405                 410                 415

Pro Gly Val Gly Val Pro Asn Val Gly Val Pro Asn Val Gly Val Pro
            420                 425                 430

Asn Val Gly Val Pro Asn Val Gly Val Pro Gly Val Gly Val Pro Asn
        435                 440                 445

Val Gly Val Pro Asn Val Gly Val Pro Asn Val Gly Val Pro Asn Val
    450                 455                 460

Gly Val Pro Gly Val Gly Val Pro Asn Val Gly Val Pro Asn Val Gly
465                 470                 475                 480

Val Pro Asn Val Gly Val Pro Asn Val Gly Val Pro Gly Val Gly Val
                485                 490                 495

Pro Asn Val Gly Val Pro Asn Val Gly Val Pro Asn Val Gly Val Pro
            500                 505                 510

9935 sequence listing

Asn Val Gly Val Pro Gly Val Gly Val Pro Asn Val Gly Val Pro Asn
        515                 520                 525

Val Gly Val Pro Asn Val Gly Val Pro Asn Val Gly Val Pro Gly Val
    530                 535                 540

Gly Val Pro Asn Val Gly Val Pro Asn Val Gly Val Pro Asn Val Gly
545                 550                 555                 560

Val Pro Asn Val Gly Val Pro Gly Val Gly Val Pro Asn Val Gly Val
                565                 570                 575

Pro Asn Val Gly Val Pro Asn Val Gly Val Pro Asn Val Gly Val Pro
            580                 585                 590

Gly Val Gly Val Pro Asn Val Gly Val Pro Asn Val Gly Val Pro Asn
        595                 600                 605

Val Gly Val Pro Asn Val Gly Val Pro Gly Val Gly Val Pro Asn Val
    610                 615                 620

Gly Val Pro Asn Val Gly Val Pro Asn Val Gly Val Pro Asn Val Gly
625                 630                 635                 640

Val Pro Gly Val Gly Val Pro Asn Val Gly Val Pro Asn Val Gly Val
                645                 650                 655

Pro Asn Val Gly Val Pro Asn Val Gly Val Pro Gly Val Gly Val Pro
            660                 665                 670

Asn Val Gly Val Pro Asn Val Gly Val Pro Asn Val Gly Val Pro Asn
        675                 680                 685

Val Gly Val Pro Gly Val Gly Val Pro Asn Val Gly Val Pro Asn Val
    690                 695                 700

Gly Val Pro Asn Val Gly Val Pro Asn Val Gly Val Pro Gly Val Gly
705                 710                 715                 720

Val Pro Ala Val Gly Ile Pro Ala Val Gly Ile Pro Ala Val Gly Ile
                725                 730                 735

Pro Ala Val Gly Ile Pro Ala Val Gly Val Pro Ala Val Gly Ile Pro
            740                 745                 750

Ala Val Gly Ile Pro Ala Val Gly Ile Pro Ala Val Gly Ile Pro Ala
        755                 760                 765

Val Gly Val Pro Ala Val Gly Ile Pro Ala Val Gly Ile Pro Ala Val
    770                 775                 780

9935 sequence listing

Gly Ile Pro Ala Val Gly Ile Pro Ala Val Gly Val Pro Ala Val Gly
785                 790                 795                 800

Ile Pro Ala Val Gly Ile Pro Ala Val Gly Ile Pro Ala Val Gly Ile
                805                 810                 815

Pro Ala Val Gly Val Pro Ala Val Gly Ile Pro Ala Val Gly Ile Pro
            820                 825                 830

Ala Val Gly Ile Pro Ala Val Gly Ile Pro Ala Val Gly Val Pro Ala
        835                 840                 845

Val Gly Ile Pro Ala Val Gly Ile Pro Ala Val Gly Ile Pro Ala Val
    850                 855                 860

Gly Ile Pro Ala Val Gly Val Pro Ala Val Gly Ile Pro Ala Val Gly
865                 870                 875                 880

Ile Pro Ala Val Gly Ile Pro Ala Val Gly Ile Pro Ala Val Gly Val
                885                 890                 895

Pro Ala Val Gly Ile Pro Ala Val Gly Ile Pro Ala Val Gly Ile Pro
            900                 905                 910

Ala Val Gly Ile Pro Ala Val Gly Val Pro Ala Val Gly Ile Pro Ala
        915                 920                 925

Val Gly Ile Pro Ala Val Gly Ile Pro Ala Val Gly Ile Pro Ala Val
    930                 935                 940

Gly Val Pro Ala Val Gly Ile Pro Ala Val Gly Ile Pro Ala Val Gly
945                 950                 955                 960

Ile Pro Ala Val Gly Ile Pro Ala Val Gly Val Pro Ala Val Gly Ile
                965                 970                 975

Pro Ala Val Gly Ile Pro Ala Val Gly Ile Pro Ala Val Gly Ile Pro
            980                 985                 990

Ala Val Gly Val Pro Ala Val Gly  Ile Pro Ala Val Gly  Ile Pro Ala
        995                 1000                 1005

Val Gly  Ile Pro Ala Val Gly  Ile Pro Ala Val Gly  Val Pro Ala
    1010                 1015                 1020

Val Gly  Ile Pro Ala Val Gly  Ile Pro Ala Val Gly  Ile Pro Ala
    1025                 1030                 1035

Val Gly  Ile Pro Ala Val Gly  Val Pro Ala Val Gly  Ile Pro Ala
    1040                 1045                 1050

9935 sequence listing

Val Gly Ile Pro Ala Val Gly Ile Pro Ala Val Gly Ile Pro Ala
1055 1060 1065

Val Gly Val Pro Ala Val Gly Ile Pro Ala Val Gly Ile Pro Ala
1070 1075 1080

Val Gly Ile Pro Ala Val Gly Ile Pro Ala Val Gly Val Pro Ala
1085 1090 1095

Val Gly Ile Pro Ala Val Gly Ile Pro Ala Val Gly Ile Pro Ala
1100 1105 1110

Val Gly Ile Pro Ala Val Gly Val Pro Ala Val Gly Ile Pro Ala
1115 1120 1125

Val Gly
1130

<210> 62
<211> 1305
<212> PRT
<213> Artificial

<220>
<223> Synthetic construct.

<400> 62

Val Pro Ala Val Gly Ile Pro Ala Val Gly Ile Pro Ala Val Gly Ile
1               5                   10                  15

Pro Ala Val Gly Ile Pro Ala Val Gly Val Pro Ala Val Gly Ile Pro
            20                  25                  30

Ala Val Gly Ile Pro Ala Val Gly Ile Pro Ala Val Gly Ile Pro Ala
        35                  40                  45

Val Gly Val Pro Ala Val Gly Ile Pro Ala Val Gly Ile Pro Ala Val
    50                  55                  60

Gly Ile Pro Ala Val Gly Ile Pro Ala Val Gly Val Pro Ala Val Gly
65                  70                  75                  80

Ile Pro Ala Val Gly Ile Pro Ala Val Gly Ile Pro Ala Val Gly Ile
                85                  90                  95

Pro Ala Val Gly Val Pro Ala Val Gly Ile Pro Ala Val Gly Ile Pro
            100                 105                 110

Ala Val Gly Ile Pro Ala Val Gly Ile Pro Ala Val Gly Val Pro Ala
        115                 120                 125

Val Gly Ile Pro Ala Val Gly Ile Pro Ala Val Gly Ile Pro Ala Val

9935 sequence listing
130                 135                 140

Gly Ile Pro Ala Val Gly Val Pro Ala Val Gly Ile Pro Ala Val Gly
145                 150                 155                 160

Ile Pro Ala Val Gly Ile Pro Ala Val Gly Ile Pro Ala Val Gly Val
                165                 170                 175

Pro Ala Val Gly Ile Pro Ala Val Gly Ile Pro Ala Val Gly Ile Pro
            180                 185                 190

Ala Val Gly Ile Pro Ala Val Gly Val Pro Ala Val Gly Ile Pro Ala
        195                 200                 205

Val Gly Ile Pro Ala Val Gly Ile Pro Ala Val Gly Ile Pro Ala Val
    210                 215                 220

Gly Val Pro Ala Val Gly Ile Pro Ala Val Gly Ile Pro Ala Val Gly
225                 230                 235                 240

Ile Pro Ala Val Gly Ile Pro Ala Val Gly Val Pro Ala Val Gly Ile
                245                 250                 255

Pro Ala Val Gly Ile Pro Ala Val Gly Ile Pro Ala Val Gly Ile Pro
            260                 265                 270

Ala Val Gly Val Pro Ala Val Gly Ile Pro Ala Val Gly Ile Pro Ala
        275                 280                 285

Val Gly Ile Pro Ala Val Gly Ile Pro Ala Val Gly Val Pro Ala Val
    290                 295                 300

Gly Ile Pro Ala Val Gly Ile Pro Ala Val Gly Ile Pro Ala Val Gly
305                 310                 315                 320

Ile Pro Ala Val Gly Val Pro Ala Val Gly Ile Pro Ala Val Gly Ile
                325                 330                 335

Pro Ala Val Gly Ile Pro Ala Val Gly Ile Pro Ala Val Gly Val Pro
            340                 345                 350

Ala Val Gly Ile Pro Ala Val Gly Ile Pro Ala Val Gly Ile Pro Ala
        355                 360                 365

Val Gly Ile Pro Ala Val Gly Val Pro Ala Val Gly Ile Pro Ala Val
    370                 375                 380

Gly Ile Pro Ala Val Gly Ile Pro Ala Val Gly Ile Pro Ala Val Gly
385                 390                 395                 400

Val Pro Ala Val Gly Ile Pro Ala Val Gly Val Pro Gly Val Gly Val

9935 sequence listing
405                 410                 415

Pro Gly Val Gly Val Pro Gly Ile Gly Val Pro Gly Val Gly Val Pro
            420                 425                 430

Gly Ile Gly Val Pro Gly Val Gly Val Pro Gly Val Gly Val Pro Gly
        435                 440                 445

Ile Gly Val Pro Gly Val Gly Val Pro Gly Ile Gly Val Pro Gly Val
    450                 455                 460

Gly Val Pro Gly Val Gly Val Pro Gly Ile Gly Val Pro Gly Val Gly
465                 470                 475                 480

Val Pro Gly Ile Gly Val Pro Gly Val Gly Val Pro Gly Val Gly Val
                485                 490                 495

Pro Gly Ile Gly Val Pro Gly Val Gly Val Pro Gly Ile Gly Val Pro
            500                 505                 510

Gly Val Gly Val Pro Gly Val Gly Val Pro Gly Ile Gly Val Pro Gly
        515                 520                 525

Val Gly Val Pro Gly Ile Gly Val Pro Gly Val Gly Val Pro Gly Val
    530                 535                 540

Gly Val Pro Gly Ile Gly Val Pro Gly Val Gly Val Pro Gly Ile Gly
545                 550                 555                 560

Val Pro Gly Val Gly Val Pro Gly Val Gly Val Pro Gly Ile Gly Val
                565                 570                 575

Pro Gly Val Gly Val Pro Gly Ile Gly Val Pro Gly Val Gly Val Pro
            580                 585                 590

Gly Val Gly Val Pro Gly Ile Gly Val Pro Gly Val Gly Val Pro Gly
        595                 600                 605

Ile Gly Val Pro Gly Val Gly Val Pro Gly Val Gly Val Pro Gly Ile
    610                 615                 620

Gly Val Pro Gly Val Gly Val Pro Gly Ile Gly Val Pro Gly Val Gly
625                 630                 635                 640

Val Pro Gly Val Gly Val Pro Gly Ile Gly Val Pro Gly Val Gly Val
                645                 650                 655

Pro Gly Ile Gly Val Pro Gly Val Gly Val Pro Gly Val Gly Val Pro
            660                 665                 670

Gly Ile Gly Val Pro Gly Val Gly Val Pro Gly Ile Gly Val Pro Gly

9935 sequence listing
675                 680                 685

Val Gly Val Pro Gly Val Gly Val Pro Gly Ile Gly Val Pro Gly Val
    690                 695                 700

Gly Val Pro Gly Ile Gly Val Pro Gly Val Gly Val Pro Gly Val Gly
705                 710                 715                 720

Val Pro Gly Ile Gly Val Pro Gly Val Gly Val Pro Gly Ile Gly Val
                725                 730                 735

Pro Gly Val Gly Val Pro Gly Val Gly Val Pro Gly Ile Gly Val Pro
            740                 745                 750

Gly Val Gly Val Pro Gly Ile Gly Val Pro Gly Val Gly Val Pro Gly
        755                 760                 765

Val Gly Val Pro Gly Ile Gly Val Pro Gly Val Gly Val Pro Gly Ile
    770                 775                 780

Gly Val Pro Gly Val Gly Val Pro Gly Val Gly Val Pro Gly Ile Gly
785                 790                 795                 800

Val Pro Gly Val Gly Val Pro Gly Ile Gly Val Pro Gly Val Gly Val
                805                 810                 815

Pro Gly Val Gly Val Pro Gly Ile Gly Val Pro Gly Val Gly Val Pro
            820                 825                 830

Gly Ile Gly Val Pro Gly Val Gly Val Pro Gly Val Gly Val Pro Gly
        835                 840                 845

Ile Gly Val Pro Gly Val Gly Val Pro Gly Ile Gly Val Pro Gly Val
    850                 855                 860

Gly Val Pro Gly Val Gly Val Pro Gly Ile Gly Val Pro Gly Val Gly
865                 870                 875                 880

Val Pro Gly Ile Gly Val Pro Gly Val Gly Val Pro Gly Val Gly Val
                885                 890                 895

Pro Ala Val Gly Ile Pro Ala Val Gly Ile Pro Ala Val Gly Ile Pro
            900                 905                 910

Ala Val Gly Ile Pro Ala Val Gly Val Pro Ala Val Gly Ile Pro Ala
        915                 920                 925

Val Gly Ile Pro Ala Val Gly Ile Pro Ala Val Gly Ile Pro Ala Val
    930                 935                 940

Gly Val Pro Ala Val Gly Ile Pro Ala Val Gly Ile Pro Ala Val Gly

9935 sequence listing
945                 950                 955                 960

Ile Pro Ala Val Gly Ile Pro Ala Val Gly Val Pro Ala Val Gly Ile
                965                 970                 975

Pro Ala Val Gly Ile Pro Ala Val Gly Ile Pro Ala Val Gly Ile Pro
            980                 985                 990

Ala Val Gly Val Pro Ala Val Gly  Ile Pro Ala Val Gly  Ile Pro Ala
        995                 1000                 1005

Val Gly  Ile Pro Ala Val Gly  Ile Pro Ala Val Gly  Val Pro Ala
    1010                 1015                 1020

Val Gly  Ile Pro Ala Val Gly  Ile Pro Ala Val Gly  Ile Pro Ala
    1025                 1030                 1035

Val Gly  Ile Pro Ala Val Gly  Val Pro Ala Val Gly  Ile Pro Ala
    1040                 1045                 1050

Val Gly  Ile Pro Ala Val Gly  Ile Pro Ala Val Gly  Ile Pro Ala
    1055                 1060                 1065

Val Gly  Val Pro Ala Val Gly  Ile Pro Ala Val Gly  Ile Pro Ala
    1070                 1075                 1080

Val Gly  Ile Pro Ala Val Gly  Ile Pro Ala Val Gly  Val Pro Ala
    1085                 1090                 1095

Val Gly  Ile Pro Ala Val Gly  Ile Pro Ala Val Gly  Ile Pro Ala
    1100                 1105                 1110

Val Gly  Ile Pro Ala Val Gly  Val Pro Ala Val Gly  Ile Pro Ala
    1115                 1120                 1125

Val Gly  Ile Pro Ala Val Gly  Ile Pro Ala Val Gly  Ile Pro Ala
    1130                 1135                 1140

Val Gly  Val Pro Ala Val Gly  Ile Pro Ala Val Gly  Ile Pro Ala
    1145                 1150                 1155

Val Gly  Ile Pro Ala Val Gly  Ile Pro Ala Val Gly  Val Pro Ala
    1160                 1165                 1170

Val Gly  Ile Pro Ala Val Gly  Ile Pro Ala Val Gly  Ile Pro Ala
    1175                 1180                 1185

Val Gly  Ile Pro Ala Val Gly  Val Pro Ala Val Gly  Ile Pro Ala
    1190                 1195                 1200

Val Gly  Ile Pro Ala Val Gly  Ile Pro Ala Val Gly  Ile Pro Ala

9935 sequence listing
1205                    1210                    1215

Val Gly Val Pro Ala Val Gly Ile Pro Ala Val Gly Ile Pro Ala
    1220                    1225                    1230

Val Gly Ile Pro Ala Val Gly Ile Pro Ala Val Gly Val Pro Ala
    1235                    1240                    1245

Val Gly Ile Pro Ala Val Gly Ile Pro Ala Val Gly Ile Pro Ala
    1250                    1255                    1260

Val Gly Ile Pro Ala Val Gly Val Pro Ala Val Gly Ile Pro Ala
    1265                    1270                    1275

Val Gly Ile Pro Ala Val Gly Ile Pro Ala Val Gly Ile Pro Ala
    1280                    1285                    1290

Val Gly Val Pro Ala Val Gly Ile Pro Ala Val Gly
    1295                    1300                    1305

<210>  63
<211>  25
<212>  PRT
<213>  Artificial

<220>
<223>  Synthetic construct.

<400>  63

Val Pro Gly Met Gly Val Pro Gly Met Gly Val Pro Gly Met Gly Val
1               5                   10                  15

Pro Gly Met Gly Val Pro Gly Met Gly
            20                  25

<210>  64
<211>  25
<212>  PRT
<213>  Artificial

<220>
<223>  Synthetic construct.

<400>  64

Val Pro Gly Val Gly Val Pro Gly Ile Gly Val Pro Gly Val Gly Val
1               5                   10                  15

Pro Gly Ile Gly Val Pro Gly Val Gly
            20                  25

<210>  65
<211>  12
<212>  PRT
<213>  Artificial

9935 sequence listing
<220>
<223>  Synthetic construct.

<400>  65

Ala Pro Gly Gly Val Pro Gly Gly Ala Pro Gly Gly
1               5                   10


<210>  66
<211>  25
<212>  PRT
<213>  Artificial

<220>
<223>  Synthetic construct.

<400>  66

Val Pro Gly Val Gly Ile Pro Gly Val Gly Val Pro Gly Val Gly Ile
1               5                   10                  15


Pro Gly Val Gly Val Pro Gly Val Gly
            20                  25


<210>  67
<211>  5
<212>  PRT
<213>  Artificial

<220>
<223>  Synthetic construct.

<400>  67

Val Pro Gly Met Gly
1               5


<210>  68
<211>  25
<212>  PRT
<213>  Artificial

<220>
<223>  Synthetic construct.

<400>  68

Val Pro Gly Met Gly Val Pro Gly Met Gly Val Pro Gly Met Gly Val
1               5                   10                  15


Pro Gly Met Gly Val Pro Gly Met Gly
            20                  25